# EUROPÄISCHE PATENTANMELDUNG

(11) **EP 3 875 584 A1**
(43) Veröffentlichungstag der Anmeldung: **08.09.2021**
(21) Anmeldenummer: 21162092.7
(22) Anmeldetag: 10.12.2014
(51) Int. Cl.: C12N 9/04, C12P 7/42, C12P 7/64, C12N 1/19, C12R 1/73

(54) **HEFESTÄMME UND VERFAHREN ZUR PRODUKTION VON OMEGA-HYDROXYFETTSÄUREN UND DICARBONSÄUREN**

(30) Priorität: 12.12.2013 DE 102013022175
(62) Teilanmeldung aus: 14830951.1
(71) Anmelder: Provivi, Inc., Santa Monica, California 90404 (US)
(72) Erfinder: GATTER, Michael, 01129 Dresden (DE); MATTHÄUS, Falk, 01187 Dresden (DE); BARTH, Gerold, 01189 Dresden (DE)
(74) Vertreter: Appleyard Lees IP LLP

(57) **Zusammenfassung**

Die Erfindung betrifft verschiedene neue Hefestämme der Art *Yarrowia lipolytica* sowie zugehörige Verfahren zur biokatalytischen Herstellung von ω-Hydroxyfettsäuren oder Dicarbonsäuren mithilfe dieser Stämme, wodurch vorteilhaft die Bildung von ω-Hydroxyfettsäuren oder Dicarbonsäuren erhöht ist.

SEQUENCE LISTING

<110>  Provivi, Inc.

<120>  Hefestämme und Verfahren zur Produktion von
       Omega-Hydroxyfettsäuren und Dicarbonsäuren

<130>  P35816EPN1

<140>
<141>  2014-12-10

<150>  DE 102013022175.7
<151>  2013-12-12

<160>  101

<170>  PatentIn version 3.5

<210>  1
<211>  2974
<212>  DNA
<213>  Artificial Sequence

<220>
<223>  pJET1.2/blunt (blunt end cloning verctor)

<400>  1
atcttgctga aaaactcgag ccatccggaa gatctggcgg ccgctctccc tatagtgagt      60

cgtattacgc cggatggata tggtgttcag gcacaagtgt taaagcagtt gattttattc     120

actatgatga aaaaaacaat gaatggaacc tgctccaagt taaaaataga gataataccg     180

aaaactcatc gagtagtaag attagagata atacaacaat aaaaaaatgg tttagaactt     240

actcacagcg tgatgctact aattgggaca attttccaga tgaagtatca tctaagaatt     300

taaatgaaga agacttcaga gcttttgtta aaaattattt ggcaaaaata atataattcg     360

gctgcagggg cggcctcgtg atacgcctat ttttataggt taatgtcatg ataataatgg     420

tttcttagac gtcaggtggc acttttcggg gaaatgtgcg cggaacccct atttgtttat     480

ttttctaaat acattcaaat atgtatccgc tcatgagaca ataaccctga taaatgcttc     540

aataatattg aaaaaggaag agtatgagta ttcaacattt ccgtgtcgcc cttattccct     600

tttttgcggc attttgcctt cctgtttttg ctcacccaga aacgctggtg aaagtaaaag     660

atgctgaaga tcagttgggt gcacgagtgg gttacatcga actggatctc aacagcggta     720

agatccttga gagttttcgc cccgaagaac gttttccaat gatgagcact tttaaagttc     780

tgctatgtgg cgcggtatta tcccgtattg acgccgggca agagcaactc ggtcgccgca     840

tacactattc tcagaatgac ttggttgagt actcaccagt cacagaaaag catcttacgg     900

atggcatgac agtaagagaa ttatgcagtg ctgccataac catgagtgat aacactgcgg     960

ccaacttact tctgacaacg atcggaggac cgaaggagct aaccgctttt ttgcacaaca    1020

tgggggatca tgtaactcgc cttgatcgtt gggaaccgga gctgaatgaa gccataccaa    1080

acgacgagcg tgacaccacg atgcctgtag caatggcaac aacgttgcgc aaactattaa 1140

ctggcgaact acttactcta gcttcccggc aacaattaat agactggatg gaggcggata 1200

aagttgcagg accacttctg cgctcggccc ttccggctgg ctggtttatt gctgataaat 1260

ctggagccgg tgagcgtggg tctcgcggta tcattgcagc actggggcca gatggtaagc 1320

cctcccgtat cgtagttatc tacacgacgg ggagtcaggc aactatggat gaacgaaata 1380

gacagatcgc tgagataggt gcctcactga ttaagcattg gtaactgtca gaccaagttt 1440

actcatatat actttagatt gatttaaaac ttcattttta atttaaaagg atctaggtga 1500

agatcctttt tgataatctc atgaccaaaa tcccttaacg tgagttttcg ttccactgag 1560

cgtcagaccc cgtagaaaag atcaaaggat cttcttgaga tccttttttt ctgcgcgtaa 1620

tctgctgctt gcaaacaaaa aaaccaccgc taccagcggt ggtttgtttg ccggatcaag 1680

agctaccaac tctttttccg aaggtaactg gcttcagcag agcgcagata ccaaatactg 1740

tccttctagt gtagccgtag ttaggccacc acttcaagaa ctctgtagca ccgcctacat 1800

acctcgctct gctaatcctg ttaccagtgg ctgctgccag tggcgataag tcgtgtctta 1860

ccgggttgga ctcaagacga tagttaccgg ataaggcgca gcggtcgggc tgaacggggg 1920

gttcgtgcac acagcccagc ttggagcgaa cgacctacac cgaactgaga tacctacagc 1980

gtgagctatg agaaagcgcc acgcttcccg aagggagaaa ggcggacagg tatccggtaa 2040

gcggcagggt cggaacagga gagcgcacga gggagcttcc agggggaaac gcctggtatc 2100

tttatagtcc tgtcgggttt cgccacctct gacttgagcg tcgatttttg tgatgctcgt 2160

caggggggcg gagcctatgg aaaaacgcca gcaacgcggc ctttttacgg ttcctggcct 2220

tttgctggcc ttttgctcac atgttctttc ctgcgttatc ccctgattct gtggataacc 2280

gtattaccgc ctttgagtga gctgataccg ctcgccgcag ccgaacgacc gagcgcagcg 2340

agtcagtgag cgaggaagcg gaagagcgcc caatacgcaa accgcctctc cccgcgcgtt 2400

ggccgattca ttaatgcagc tggcacgaca ggtttcccga ctggaaagca attggcagtg 2460

agcgcaacgc aattaatgtg agttagctca ctcattaggc accccaggct ttacacttta 2520

tgcttccggc tcgtataatg tgtggaatta tgagcggata ataatttcac acaggaggtt 2580

taaactttaa acatgtcaaa agagacgtct tttgttaaga atgctgagga acttgcaaag 2640

caaaaaatgg atgctattaa ccctgaactt tcttcaaaat ttaaattttt aataaaattc 2700

ctgtctcagt ttcctgaagc ttgctctaaa cctcgttcaa aaaaaatgca gaataaagtt 2760

ggtcaagagg aacatattga atatttagct cgtagttttc atgagagtcg attgccaaga 2820

aaacccacgc cacctacaac ggttcctgat gaggtggtta gcatagttct taatataagt 2880

tttaatatac agcctgaaaa tcttgagaga ataaaagaag aacatcgatt ttccatggca 2940

gctgagaata ttgtaggaga tcttctagaa agat                                2974

<210>  2
<211>  2725
<212>  DNA
<213>  Artificial Sequence

<220>
<223>  pUCBM21 (cloning vector)

<400>  2
gcgcccaata cgcaaaccgc ctctccccgc gcgttggccg attcattaat gcagctggca      60

cgacaggttt cccgactgga aagcgggcag tgagcgcaac gcaattaatg tgagttagct     120

cactcattag gcaccccagg ctttacactt tatgcttccg gctcgtatgt tgtgtggaat     180

tgtgagcgga taacaatttc acacaggaaa cagctatgac catgattacg ccaagcttcc     240

atgggatatc gcatgcctgc agagctctag agtcgacggg cccggtaccg cggccgcact     300

taagttacgc gtggatcccc gggaattcac tggccgtcgt tttacaacgt cgtgactggg     360

aaaaccctgg cgttacccaa cttaatcgcc ttgcagcaca tccccctttc gccagctggc     420

gtaatagcga agaggcccgc accgatcgcc cttcccaaca gttgcgcagc ctgaatggcg     480

aatggcgcct gatgcggtat tttctcctta cgcatctgtg cggtatttca caccgcatat     540

ggtgcactct cagtacaatc tgctctgatg ccgcatagtt aagccagccc cgacacccgc     600

caacacccgc tgacgcgccc tgacgggctt gtctgctccc ggcatccgct tacagacaag     660

ctgtgaccgt ctccgggagc tgcatgtgtc agaggttttc accgtcatca ccgaaacgcg     720

cgagacgaaa gggcctcgtg atacgcctat ttttataggt taatgtcatg ataataatgg     780

tttcttagac gtcaggtggc acttttcggg gaaatgtgcg cggaacccct atttgtttat     840

ttttctaaat acattcaaat atgtatccgc tcatgagaca ataaccctga taaatgcttc     900

aataatattg aaaaaggaag agtatgagta ttcaacattt ccgtgtcgcc cttattccct     960

tttttgcggc attttgcctt cctgtttttg ctcacccaga aacgctggtg aaagtaaaag    1020

atgctgaaga tcagttgggt gcacgagtgg gttacatcga actggatctc aacagcggta    1080

agatccttga gagttttcgc cccgaagaac gttttccaat gatgagcact tttaaagttc    1140

tgctatgtgg cgcggtatta tcccgtattg acgccgggca agagcaactc ggtcgccgca    1200

tacactattc tcagaatgac ttggttgagt actcaccagt cacagaaaag catcttacgg    1260

atggcatgac agtaagagaa ttatgcagtg ctgccataac catgagtgat aacactgcgg    1320

ccaacttact tctgacaacg atcggaggac cgaaggagct aaccgctttt ttgcacaaca    1380

tgggggatca tgtaactcgc cttgatcgtt gggaaccgga gctgaatgaa gccataccaa    1440

acgacgagcg tgacaccacg atgcctgtag caatggcaac aacgttgcgc aaactattaa    1500

ctggcgaact acttactcta gcttcccggc aacaattaat agactggatg gaggcggata    1560

aagttgcagg accacttctg cgctcggccc ttccggctgg ctggtttatt gctgataaat 1620

ctggagcccg tgagcgtggg tctcgcggta tcattgcagc actggggcca gatggtaagc 1680

cctcccgtat cgtagttatc tacacgacgg ggagtcaggc aactatggat gaacgaaata 1740

gacagatcgc tgagataggt gcctcactga ttaagcattg gtaactgtca gaccaagttt 1800

actcatatat actttagatt gatttaaaac ttcattttta atttaaaagg atctaggtga 1860

agatcctttt tgataatctc atgaccaaaa tcccttaacg tgagttttcg ttccactgag 1920

cgtcagaccc cgtagaaaag atcaaaggat cttcttgaga tccttttttt ctgcgcgtaa 1980

tctgctgctt gcaaacaaaa aaaccaccgc taccagcggt ggtttgtttg ccggatcaag 2040

agctaccaac tctttttccg aaggtaactg gcttcagcag agcgcagata ccaaatactg 2100

tccttctagt gtagccgtag ttaggccacc acttcaagaa ctctgtagca ccgcctacat 2160

acctcgctct gctaatcctg ttaccagtgg ctgctgccag tggcgataag tcgtgtctta 2220

ccgggttgga ctcaagacga tagttaccgg ataaggcgca gcggtcgggc tgaacggggg 2280

gttcgtgcac acagcccagc ttggagcgaa cgacctacac cgaactgaga tacctacagc 2340

gtgagctatg agaaagcgcc acgcttcccg aagggagaaa ggcggacagg tatccggtaa 2400

gcggcagggt cggaacagga gagcgcacga gggagcttcc agggggaaac gcctggtatc 2460

tttatagtcc tgtcgggttt cgccacctct gacttgagcg tcgatttttg tgatgctcgt 2520

caggggggcg gagcctatgg aaaaacgcca gcaacgcggc ctttttacgg ttcctggcct 2580

tttgctggcc ttttgctcac atgttctttc ctgcgttatc ccctgattct gtggataacc 2640

gtattaccgc ctttgagtga gctgataccg ctcgccgcag ccgaacgacc gagcgcagcg 2700

agtcagtgag cgaggaagcg gaaga 2725

<210> 3
<211> 9203
<212> DNA
<213> Artificial Sequence

<220>
<223> pUC-Lys2-DK2 (source of URA blaster)

<220>
<221> CDS
<222> (5937)..(6797)

<400> 3
catgcgatat cccatggaag cttggcgtaa tcatggtcat agctgtttcc tgtgtgaaat 60

tgttatccgc tcacaattcc acacaacata cgagccggaa gcataaagtg taaagcctgg 120

ggtgcctaat gagtgagcta actcacatta attgcgttgc gctcactgcc cgctttccag 180

tcgggaaacc tgtcgtgcca gctgcattaa tgaatcggcc aacgcgcggg gagaggcggt 240

ttgcgtattg ggcgctcttc cgcttcctcg ctcactgact cgctgcgctc ggtcgttcgg 300

ctgcggcgag cggtatcagc tcactcaaag gcggtaatac ggttatccac agaatcaggg 360

gataacgcag gaaagaacat gtgagcaaaa ggccagcaaa aggccaggaa ccgtaaaaag 420

gccgcgttgc tggcgttttt ccataggctc cgcccccctg acgagcatca caaaaatcga 480

cgctcaagtc agaggtggcg aaacccgaca ggactataaa gataccaggc gtttccccct 540

ggaagctccc tcgtgcgctc tcctgttccg accctgccgc ttaccggata cctgtccgcc 600

tttctccctt cgggaagcgt ggcgctttct catagctcac gctgtaggta tctcagttcg 660

gtgtaggtcg ttcgctccaa gctgggctgt gtgcacgaac cccccgttca gcccgaccgc 720

tgcgccttat ccggtaacta tcgtcttgag tccaacccgg taagacacga cttatcgcca 780

ctggcagcag ccactggtaa caggattagc agagcgaggt atgtaggcgg tgctacagag 840

ttcttgaagt ggtggcctaa ctacggctac actagaagga cagtatttgg tatctgcgct 900

ctgctgaagc cagttacctt cggaaaaaga gttggtagct cttgatccgg caaacaaacc 960

accgctggta gcggtggttt ttttgtttgc aagcagcaga ttacgcgcag aaaaaaagga 1020

tctcaagaag atcctttgat cttttctacg gggtctgacg ctcagtggaa cgaaaactca 1080

cgttaaggga ttttggtcat gagattatca aaaaggatct tcacctagat ccttttaaat 1140

taaaaatgaa gttttaaatc aatctaaagt atatatgagt aaacttggtc tgacagttac 1200

caatgcttaa tcagtgaggc acctatctca gcgatctgtc tatttcgttc atccatagtt 1260

gcctgactcc ccgtcgtgta gataactacg atacgggagg gcttaccatc tggccccagt 1320

gctgcaatga taccgcgaga cccacgctca ccggctccag atttatcagc aataaaccag 1380

ccagccggaa gggccgagcg cagaagtggt cctgcaactt tatccgcctc catccagtct 1440

attaattgtt gccgggaagc tagagtaagt agttcgccag ttaatagttt gcgcaacgtt 1500

gttgccattg ctacaggcat cgtggtgtca cgctcgtcgt ttggtatggc ttcattcagc 1560

tccggttccc aacgatcaag gcgagttaca tgatccccca tgttgtgcaa aaaagcggtt 1620

agctccttcg gtcctccgat cgttgtcaga agtaagttgg ccgcagtgtt atcactcatg 1680

gttatggcag cactgcataa ttctcttact gtcatgccat ccgtaagatg cttttctgtg 1740

actggtgagt actcaaccaa gtcattctga gaatagtgta tgcggcgacc gagttgctct 1800

tgcccggcgt caatacggga taataccgcg ccacatagca gaactttaaa agtgctcatc 1860

attggaaaac gttcttcggg gcgaaaactc tcaaggatct taccgctgtt gagatccagt 1920

tcgatgtaac ccactcgtgc acccaactga tcttcagcat cttttacttt caccagcgtt 1980

tctgggtgag caaaaacagg aaggcaaaat gccgcaaaaa agggaataag ggcgacacgg 2040

aaatgttgaa tactcatact cttccttttt caatattatt gaagcattta tcagggttat 2100

tgtctcatga gcggatacat atttgaatgt atttagaaaa ataaacaaat aggggttccg 2160

cgcacatttc cccgaaaagt gccacctgac gtctaagaaa ccattattat catgacatta 2220

acctataaaa ataggcgtat cacgaggccc tttcgtctcg cgcgtttcgg tgatgacggt 2280

gaaaacctct gacacatgca gctcccggag acggtcacag cttgtctgta agcggatgcc 2340

gggagcagac aagcccgtca gggcgcgtca gcgggtgttg gcgggtgtcg gggctggctt 2400

aactatgcgg catcagagca gattgtactg agagtgcacc atatgcggtg tgaaataccg 2460

cacagatgcg taaggagaaa ataccgcatc aggcgccaac gccgagaagg agtggaacga 2520

gctcttcgcc aagtacggta aggagtatcc caaggagcac tctgagatca tccgacgatt 2580

caagcgagag ctgcccgagg gatgggagaa ggctctgcct acctacaccc ccgccgacaa 2640

tgccgttgct tctcgaaagc tgtccgagat tgtcctcacc aagatccacg aggtcctccc 2700

cgagcttgtt ggtggttccg ccgatctgac cggctcaaac ctgacccgat ggaaggacgc 2760

tgttgatttc cagcctcctg tcacccacct tggtgactac tccggccgat atatccgata 2820

cggtgttcga gagcacggca tgggcgctat catgaacggt atgaacgctt acggaggtat 2880

catcccctac ggaggtactt tccttaactt cgtctcctac gccgctggtg ccgtccgact 2940

gtctgccctg tctggccacc acgttatctg ggttgctacc catgactcca ttggtctggg 3000

tgaggatggc cctacccatc agcccattga gactgtcgcc tggctccgag ccacccccaa 3060

cctctctgtg tggcgacctg ccgacggtaa cgagacctcc gctgcttact acaaggccat 3120

caccaactac cacactccct ctgtcctgtc tctgacccga cagaacctgc ctcagcttga 3180

gggctcttcc atcgagaagg cctccaaggg tggttaccag ctcatctccg aggacaaggg 3240

tgacatctac cttgtgtcca ctggttctga ggttgccatc tgtgttgctg ccgccaagct 3300

cctcaaggag aagaagggta tcactgccgg tgtcatctct ctgcccgact ggttcacctt 3360

cgagcagcag tctctcgagt accgaaagtc tgttttcccc gatggcatcc ccatgctttc 3420

cgtcgaggtc tactccgact ttggctggtc tcgatactct caccagcagt ttggtctgga 3480

ccgattcggt gcttctgctc ccttccagca ggtctacgat gcctttgagt tcaatgccga 3540

gggtgtcgcc aagcgagctg aggccaccat taactactac aagggccaga ctgtcaagtc 3600

tcctattcag cgagccttcg accccattga cgtcaacacc cgacccggcc acggtgtcta 3660

agtgggttgc ccgtctgata taagtattga tatagagtat atttaaatgg aaccccatga 3720

gtttgtagaa ccttgcgtga caactggcat gtatgtgatg tcactgatag ttgtattgcc 3780

gtatgtaact attacgagtt ctggggatga gattcccatg cttccacttg ctcatcagca 3840

aacgctatca aatttacgct caatttttga gcactggcta caatacaaac catctcaaac 3900

gaaaaatgct gtaattattg cgacacctca catttagtca tatatatcat aattaattaa 3960

atcatacaaa aatagtctgc tcacagacaa gcaataatca ccattacacg gtaacaaata 4020

acaaaatggc aatgcttcgc atcgccttat ggtcaaggca ctccctacgg cactgcttac 4080

ttgttaccac cacgtccacc gacactcttc agcttatcaa gggtctgctc agacagctcc 4140

accttcggca aagcaagctc aaccttggac tggggggcat ccaggaagcc gacagccacc 4200

aggtaagcca ggtagatacc catttgagcc tcgtcaacgc ccttaccagc ggacaaatcg 4260

actccggtcc actcagcgtc tcgagagaga gcagatcgag cattggagtc atccagctcg 4320

ggggccttgg tagactgggg aaggtcatcg agcacaaagt gtagcagagg atacagggca 4380

gagtcctgcg agtcctcaac aacaaacttc tccagagcca gacgccaggt gacgtagtcc 4440

tcgacagaaa ctttgtaacc gtacttctgg agggtctgca ggaattcgtt gaaccgtgtt 4500

cgaggctggg aagtcacatg ggcgacaacc acgccggaag gctgcttggg ccagaaagag 4560

gcggcagtaa caacccgagc cacgtgatcg acgggcacca tgttgaccga gttgtggatg 4620

ttgggaatct cacccagctg gatgcatccc ttgatcattc gaaccaggaa atcgtccgtg 4680

ttggtggctc cggtcttgga gtctccgaca acgtaaccag gtcgaatgat ggttccggtg 4740

agtccccggg ctccggcctc tcggatcacg tgctctgcga cccacttgga ctgtccgtag 4800

ccgttgccga gacccacaga agaacccatg agatcgtcgg actcgggaac gccagccttg 4860

ccctcctcca ccagatcggc agacagcttg ttgaagtgct cggtgtcgac ggcagaggtg 4920

gaggacacaa agttgaagat ctactgggct gcttcctaat gcaggagtcg cataagggag 4980

agcgtcgacc gatgcccttg agagccttca acccagtcag ctccttccgg tgggcgcggg 5040

gcatgactat cgtcgccgca cttatgactg tcttctttat catgcaactc gtaggacagg 5100

tgccggcagc gctctgggtc attttcggcg aggaccgctt tcgctggagc gcgacgatga 5160

tcggcctgtc gcttgcggta ttcggaatct tgcacgccct cgctcaagcc ttcgtcactg 5220

gtcccgccac caaacgtttc ggcgagaagc aggccattat cgccggcatg gcggccgacg 5280

cgctgggcta cgtcttgctg gcgttcgcga cgcgaggctg gatggccttc cccattatga 5340

ttcttctcgc ttccggcggc atcgggatgc ccgcgttgca ggccatgctg tccaggcagg 5400

tagatgacga ccatcaggga cagcttcaag gatcgctcgc ggctcttacc agcctaactt 5460

cgatcactgg accgctgatc gtcacggcga tttatgccgc ctcggcgagc acatggaacg 5520

ggttggcatg gattgtaggc gccgccctat accttgtctg cctcctgtct gactcgtcat 5580

tgccgccttt ggagtacgac tccaactatg agtgtgcttg gatcactttg acgatacatt 5640

cttcgttgga ggctgtgggt ctgacagctg cgttttcggc gcggttggcc gacaacaata 5700

tcagctgcaa cgtcattgct ggctttcatc atgatcacat ttttgtcggc aaaggcgacg 5760

cccagagagc cattgacgtt ctttctaatt tggaccgata gccgtatagt ccagtctatc 5820

tataagttca actaactcgt aactattacc ataacatata cttcactgcc ccagataagg 5880

ttccgataaa aagttctgca gactaaattt atttcagtct cctcttcacc accaaa atg 5939
Met
1

ccc tcc tac gaa gct cga gct aac gtc cac aag tcc gcc ttt gcc gct 5987
Pro Ser Tyr Glu Ala Arg Ala Asn Val His Lys Ser Ala Phe Ala Ala
            5                   10                  15

cga gtg ctc aag ctc gtg gca gcc aag aaa acc aac ctg tgt gct tct 6035
Arg Val Leu Lys Leu Val Ala Ala Lys Lys Thr Asn Leu Cys Ala Ser
        20                  25                  30

ctg gat gtt acc acc acc aag gag ctc att gag ctt gcc gat aag gtc 6083
Leu Asp Val Thr Thr Thr Lys Glu Leu Ile Glu Leu Ala Asp Lys Val
    35                  40                  45

gga cct tat gtg tgc atg atc aag acc cat atc gac atc att gac gac 6131
Gly Pro Tyr Val Cys Met Ile Lys Thr His Ile Asp Ile Ile Asp Asp
50                  55                  60                  65

ttc acc tac gcc ggc act gtg ctc ccc ctc aag gaa ctt gct ctt aag 6179
Phe Thr Tyr Ala Gly Thr Val Leu Pro Leu Lys Glu Leu Ala Leu Lys
                70                  75                  80

cac ggt ttc ttc ctg ttc gag gac aga aag ttc gca gat att ggc aac 6227
His Gly Phe Phe Leu Phe Glu Asp Arg Lys Phe Ala Asp Ile Gly Asn
            85                  90                  95

act gtc aag cac cag tac aag aac ggt gtc tac cga atc gcc gag tgg 6275
Thr Val Lys His Gln Tyr Lys Asn Gly Val Tyr Arg Ile Ala Glu Trp
        100                 105                 110

tcc gat atc acc aac gcc cac ggt gta ccc gga acc gga atc att gct 6323
Ser Asp Ile Thr Asn Ala His Gly Val Pro Gly Thr Gly Ile Ile Ala
    115                 120                 125

ggc ctg cga gct ggt gcc gag gaa act gtc tct gaa cag aag aag gag 6371
Gly Leu Arg Ala Gly Ala Glu Glu Thr Val Ser Glu Gln Lys Lys Glu
130                 135                 140                 145

gac gtc tct gac tac gag aac tcc cag tac aag gag ttc ctg gtc ccc 6419
Asp Val Ser Asp Tyr Glu Asn Ser Gln Tyr Lys Glu Phe Leu Val Pro
                150                 155                 160

tct ccc aac gag aag ctg gcc aga ggt ctg ctc atg ctg gcc gag ctg 6467
Ser Pro Asn Glu Lys Leu Ala Arg Gly Leu Leu Met Leu Ala Glu Leu
            165                 170                 175

tct tgc aag ggc tct ctg gcc act ggc gag tac tcc aag cag acc att 6515
Ser Cys Lys Gly Ser Leu Ala Thr Gly Glu Tyr Ser Lys Gln Thr Ile
        180                 185                 190

gag ctt gcc cga tcc gac ccc gag ttt gtg gtt ggc ttc att gcc cag 6563
Glu Leu Ala Arg Ser Asp Pro Glu Phe Val Val Gly Phe Ile Ala Gln
    195                 200                 205

aac cga cct aag ggc gac tct gag gac tgg ctt att ctg acc ccc ggg 6611
Asn Arg Pro Lys Gly Asp Ser Glu Asp Trp Leu Ile Leu Thr Pro Gly
210                 215                 220                 225

gtg ggt ctt gac gac aag gga gac gct ctc gga cag cag tac cga act 6659
Val Gly Leu Asp Asp Lys Gly Asp Ala Leu Gly Gln Gln Tyr Arg Thr
                230                 235                 240

gtt gag gat gtc atg tct acc gga acg gat atc ata att gtc ggc cga     6707
Val Glu Asp Val Met Ser Thr Gly Thr Asp Ile Ile Ile Val Gly Arg
            245                 250                 255

ggt ctg tac ggc cag aac cga gat cct att gag gag gcc aag cga tac     6755
Gly Leu Tyr Gly Gln Asn Arg Asp Pro Ile Glu Glu Ala Lys Arg Tyr
        260                 265                 270

cag aag gct ggc tgg gag gct tac cag aag att aac tgt tag             6797
Gln Lys Ala Gly Trp Glu Ala Tyr Gln Lys Ile Asn Cys
    275                 280                 285

aggttagact atggatatgt catttaactg tgtatataga gagcgtgcaa gtatggagcg     6857

cttgttcagc ttgtatgatg gtcagacgac ctgtctgatc gagtatgtat gatactgcac     6917

aacctgtgta tccgcatgat ctgtccaatg gggcatgttg ttgtgtttct cgatacggag     6977

atgctgggta caagtagcta atacgattga actacttata cttatatgag gcttgaagaa     7037

agctgacttg tgtatgactt attctcaact acatccccag tcacaatacc accagaattc     7097

tactgggctg cttcctaatg caggagtcgc ataaggagaa gcgtcgaccg atgcccttga     7157

gagccttcaa cccagtcagc tccttccggt gggcgcgggg catgactatc gtcgccgcac     7217

ttatgactgt cttctttatc atgcaactcg taggacaggt gccggcagcg ctctgggtca     7277

ttttcggcga ggaccgcttt cgctggagcg cgacgatgat cggcctgtcg cttgcggtat     7337

tcggaatctt gcacgccctc gctcaagcct tcgtcactgg tcccgccacc aaacgtttcg     7397

gcgagaagca ggccattatc gccggcatgg cggccgacgc gctgggctac gtcttgctgg     7457

cgttcgcgac gcgaggctgg atggccttcc ccattatgat tcttctcgct tccggcggca     7517

tcgggatgcc cgcgttgcag gccatgctgt ccaggcaggt agatgacgac catcagggac     7577

agcttcaagg atcgctcgcg gctcttacca gcctaacttc gatcactgga ccgctgatcg     7637

tcacggcgat ttatgccgcc tcggcgagca catggaacgg gttggcatgg attgtaggcg     7697

ccgccctata ccttgtctgc ctggatctcc agttctgtac agccgatccc ggggaccctt     7757

gtagaactct ctccaggcct cacccttgtt gaccttctcg tcctcctgga tccgagacac     7817

acgtcctcgt cgcccgagag acggtagacg agaatggcgt agagagccag agctgcagag     7877

aaatcagtag cgcctgctac accgctgttc ttggcaatct gatcgagctc ggccacggtc     7937

ttggagctaa gagcctccgt caattgtgct tcgactacac tgccttcagc ggggcggacg     7997

aaatcgaacg ggaggactga gagtgtgggg ttggtaaggg cggttgccca ttgtgttact     8057

gtattagtac ggtacatagg gccgaaggtc agagcggacc taataaatag ctgacacaga     8117

ataagtaccc tttctttgtc caggtcatga gtctcggtat cacgtcttaa aagagtcctg     8177

ccgttgtcca tgagtatagt atagcagaca acatagtatt catactgcga tgacggcggg     8237

aattggaggc tacagaactc gaagcggggt catatagaca atcgcatttg ccaacgagtg     8297

tctgattaag gcttctccgt gtgactgctc attgtgtcaa tgctatactg tagtcatagc 8357

tacattgaca gaaagacaac gacagacagt gataaagtaa ccagatcctc aatcaaaaga 8417

gccgcaatga tccatttctc atatcgcata ccatccactc tgtttgtgcc gcatcctcca 8477

caacgatttg ctcatggcac tgttctggtc agatcagaca tcggtagaca caatccagat 8537

gacagttcac agacgattga tctcaatttt aagcctgacc aagcgatctc acgaacgatt 8597

ccttcaattt cccatggata ctcacacgac attgtcggta gttttttttt tttgtagaga 8657

caagttttca aacagatgtc aaaaccgaaa gttgcaagct ttcgatttgg aaggttatat 8717

cgagacaggt gcactgagtc atactgcagt ggagtgcagc tgatgggggg tgatcaaaga 8777

aaatggtcac gtgattttac tgacctatag atattatcac tgcaaatgca gatgacataa 8837

taaatcatac cacgagctcc gcaccccaac tataccctaa ccctacagtc ttctgaaaaa 8897

cctgatctgt gaacacagac cccaacccgg acaaggcttg aaccgctact cgtacagtac 8957

atctccaagg tgggttgatg tgcagagcga tgtcattaat gacaggctgt ggttggctgt 9017

ggtatcaaat gataaagtga taaaaatttg agacacggtt acagattatg agcaacaagg 9077

aggaatcagg gagatgaatg ggagtaagtt gtggtctcaa tgagtggaac agttgacttt 9137

tatacagact aatcatagct tcttgcagtc tcattattat gggctctgta acacattcta 9197

ttgaag 9203

<210> 4
<211> 286
<212> PRT
<213> Artificial Sequence

<220>
<223> Synthetic Construct

<400> 4

Met Pro Ser Tyr Glu Ala Arg Ala Asn Val His Lys Ser Ala Phe Ala
1               5                   10                  15

Ala Arg Val Leu Lys Leu Val Ala Ala Lys Lys Thr Asn Leu Cys Ala
            20                  25                  30

Ser Leu Asp Val Thr Thr Thr Lys Glu Leu Ile Glu Leu Ala Asp Lys
        35                  40                  45

Val Gly Pro Tyr Val Cys Met Ile Lys Thr His Ile Asp Ile Ile Asp
    50                  55                  60

Asp Phe Thr Tyr Ala Gly Thr Val Leu Pro Leu Lys Glu Leu Ala Leu
65                  70                  75                  80

Lys His Gly Phe Phe Leu Phe Glu Asp Arg Lys Phe Ala Asp Ile Gly
                85                  90                  95

Asn Thr Val Lys His Gln Tyr Lys Asn Gly Val Tyr Arg Ile Ala Glu
            100                 105                 110

Trp Ser Asp Ile Thr Asn Ala His Gly Val Pro Gly Thr Gly Ile Ile
        115                 120                 125

Ala Gly Leu Arg Ala Gly Ala Glu Glu Thr Val Ser Glu Gln Lys Lys
    130                 135                 140

Glu Asp Val Ser Asp Tyr Glu Asn Ser Gln Tyr Lys Glu Phe Leu Val
145                 150                 155                 160

Pro Ser Pro Asn Glu Lys Leu Ala Arg Gly Leu Leu Met Leu Ala Glu
                165                 170                 175

Leu Ser Cys Lys Gly Ser Leu Ala Thr Gly Glu Tyr Ser Lys Gln Thr
            180                 185                 190

Ile Glu Leu Ala Arg Ser Asp Pro Glu Phe Val Val Gly Phe Ile Ala
        195                 200                 205

Gln Asn Arg Pro Lys Gly Asp Ser Glu Asp Trp Leu Ile Leu Thr Pro
    210                 215                 220

Gly Val Gly Leu Asp Asp Lys Gly Asp Ala Leu Gly Gln Gln Tyr Arg
225                 230                 235                 240

Thr Val Glu Asp Val Met Ser Thr Gly Thr Asp Ile Ile Ile Val Gly
                245                 250                 255

Arg Gly Leu Tyr Gly Gln Asn Arg Asp Pro Ile Glu Glu Ala Lys Arg
            260                 265                 270

Tyr Gln Lys Ala Gly Trp Glu Ala Tyr Gln Lys Ile Asn Cys
        275                 280                 285

<210>  5
<211>  10839
<212>  DNA
<213>  Artificial Sequence

<220>
<223>  pINTB-HMG1 (cloning vector)

<220>
<221>  CDS

<222>   (207)..(3206)
<223>   3-methyl-glutaryl-CoA reductase (HMG1) gene

<400>   5
ctagtttctt tgtctggcca tccgggtaac ccatgccgga cgcaaaatag actactgaaa      60

atttttttgc tttgtggttg ggactttagc caagggtata aaagaccacc gtccccgaat     120

tacctttcct cttcttttct ctctctcctt gtcaactcac acccgaaatc gttaagcatt     180

tccttctgag tataagaatc attcaa atg cta caa gca gct att gga aag att     233
                             Met Leu Gln Ala Ala Ile Gly Lys Ile
                             1               5

gtg gga ttt gcg gtc aac cga ccc atc cac aca gtt gtc ctg acg tcc     281
Val Gly Phe Ala Val Asn Arg Pro Ile His Thr Val Val Leu Thr Ser
10                  15                  20                  25

atc gtg gcg tca acc gca tac ctc gcc atc ctc gac att gcc atc ccg     329
Ile Val Ala Ser Thr Ala Tyr Leu Ala Ile Leu Asp Ile Ala Ile Pro
                30                  35                  40

ggt ttc gag ggc aca caa ccc atc tca tac tac cac cct gca gca aaa     377
Gly Phe Glu Gly Thr Gln Pro Ile Ser Tyr Tyr His Pro Ala Ala Lys
            45                  50                  55

tct tac gac aac cct gct gat tgg acc cac att gca gag gcc gac atc     425
Ser Tyr Asp Asn Pro Ala Asp Trp Thr His Ile Ala Glu Ala Asp Ile
        60                  65                  70

cct tca gac gcc tac cga ctt gca ttt gcc cag atc cgt gtc agt gat     473
Pro Ser Asp Ala Tyr Arg Leu Ala Phe Ala Gln Ile Arg Val Ser Asp
    75                  80                  85

gtt cag ggc gga gag gcc ccc acc atc cct ggc gcc gtg gcc gtg tct     521
Val Gln Gly Gly Glu Ala Pro Thr Ile Pro Gly Ala Val Ala Val Ser
90                  95                  100                 105

gat ctc gac cac aga atc gtc atg gac tac aaa cag tgg gcc ccc tgg     569
Asp Leu Asp His Arg Ile Val Met Asp Tyr Lys Gln Trp Ala Pro Trp
                110                 115                 120

acc gcc agc aac gag cag atc gcc tcg gag aac cac atc tgg aag cac     617
Thr Ala Ser Asn Glu Gln Ile Ala Ser Glu Asn His Ile Trp Lys His
            125                 130                 135

tcc ttc aag gac cac gtg gcc ttc agc tgg atc aag tgg ttc cga tgg     665
Ser Phe Lys Asp His Val Ala Phe Ser Trp Ile Lys Trp Phe Arg Trp
        140                 145                 150

gcc tac ctg cgt ttg tcc act ctc atc cag ggg gca gac aac ttc gac     713
Ala Tyr Leu Arg Leu Ser Thr Leu Ile Gln Gly Ala Asp Asn Phe Asp
    155                 160                 165

att gcc gtg gtc gcc ctt ggc tat ctt gcc atg cac tac acc ttc ttc     761
Ile Ala Val Val Ala Leu Gly Tyr Leu Ala Met His Tyr Thr Phe Phe
170                 175                 180                 185

agt ctc ttc cga tcc atg cga aag gtt ggc tcg cac ttt tgg ctt gcc     809
Ser Leu Phe Arg Ser Met Arg Lys Val Gly Ser His Phe Trp Leu Ala
                190                 195                 200

tcc atg gct ctg gtc tct tcc acc ttc gct ttc ctg ctt gcg gtg gtg     857

Ser Met Ala Leu Val Ser Ser Thr Phe Ala Phe Leu Leu Ala Val Val
            205                 210                 215

gct tcc tct agc ctg ggt tac cga cct agc atg atc acc atg tcc gag 905
Ala Ser Ser Ser Leu Gly Tyr Arg Pro Ser Met Ile Thr Met Ser Glu
        220                 225                 230

ggc ctg ccc ttc ctc gtg gtc gcc att ggc ttt gac cga aag gtc aac 953
Gly Leu Pro Phe Leu Val Val Ala Ile Gly Phe Asp Arg Lys Val Asn
    235                 240                 245

ctg gct agc gag gtg ctc aca tcc aag agc agc cag ctc gct ccc atg 1001
Leu Ala Ser Glu Val Leu Thr Ser Lys Ser Ser Gln Leu Ala Pro Met
250                 255                 260                 265

gtg cag gtg atc aca aag atc gcc tcc aag gcg ctg ttt gag tac agc 1049
Val Gln Val Ile Thr Lys Ile Ala Ser Lys Ala Leu Phe Glu Tyr Ser
                270                 275                 280

ctt gag gtg gcc gcc ctg ttt gct ggc gcc tat acc gga gtt cct cga 1097
Leu Glu Val Ala Ala Leu Phe Ala Gly Ala Tyr Thr Gly Val Pro Arg
            285                 290                 295

ctg tcc cag ttt tgc ttc tta tct gct tgg atc ctc atc ttc gac tac 1145
Leu Ser Gln Phe Cys Phe Leu Ser Ala Trp Ile Leu Ile Phe Asp Tyr
        300                 305                 310

atg ttt ttg ctg acc ttc tac tct gct gtc ctt gct atc aag ttt gag 1193
Met Phe Leu Leu Thr Phe Tyr Ser Ala Val Leu Ala Ile Lys Phe Glu
    315                 320                 325

atc aat cac att aag cga aac cga atg atc cag gat gct ctc aag gag 1241
Ile Asn His Ile Lys Arg Asn Arg Met Ile Gln Asp Ala Leu Lys Glu
330                 335                 340                 345

gat ggt gta tct gct gct gtt gcc gag aag gta gcc gac tct tct ccc 1289
Asp Gly Val Ser Ala Ala Val Ala Glu Lys Val Ala Asp Ser Ser Pro
                350                 355                 360

gac gcc aag ctc gac cga aag tcc gac gtt tct ctt ttt gga gcc tct 1337
Asp Ala Lys Leu Asp Arg Lys Ser Asp Val Ser Leu Phe Gly Ala Ser
            365                 370                 375

ggc gcc att gcg gtg ttc aag atc ttc atg gtc ctt ggg ttc ctt ggt 1385
Gly Ala Ile Ala Val Phe Lys Ile Phe Met Val Leu Gly Phe Leu Gly
        380                 385                 390

ctc aac ctc atc aac ctg act gcc atc cct cac ctt ggc aag gcg gcc 1433
Leu Asn Leu Ile Asn Leu Thr Ala Ile Pro His Leu Gly Lys Ala Ala
    395                 400                 405

gcc gct gcc cag tct gtg act ccc atc acc ctc tcc ccc gag ctt ctc 1481
Ala Ala Ala Gln Ser Val Thr Pro Ile Thr Leu Ser Pro Glu Leu Leu
410                 415                 420                 425

cat gcc atc ccc gcc tct gtg ccc gtt gtt gtc acc ttt gtg ccc agc 1529
His Ala Ile Pro Ala Ser Val Pro Val Val Val Thr Phe Val Pro Ser
                430                 435                 440

gtt gtg tac gag cac tcc cag ctc att ctg cag ctg gag gac gcc ctc 1577
Val Val Tyr Glu His Ser Gln Leu Ile Leu Gln Leu Glu Asp Ala Leu
            445                 450                 455

act acc ttc ctg gct gcc tgc tcc aaa act att ggt gac ccc gtc atc 1625
Thr Thr Phe Leu Ala Ala Cys Ser Lys Thr Ile Gly Asp Pro Val Ile
        460                 465                 470

tcc aag tac atc ttc ctg tgc ctg atg gtc tcc acc gcc ctg aac gtc 1673
Ser Lys Tyr Ile Phe Leu Cys Leu Met Val Ser Thr Ala Leu Asn Val
    475                 480                 485

tac ctg ttt gga gcc acc cga gaa gtt gtg cga acc cag tct gtg aag 1721
Tyr Leu Phe Gly Ala Thr Arg Glu Val Val Arg Thr Gln Ser Val Lys
490                 495                 500                 505

gtg gtt gag aag cac gtt cct atc gtc att gag aag ccc agc gag aag 1769
Val Val Glu Lys His Val Pro Ile Val Ile Glu Lys Pro Ser Glu Lys
                510                 515                 520

gag gag gac acc tct tct gaa gac tcc att gag ctg act gtc gga aag 1817
Glu Glu Asp Thr Ser Ser Glu Asp Ser Ile Glu Leu Thr Val Gly Lys
            525                 530                 535

cag ccc aag ccc gtg acc gag acc cgt tct ctg gac gac cta gag gct 1865
Gln Pro Lys Pro Val Thr Glu Thr Arg Ser Leu Asp Asp Leu Glu Ala
        540                 545                 550

atc atg aag gca ggt aag acc aag ctt ctg gag gac cac gag gtt gtc 1913
Ile Met Lys Ala Gly Lys Thr Lys Leu Leu Glu Asp His Glu Val Val
    555                 560                 565

aag ctc tct ctc gag ggc aag ctt cct ttg tat gct ctt gag aag cag 1961
Lys Leu Ser Leu Glu Gly Lys Leu Pro Leu Tyr Ala Leu Glu Lys Gln
570                 575                 580                 585

ctt ggt gac aac acc cga gct gtt ggc atc cga cga tct atc atc tcc 2009
Leu Gly Asp Asn Thr Arg Ala Val Gly Ile Arg Arg Ser Ile Ile Ser
                590                 595                 600

cag cag tct aat acc aag act tta gag acc tca aag ctt cct tac ctg 2057
Gln Gln Ser Asn Thr Lys Thr Leu Glu Thr Ser Lys Leu Pro Tyr Leu
            605                 610                 615

cac tac gac tac gac cgt gtt ttt gga gcc tgt tgc gag aac gtt att 2105
His Tyr Asp Tyr Asp Arg Val Phe Gly Ala Cys Cys Glu Asn Val Ile
        620                 625                 630

ggt tac atg cct ctc ccc gtt ggt gtt gct ggc ccc atg aac att gat 2153
Gly Tyr Met Pro Leu Pro Val Gly Val Ala Gly Pro Met Asn Ile Asp
    635                 640                 645

ggc aag aac tac cac att cct atg gcc acc act gag ggt tgt ctt gtt 2201
Gly Lys Asn Tyr His Ile Pro Met Ala Thr Thr Glu Gly Cys Leu Val
650                 655                 660                 665

gcc tca acc atg cga ggt tgc aag gcc atc aac gcc ggt ggc ggt gtt 2249
Ala Ser Thr Met Arg Gly Cys Lys Ala Ile Asn Ala Gly Gly Gly Val
                670                 675                 680

acc act gtg ctt act cag gac ggt atg aca cga ggt cct tgt gtt tcc 2297
Thr Thr Val Leu Thr Gln Asp Gly Met Thr Arg Gly Pro Cys Val Ser
            685                 690                 695

ttc ccc tct ctc aag cgg gct gga gcc gct aag atc tgg ctt gat tcc 2345
Phe Pro Ser Leu Lys Arg Ala Gly Ala Ala Lys Ile Trp Leu Asp Ser
        700                 705                 710

gag gag ggt ctc aag tcc atg cga aag gcc ttc aac tcc acc tct cga 2393
Glu Glu Gly Leu Lys Ser Met Arg Lys Ala Phe Asn Ser Thr Ser Arg
    715                 720                 725

ttt gct cgt ctc cag tct ctt cac tct acc ctt gct ggt aac ctg ctg 2441
Phe Ala Arg Leu Gln Ser Leu His Ser Thr Leu Ala Gly Asn Leu Leu
730                 735                 740                 745

ttt att cga ttc cga acc acc act ggt gat gcc atg ggc atg aac atg 2489
Phe Ile Arg Phe Arg Thr Thr Thr Gly Asp Ala Met Gly Met Asn Met
                750                 755                 760

atc tcc aag ggc gtc gaa cac tct ctg gcc gtc atg gtc aag gag tac 2537
Ile Ser Lys Gly Val Glu His Ser Leu Ala Val Met Val Lys Glu Tyr
            765                 770                 775

ggc ttc cct gat atg gac att gtg tct gtc tcg ggt aac tac tgc act 2585
Gly Phe Pro Asp Met Asp Ile Val Ser Val Ser Gly Asn Tyr Cys Thr
        780                 785                 790

gac aag aag ccc gca gcg atc aac tgg atc gaa ggc cga ggc aag agt 2633
Asp Lys Lys Pro Ala Ala Ile Asn Trp Ile Glu Gly Arg Gly Lys Ser
    795                 800                 805

gtt gtt gcc gaa gcc acc atc cct gct cac att gtc aag tct gtt ctc 2681
Val Val Ala Glu Ala Thr Ile Pro Ala His Ile Val Lys Ser Val Leu
810                 815                 820                 825

aaa agt gag gtt gac gct ctt gtt gag ctc aac atc agc aag aat ctg 2729
Lys Ser Glu Val Asp Ala Leu Val Glu Leu Asn Ile Ser Lys Asn Leu
                830                 835                 840

atc ggt agt gcc atg gct ggc tct gtg gga ggt ttc aat gca cac gcc 2777
Ile Gly Ser Ala Met Ala Gly Ser Val Gly Gly Phe Asn Ala His Ala
            845                 850                 855

gca aac ctg gtg acc gcc atc tac ctt gcc act ggc cag gat cct gct 2825
Ala Asn Leu Val Thr Ala Ile Tyr Leu Ala Thr Gly Gln Asp Pro Ala
        860                 865                 870

cag aat gtc gag tct tcc aac tgc atc acg ctg atg agc aac gtc gac 2873
Gln Asn Val Glu Ser Ser Asn Cys Ile Thr Leu Met Ser Asn Val Asp
    875                 880                 885

ggt aac ctg ctc atc tcc gtt tcc atg cct tct atc gag gtc ggt acc 2921
Gly Asn Leu Leu Ile Ser Val Ser Met Pro Ser Ile Glu Val Gly Thr
890                 895                 900                 905

att ggt gga ggt act att ttg gag ccc cag ggg gct atg ctg gag atg 2969
Ile Gly Gly Gly Thr Ile Leu Glu Pro Gln Gly Ala Met Leu Glu Met
                910                 915                 920

ctt ggc gtg cga ggt cct cac atc gag acc ccc ggt gcc aac gcc caa 3017
Leu Gly Val Arg Gly Pro His Ile Glu Thr Pro Gly Ala Asn Ala Gln
            925                 930                 935

cag ctt gct cgc atc att gct tct gga gtt ctt gca gcg gag ctt tcg 3065
Gln Leu Ala Arg Ile Ile Ala Ser Gly Val Leu Ala Ala Glu Leu Ser
        940                 945                 950

ctg tgt tct gct ctt gct gcc ggc cat ctt gtg caa agt cat atg acc 3113
Leu Cys Ser Ala Leu Ala Ala Gly His Leu Val Gln Ser His Met Thr

955                 960                 965

cac aac cgg tcc cag gct cct act ccg gcc aag cag tct cag gcc gat      3161
His Asn Arg Ser Gln Ala Pro Thr Pro Ala Lys Gln Ser Gln Ala Asp
970                 975                 980                 985

ctg cag cgt cta caa aac ggt tcg aat att tgc ata cgg tca tag          3206
Leu Gln Arg Leu Gln Asn Gly Ser Asn Ile Cys Ile Arg Ser
                990                 995

gcatgcaagc tttaagcagt ttgtttagca aaatatattt aacgagtttg atagaggcgc      3266

tggactacat cattactgaa tcacgcgtac atgtctcagc tcaaattgta tcacggtttc      3326

tttgtagcaa tggaggggga gagttgacaa ggcattagag aagagagcga gaggagaaga      3386

caagtggata gacgactgca atcatatgat ctgcacaaac tgcgatgttt tcctgtcaga      3446

tcatgttctt ttgctcatag ttaagctatc gtgactttac ggatccagga ggacgagaag      3506

gtcaacaagg gtgaggcctg gagagagttc tacaagggtc cccgggatcg gctgtacaga      3566

actggagatc caggcagaca aggtataggg cggcgcctac aatccatgcc aacccgttcc      3626

atgtgctcgc cgaggcggca taaatcgccg tgacgatcag cggtccagtg atcgaagtta      3686

ggctggtaag agccgcgagc gatccttgaa gctgtccctg atggtcgtca tctacctgcc      3746

tggacagcat ggcctgcaac gcgggcatcc cgatgccgcc ggaagcgaga agaatcataa      3806

tggggaaggc catccagcct cgcgtcgcga acgccagcaa gacgtagccc agcgcgtcgg      3866

ccgccatgcc ggcgataatg gcctgcttct cgccgaaacg tttggtggcg ggaccagtga      3926

cgaaggcttg agcgagggcg tgcaagattc cgaataccgc aagcgacagg ccgatcatcg      3986

tcgcgctcca gcgaaagcgg tcctcgccga aaatgaccca gagcgctgcc ggcacctgtc      4046

ctacgagttg catgataaag aagacagtca taagtgcggc gacgatagtc atgccccgcg      4106

cccaccggaa ggagctgact gggttgaagg ctctcaaggg catcggtcga cgctctccct      4166

tatgcgactc ctgcattagg aagcagccca gtagaattct ggtggtattg tgactgggga      4226

tgtagttgag aataagtcat acacaagtca gctttcttca agcctcatat aagtataagt      4286

agttcaatcg tattagctac ttgtacccag catctccgta tcgagaaaca caacaacatg      4346

ccccattgga cagatcatgc ggatacacag gttgtgcagt atcatacata ctcgatcaga      4406

caggtcgtct gaccatcata caagctgaac aagcgctcca tacttgcacg ctctctatat      4466

acacagttaa atgacatatc catagtctaa cctctaacag ttaatcttct ggtaagcctc      4526

ccagccagcc ttctggtatc gcttggcctc ctcaatagga tctcggttct ggccgtacag      4586

acctcggccg acaattatga tatccgttcc ggtagacatg acatcctcaa cagttcggta      4646

ctgctgtccg agagcgtctc ccttgtcgtc aagacccacc ccgggggtca gaataagcca      4706

gtcctcagag tcgcccttag gtcggttctg ggcaatgaag ccaaccacaa actcggggtc      4766

ggatcgggca agctcaatgg tctgcttgga gtactcgcca gtggccagag agcccttgca      4826

agacagctcg gccagcatga gcagacctct ggccagcttc tcgttgggag aggggaccag 4886

gaactccttg tactgggagt tctcgtagtc agagacgtcc tccttcttct gttcagagac 4946

agtttcctcg gcaccagctc gcaggccagc aatgattccg gttccgggta caccgtgggc 5006

gttggtgata tcggaccact cggcgattcg gtagacaccg ttcttgtact ggtgcttgac 5066

agtgttgcca atatctgcga actttctgtc ctcgaacagg aagaaaccgt gcttaagagc 5126

aagttccttg agggggagca cagtgccggc gtaggtgaag tcgtcaatga tgtcgatatg 5186

ggtcttgatc atgcacacat aaggtccgac cttatcggca agctcaatga gctccttggt 5246

ggtggtaaca tccagagaag cacacaggtt ggttttcttg gctgccacga gcttgagcac 5306

tcgagcggca aaggcggact tgtggacgtt agctcgagct tcgtaggagg gcattttggt 5366

ggtgaagagg agactgaaat aaatttagtc tgcagaactt tttatcggaa ccttatctgg 5426

ggcagtgaag tatatgttat ggtaatagtt acgagttagt tgaacttata gatagactgg 5486

actatacggc tatcggtcca aattagaaag aacgtcaatg gctctctggg cgtcgccttt 5546

gccgacaaaa atgtgatcat gatgaaagcc agcaatgacg ttgcagctga tattgttgtc 5606

ggccaaccgc gccgaaaacg cagctgtcag acccacagcc tccaacgaag aatgtatcgt 5666

caaagtgatc caagcacact catagttgga gtcgtactcc aaaggcggca atgacgagtc 5726

agacaggagg cagacaaggt atagggcggc gcctacaatc catgccaacc cgttccatgt 5786

gctcgccgag gcggcataaa tcgccgtgac gatcagcggt ccagtgatcg aagttaggct 5846

ggtaagagcc gcgagcgatc cttgaagctg tccctgatgg tcgtcatcta cctgcctgga 5906

cagcatggcc tgcaacgcgg gcatcccgat gccgccggaa gcgagaagaa tcataatggg 5966

gaaggccatc cagcctcgcg tcgcgaacgc cagcaagacg tagcccagcg cgtcggccgc 6026

catgccggcg ataatggcct gcttctcgcc gaaacgtttg gtggcgggac cagtgacgaa 6086

ggcttgagcg agggcgtgca agattccgaa taccgcaagc gacaggccga tcatcgtcgc 6146

gctccagcga aagcggtcct cgccgaaaat gacccagagc gctgccggca cctgtcctac 6206

gagttgcatg ataaagaaga cagtcataag tgcggcgacg atagtcatgc cccgcgccca 6266

ccggaaggag ctgactgggt tgaaggctct caagggcatc ggtcgacgct ctcccttatg 6326

cgactcctgc attaggaagc agcccagtag atccccggga attcactggc cgtcgtttta 6386

caacgtcgtg actgggaaaa ccctggcgtt acccaactta atcgccttgc agcacatccc 6446

cctttcgcca gctggcgtaa tagcgaagag gcccgcaccg atcgcccttc ccaacagttg 6506

cgcagcctga atggcgaatg gcgcctgatg cggtattttc tccttacgca tctgtgcggt 6566

atttcacacc gcatatggtg cactctcagt acaatctgct ctgatgccgc atagttaagc 6626

cagccccgac acccgccaac acccgctgac gcgccctgac gggcttgtct gctcccggca 6686

tccgcttaca gacaagctgt gaccgtctcc gggagctgca tgtgtcagag gttttcaccg 6746

tcatcaccga aacgcgcgag acgaaagggc ctcgtgatac gcctattttt ataggttaat 6806

gtcatgataa taatggtttc ttagacgtca ggtggcactt ttcggggaaa tgtgcgcgga 6866

acccctattt gtttattttt ctaaatacat tcaaatatgt atccgctcat gagacaataa 6926

ccctgataaa tgcttcaata atattgaaaa aggaagagta tgagtattca acatttccgt 6986

gtcgccctta ttcccttttt tgcggcattt tgccttcctg tttttgctca cccagaaacg 7046

ctggtgaaag taaaagatgc tgaagatcag ttgggtgcac gagtgggtta catcgaactg 7106

gatctcaaca gcggtaagat ccttgagagt tttcgccccg aagaacgttt tccaatgatg 7166

agcactttta aagttctgct atgtggcgcg gtattatccc gtattgacgc cgggcaagag 7226

caactcggtc gccgcataca ctattctcag aatgacttgg ttgagtactc accagtcaca 7286

gaaaagcatc ttacggatgg catgacagta agagaattat gcagtgctgc cataaccatg 7346

agtgataaca ctgcggccaa cttacttctg acaacgatcg gaggaccgaa ggagctaacc 7406

gcttttttgc acaacatggg ggatcatgta actcgccttg atcgttggga accggagctg 7466

aatgaagcca taccaaacga cgagcgtgac accacgatgc ctgtagcaat ggcaacaacg 7526

ttgcgcaaac tattaactgg cgaactactt actctagctt cccggcaaca attaatagac 7586

tggatggagg cggataaagt tgcaggacca cttctgcgct cggcccttcc ggctggctgg 7646

tttattgctg ataaatctgg agcccgtgag cgtgggtctc gcggtatcat tgcagcactg 7706

gggccagatg gtaagccctc ccgtatcgta gttatctaca cgacggggag tcaggcaact 7766

atggatgaac gaaatagaca gatcgctgag ataggtgcct cactgattaa gcattggtaa 7826

ctgtcagacc aagtttactc atatatactt tagattgatt taaaacttca tttttaattt 7886

aaaaggatct aggtgaagat cctttttgat aatctcatga ccaaaatccc ttaacgtgag 7946

ttttcgttcc actgagcgtc agaccccgta gaaaagatca aaggatcttc ttgagatcct 8006

ttttttctgc gcgtaatctg ctgcttgcaa acaaaaaaac caccgctacc agcggtggtt 8066

tgtttgccgg atcaagagct accaactctt tttccgaagg taactggctt cagcagagcg 8126

cagataccaa atactgtcct tctagtgtag ccgtagttag gccaccactt caagaactct 8186

gtagcaccgc ctacatacct cgctctgcta atcctgttac cagtggctgc tgccagtggc 8246

gataagtcgt gtcttaccgg gttggactca agacgatagt taccggataa ggcgcagcgg 8306

tcgggctgaa cggggggttc gtgcacacag cccagcttgg agcgaacgac ctacaccgaa 8366

ctgagatacc tacagcgtga gctatgagaa agcgccacgc ttcccgaagg gagaaaggcg 8426

gacaggtatc cggtaagcgg cagggtcgga acaggagagc gcacgaggga gcttccaggg 8486

ggaaacgcct ggtatcttta tagtcctgtc gggtttcgcc acctctgact tgagcgtcga 8546

tttttgtgat gctcgtcagg ggggcggagc ctatggaaaa acgccagcaa cgcggccttt 8606

ttacggttcc tggccttttg ctggcctttt gctcacatgt tctttcctgc gttatcccct 8666

gattctgtgg ataaccgtat taccgccttt gagtgagctg ataccgctcg ccgcagccga 8726

acgaccgagc gcagcgagtc agtgagcgag gaagcggaag agcgcccaat acgcaaaccg 8786

cctctccccg cgcgttggcc gattcattaa tgcagctggc acgacaggtt tcccgactgg 8846

aaagcgggca gtgagcgcaa cgcaattaat gtgagttagc tcactcatta ggcaccccag 8906

gctttacact ttatgcttcc ggctcgtatg ttgtgtggaa ttgtgagcgg ataacaattt 8966

cacacaggaa acagctatga ccatgattac gccaagcttc catgggatct ctagagtcga 9026

cgggcccggt acccccacag ttctcactca gatcatggag actctaacct tgagacatca 9086

attatcagct ctcgaggata atgttagtgc agttccagga ctcattgtgc aactgtcacc 9146

acggcatttt gggtctgttc ttttgaagta cagaaatatc ctcattgttg gtatactttg 9206

ggactttttc ttgttacaga agaataaaaa aacctcgact gatgtactaa ttacatggtt 9266

aacatcccca aggtcaaagt acagatattg taccgacttc tgaaatttgt gggatccaca 9326

cacggacctc tgcgatgata caatatcatg gttccatggt ctcttgaatc acaccactca 9386

ataataaaca ccagctcttt caatccaact tagctttctt ccacttgaac ataggccatc 9446

ccccttcttt tctattttac ataaatagca agatccttac ccctacatgt ctcataacac 9506

aatctcaact tgacttccca taagaagttc actaccctaa ggactctggc agtatttact 9566

gctctcgtgg cggccgcacc tgctcctgca cctaagttcg tcaaaatcgg ttttactcag 9626

agaatcaaca accctaccaa ctgtacatac tgctaaccct gattctttga ataaccccaa 9686

taaggctcct gctgaccctt ctgccgttct aggaaaccag cttgttgagt acgctgtgaa 9746

catcactctt ggtatacccg gtcagccctt ctcagttcag attgacacag gctcgtctga 9806

cttgtgggtg aagagtgacg gctcctccgg tgcattcaac aagaaggctt cttctacttt 9866

tcaggaggac gttcccaacg gctttgcaat tgcccacgga gacaaaacct ctgccattgg 9926

agattgggtc aaagatacca tcaatatcgg tggtgttagt attgaccagt atagtattga 9986

ccagtatgag ttcgccatgg ctactcagac aaatactgac ccggttttgg gtatcggcta 10046

cccgagcaac gaggcgtctt atgggtaccc attccccaca gtaaccatct atattgcaaa 10106

acaatacgac tgcaattggg gattgttgct cgtatcgcac ccgtaccgtg ccaccgtaac 10166

accccccaga caccaatcac tacgaccgat ccaactccac gtttctgcta cccagtctgc 10226

tggccggctt ggctttgcgt ttcgctccca cactacaccg taataccacg tcactctcat 10286

tgcaggttac cctgcccgta gtcgctcgat ccacctcctc cttctctcgt gtgtgcagca 10346

aagaggcaga gatggagccc gtatggtgag ccgtagagtg gagcgagggg cgatcttaca 10406

acggctgtcg gcggatataa acggtatttt cacaattgca ccccagccag accgatagcc 10466

ggtcgcaatc cgccacccac aaccgtctac ctcccacaga accccgtcac ttccaccctt 10526

ttccaccaga tcatatgtcc caacttgcca aattaaaacc gtgcgaattt tcaaaataaa 10586

ctttggcaaa gaggctgcaa aggaggggct ggtgagggcg tctggaagtc gaccagagac 10646

cgggttggcg gcgcatttgt gtcccaaaaa acagccccaa ttgccccaat tgaccccaaa 10706

ttgacccagt agcgggccca accccggcga gagccccctt ctccccacat atcaaacctc 10766

ccccggttcc cacacttgcc gttaagggcg tagggtactg cagtctggaa tctacgcttg 10826

ttcagacttt gta 10839

<210> 6
<211> 999
<212> PRT
<213> Artificial Sequence

<220>
<223> Synthetic Construct

<400> 6

Met Leu Gln Ala Ala Ile Gly Lys Ile Val Gly Phe Ala Val Asn Arg
1               5                   10                  15

Pro Ile His Thr Val Val Leu Thr Ser Ile Val Ala Ser Thr Ala Tyr
            20                  25                  30

Leu Ala Ile Leu Asp Ile Ala Ile Pro Gly Phe Glu Gly Thr Gln Pro
        35                  40                  45

Ile Ser Tyr Tyr His Pro Ala Ala Lys Ser Tyr Asp Asn Pro Ala Asp
    50                  55                  60

Trp Thr His Ile Ala Glu Ala Asp Ile Pro Ser Asp Ala Tyr Arg Leu
65                  70                  75                  80

Ala Phe Ala Gln Ile Arg Val Ser Asp Val Gln Gly Gly Glu Ala Pro
                85                  90                  95

Thr Ile Pro Gly Ala Val Ala Val Ser Asp Leu Asp His Arg Ile Val
            100                 105                 110

Met Asp Tyr Lys Gln Trp Ala Pro Trp Thr Ala Ser Asn Glu Gln Ile
        115                 120                 125

Ala Ser Glu Asn His Ile Trp Lys His Ser Phe Lys Asp His Val Ala
    130                 135                 140

Phe Ser Trp Ile Lys Trp Phe Arg Trp Ala Tyr Leu Arg Leu Ser Thr
145                 150                 155                 160

Leu Ile Gln Gly Ala Asp Asn Phe Asp Ile Ala Val Val Ala Leu Gly
                165                 170                 175

Tyr Leu Ala Met His Tyr Thr Phe Phe Ser Leu Phe Arg Ser Met Arg
            180                 185                 190

Lys Val Gly Ser His Phe Trp Leu Ala Ser Met Ala Leu Val Ser Ser
        195                 200                 205

Thr Phe Ala Phe Leu Leu Ala Val Val Ala Ser Ser Ser Leu Gly Tyr
    210                 215                 220

Arg Pro Ser Met Ile Thr Met Ser Glu Gly Leu Pro Phe Leu Val Val
225                 230                 235                 240

Ala Ile Gly Phe Asp Arg Lys Val Asn Leu Ala Ser Glu Val Leu Thr
                245                 250                 255

Ser Lys Ser Ser Gln Leu Ala Pro Met Val Gln Val Ile Thr Lys Ile
            260                 265                 270

Ala Ser Lys Ala Leu Phe Glu Tyr Ser Leu Glu Val Ala Ala Leu Phe
        275                 280                 285

Ala Gly Ala Tyr Thr Gly Val Pro Arg Leu Ser Gln Phe Cys Phe Leu
    290                 295                 300

Ser Ala Trp Ile Leu Ile Phe Asp Tyr Met Phe Leu Leu Thr Phe Tyr
305                 310                 315                 320

Ser Ala Val Leu Ala Ile Lys Phe Glu Ile Asn His Ile Lys Arg Asn
                325                 330                 335

Arg Met Ile Gln Asp Ala Leu Lys Glu Asp Gly Val Ser Ala Ala Val
            340                 345                 350

Ala Glu Lys Val Ala Asp Ser Ser Pro Asp Ala Lys Leu Asp Arg Lys
        355                 360                 365

Ser Asp Val Ser Leu Phe Gly Ala Ser Gly Ala Ile Ala Val Phe Lys
    370                 375                 380

Ile Phe Met Val Leu Gly Phe Leu Gly Leu Asn Leu Ile Asn Leu Thr
385                 390                 395                 400

Ala Ile Pro His Leu Gly Lys Ala Ala Ala Ala Ala Gln Ser Val Thr

                405                 410                 415

Pro Ile Thr Leu Ser Pro Glu Leu Leu His Ala Ile Pro Ala Ser Val
            420                 425                 430

Pro Val Val Val Thr Phe Val Pro Ser Val Val Tyr Glu His Ser Gln
        435                 440                 445

Leu Ile Leu Gln Leu Glu Asp Ala Leu Thr Thr Phe Leu Ala Ala Cys
    450                 455                 460

Ser Lys Thr Ile Gly Asp Pro Val Ile Ser Lys Tyr Ile Phe Leu Cys
465                 470                 475                 480

Leu Met Val Ser Thr Ala Leu Asn Val Tyr Leu Phe Gly Ala Thr Arg
                485                 490                 495

Glu Val Val Arg Thr Gln Ser Val Lys Val Val Glu Lys His Val Pro
            500                 505                 510

Ile Val Ile Glu Lys Pro Ser Glu Lys Glu Glu Asp Thr Ser Ser Glu
        515                 520                 525

Asp Ser Ile Glu Leu Thr Val Gly Lys Gln Pro Lys Pro Val Thr Glu
    530                 535                 540

Thr Arg Ser Leu Asp Asp Leu Glu Ala Ile Met Lys Ala Gly Lys Thr
545                 550                 555                 560

Lys Leu Leu Glu Asp His Glu Val Val Lys Leu Ser Leu Glu Gly Lys
                565                 570                 575

Leu Pro Leu Tyr Ala Leu Glu Lys Gln Leu Gly Asp Asn Thr Arg Ala
            580                 585                 590

Val Gly Ile Arg Arg Ser Ile Ile Ser Gln Gln Ser Asn Thr Lys Thr
        595                 600                 605

Leu Glu Thr Ser Lys Leu Pro Tyr Leu His Tyr Asp Tyr Asp Arg Val
    610                 615                 620

Phe Gly Ala Cys Cys Glu Asn Val Ile Gly Tyr Met Pro Leu Pro Val
625                 630                 635                 640

Gly Val Ala Gly Pro Met Asn Ile Asp Gly Lys Asn Tyr His Ile Pro
                645                 650                 655

Met Ala Thr Thr Glu Gly Cys Leu Val Ala Ser Thr Met Arg Gly Cys
            660                 665                 670

Lys Ala Ile Asn Ala Gly Gly Gly Val Thr Thr Val Leu Thr Gln Asp
        675                 680                 685

Gly Met Thr Arg Gly Pro Cys Val Ser Phe Pro Ser Leu Lys Arg Ala
    690                 695                 700

Gly Ala Ala Lys Ile Trp Leu Asp Ser Glu Glu Gly Leu Lys Ser Met
705                 710                 715                 720

Arg Lys Ala Phe Asn Ser Thr Ser Arg Phe Ala Arg Leu Gln Ser Leu
                725                 730                 735

His Ser Thr Leu Ala Gly Asn Leu Leu Phe Ile Arg Phe Arg Thr Thr
            740                 745                 750

Thr Gly Asp Ala Met Gly Met Asn Met Ile Ser Lys Gly Val Glu His
        755                 760                 765

Ser Leu Ala Val Met Val Lys Glu Tyr Gly Phe Pro Asp Met Asp Ile
    770                 775                 780

Val Ser Val Ser Gly Asn Tyr Cys Thr Asp Lys Lys Pro Ala Ala Ile
785                 790                 795                 800

Asn Trp Ile Glu Gly Arg Gly Lys Ser Val Val Ala Glu Ala Thr Ile
                805                 810                 815

Pro Ala His Ile Val Lys Ser Val Leu Lys Ser Glu Val Asp Ala Leu
            820                 825                 830

Val Glu Leu Asn Ile Ser Lys Asn Leu Ile Gly Ser Ala Met Ala Gly
        835                 840                 845

Ser Val Gly Gly Phe Asn Ala His Ala Ala Asn Leu Val Thr Ala Ile
    850                 855                 860

Tyr Leu Ala Thr Gly Gln Asp Pro Ala Gln Asn Val Glu Ser Ser Asn
865                 870                 875                 880

Cys Ile Thr Leu Met Ser Asn Val Asp Gly Asn Leu Leu Ile Ser Val
                885                 890                 895

Ser Met Pro Ser Ile Glu Val Gly Thr Ile Gly Gly Gly Thr Ile Leu
            900                 905                 910

Glu Pro Gln Gly Ala Met Leu Glu Met Leu Gly Val Arg Gly Pro His
        915                 920                 925

Ile Glu Thr Pro Gly Ala Asn Ala Gln Gln Leu Ala Arg Ile Ile Ala
    930                 935                 940

Ser Gly Val Leu Ala Ala Glu Leu Ser Leu Cys Ser Ala Leu Ala Ala
945                 950                 955                 960

Gly His Leu Val Gln Ser His Met Thr His Asn Arg Ser Gln Ala Pro
                965                 970                 975

Thr Pro Ala Lys Gln Ser Gln Ala Asp Leu Gln Arg Leu Gln Asn Gly
            980                 985                 990

Ser Asn Ile Cys Ile Arg Ser
        995

<210>  7
<211>  20
<212>  DNA
<213>  Yarrowia lipolytica

<400>  7
tccagaagcg ctacaaagag                                                  20

<210>  8
<211>  38
<212>  DNA
<213>  Artificial Sequence

<220>
<223>  primer

<400>  8
attaccctgt tatccctatg aaggttgcag tcgtagtc                              38

<210>  9
<211>  38
<212>  DNA
<213>  Artificial Sequence

<220>
<223>  primer

<400>  9
tagggataac agggtaattg cgatctcgat gagtgatg                              38

<210>  10
<211>  20
<212>  DNA
<213>  Yarrowia lipolytica

<400>  10
gcccagaaga ttggaatgac                                                   20


<210>  11
<211>  31
<212>  DNA
<213>  Artificial Sequence

<220>
<223>  primer

<400>  11
atataccgcg ggattccgcc aagtgagact g                                      31


<210>  12
<211>  38
<212>  DNA
<213>  Artificial Sequence

<220>
<223>  primer

<400>  12
attaccctgt tatccctacg tcgaggaagt aggtcatc                               38


<210>  13
<211>  38
<212>  DNA
<213>  Artificial Sequence

<220>
<223>  primer

<400>  13
tagggataac agggtaatgc gagcttgatg aggaatag                               38


<210>  14
<211>  31
<212>  DNA
<213>  Artificial Sequence

<220>
<223>  primer

<400>  14
atataccgcg gcctgacgcc aatttgaaga g                                      31


<210>  15
<211>  31
<212>  DNA
<213>  Artificial Sequence

<220>
<223>  primer

<400>  15
atataccgcg gctgggctgt tcggtcgata g                                      31

<210>  16
<211>  38
<212>  DNA
<213>  Artificial Sequence

<220>
<223>  primer

<400>  16
tagggataac agggtaatag gacgcacaac gccatcac                              38


<210>  17
<211>  36
<212>  DNA
<213>  Artificial Sequence

<220>
<223>  primer

<400>  17
attaccctgt tatccctacg ctcccattgg aaacta                                36


<210>  18
<211>  31
<212>  DNA
<213>  Artificial Sequence

<220>
<223>  primer

<400>  18
atataccgcg gtctcttcgc tgtggtctag g                                     31


<210>  19
<211>  31
<212>  DNA
<213>  Artificial Sequence

<220>
<223>  primer

<400>  19
atataccgcg gtccaccgtt ctccttcata c                                     31


<210>  20
<211>  38
<212>  DNA
<213>  Artificial Sequence

<220>
<223>  primer

<400>  20
tagggataac agggtaatat gtctctaggg tcgaagtc                              38


<210>  21
<211>  38

<212>  DNA
<213>  Artificial Sequence

<220>
<223>  primer

<400>  21
attaccctgt tatccctatg gcaagcctca ctactacg                              38


<210>  22
<211>  31
<212>  DNA
<213>  Artificial Sequence

<220>
<223>  primer

<400>  22
atataccgcg gtgcggcgga actactgtat c                                     31


<210>  23
<211>  31
<212>  DNA
<213>  Artificial Sequence

<220>
<223>  primer

<400>  23
atataccgcg ggggattctc cgggttattt g                                      31


<210>  24
<211>  38
<212>  DNA
<213>  Artificial Sequence

<220>
<223>  primer

<400>  24
tagggataac agggtaatac gtctcggacc ttgaattg                              38


<210>  25
<211>  38
<212>  DNA
<213>  Artificial Sequence

<220>
<223>  primer

<400>  25
attaccctgt tatccctacc ttcaacctgt ccgacttc                              38


<210>  26
<211>  31
<212>  DNA
<213>  Artificial Sequence

<220>
<223>  primer

<400>  26
atataccgcg ggaagcggtc ctcgttgtat g                                    31


<210>  27
<211>  20
<212>  DNA
<213>  Yarrowia lipolytica

<400>  27
gtgtagcaac tcggatacag                                                 20


<210>  28
<211>  38
<212>  DNA
<213>  Artificial Sequence

<220>
<223>  primer

<400>  28
tagggataac agggtaatgg tccataagca gagtgttc                             38


<210>  29
<211>  38
<212>  DNA
<213>  Artificial Sequence

<220>
<223>  primer

<400>  29
attaccctgt tatccctaac cctcgacctc cttattac                             38


<210>  30
<211>  20
<212>  DNA
<213>  Yarrowia lipolytica

<400>  30
ctcttcttga ctggcatagc                                                 20


<210>  31
<211>  31
<212>  DNA
<213>  Artificial Sequence

<220>
<223>  primer

<400>  31
atataaagct tgcggctcgg cacgtgatct g                                    31


<210>  32
<211>  32

<212>  DNA
<213>  Artificial Sequence

<220>
<223>  primer

<400>  32
atataggatc catcgtgcgt acgtcgctag tg                                    32


<210>  33
<211>  31
<212>  DNA
<213>  Artificial Sequence

<220>
<223>  primer

<400>  33
atataggatc ccgaccggca cgatcaattg g                                     31


<210>  34
<211>  32
<212>  DNA
<213>  Artificial Sequence

<220>
<223>  primer

<400>  34
atatacatat gggtgcatct cagcccgacc tc                                    32


<210>  35
<211>  20
<212>  DNA
<213>  Yarrowia lipolytica

<400>  35
tcccgaaaca cagaacttcc                                                  20


<210>  36
<211>  32
<212>  DNA
<213>  Artificial Sequence

<220>
<223>  primer

<400>  36
atataaagct tggtggacgt tccggcagac ag                                    32


<210>  37
<211>  32
<212>  DNA
<213>  Artificial Sequence

<220>
<223>  primer

<400>  37
atataggatc cctcccaggc atctccacac tc                                    32


<210>  38
<211>  31
<212>  DNA
<213>  Artificial Sequence

<220>
<223>  primer

<400>  38
atataggatc ccacttacag gcttagcaag g                                     31


<210>  39
<211>  32
<212>  DNA
<213>  Artificial Sequence

<220>
<223>  primer

<400>  39
atatagcggc cgcggaaatc acgcttgatt cg                                    32


<210>  40
<211>  20
<212>  DNA
<213>  Yarrowia lipolytica

<400>  40
taggcgctgg tacagaagag                                                  20


<210>  41
<211>  31
<212>  DNA
<213>  Artificial Sequence

<220>
<223>  primer

<400>  41
atataaagct tgagtacagt aggtggtact c                                     31


<210>  42
<211>  31
<212>  DNA
<213>  Artificial Sequence

<220>
<223>  primer

<400>  42
atataggatc cagtggtggt ggtggtggta g                                     31


<210>  43
<211>  31

<212>  DNA
<213>  Artificial Sequence

<220>
<223>  primer

<400>  43
atataggatc ctttacgtgc aacaggagga g                                    31


<210>  44
<211>  31
<212>  DNA
<213>  Artificial Sequence

<220>
<223>  primer

<400>  44
atatacatat ggcctgtctt gagttctttg g                                    31


<210>  45
<211>  20
<212>  DNA
<213>  Yarrowia lipolytica

<400>  45
agggtcgtag ataacgagtc                                                 20


<210>  46
<211>  32
<212>  DNA
<213>  Artificial Sequence

<220>
<223>  primer

<400>  46
atataaagct tcacgtggct gctgggccaa cc                                   32


<210>  47
<211>  31
<212>  DNA
<213>  Artificial Sequence

<220>
<223>  primer

<400>  47
atataggatc ccgcacggta tcggagcatc g                                    31


<210>  48
<211>  31
<212>  DNA
<213>  Artificial Sequence

<220>
<223>  primer

<400>  48
atataggatc ccgcggctat tgacgctgag g                                    31


<210>  49
<211>  34
<212>  DNA
<213>  Artificial Sequence

<220>
<223>  primer

<400>  49
atatacatat gcccgtcagc tccatcgacg agtg                                 34


<210>  50
<211>  23
<212>  DNA
<213>  Artificial Sequence

<220>
<223>  primer

<400>  50
   aggtgtactg tagccaccct gac                                            23


<210>  51
<211>  31
<212>  DNA
<213>  Artificial Sequence

<220>
<223>  primer

<400>  51
atataaagct tccggccagc cgctggcaac g                                    31


<210>  52
<211>  31
<212>  DNA
<213>  Artificial Sequence

<220>
<223>  primer

<400>  52
atataggatc cacacgacag ctgcacctga c                                    31


<210>  53
<211>  31
<212>  DNA
<213>  Artificial Sequence

<220>
<223>  primer

<400>  53
atataggatc ccagccatga gccaggcatt g                                    31

<210>  54
<211>  33
<212>  DNA
<213>  Artificial Sequence

<220>
<223>  primer

<400>  54
atatacatat gggcgccagc cacatttgcc ctc                                    33


<210>  55
<211>  20
<212>  DNA
<213>  Yarrowia lipolytica

<400>  55
agcgatacag cagttgactc                                                   20


<210>  56
<211>  20
<212>  DNA
<213>  Yarrowia lipolytica

<400>  56
tcagccgtct acttgtagag                                                   20


<210>  57
<211>  31
<212>  DNA
<213>  Artificial Sequence

<220>
<223>  primer

<400>  57
atataggatc cgtggctcgg atactcctga c                                      31


<210>  58
<211>  31
<212>  DNA
<213>  Artificial Sequence

<220>
<223>  primer

<400>  58
atataggatc cagccggagg tcagatcaag c                                      31


<210>  59
<211>  32
<212>  DNA
<213>  Artificial Sequence

<220>
<223>  primer

<400>  59
atatacatat ggcgcaatag ttcgccggcc tg                                    32


<210>  60
<211>  20
<212>  DNA
<213>  Yarrowia lipolytica

<400>  60
ctcgtgttgt gcctttcttg                                                  20


<210>  61
<211>  34
<212>  DNA
<213>  Artificial Sequence

<220>
<223>  primer

<400>  61
atataaagct tgcgcgacaa cccatagcga tggc                                  34


<210>  62
<211>  31
<212>  DNA
<213>  Artificial Sequence

<220>
<223>  primer

<400>  62
atataggatc cgataagagg gcgctctgac c                                     31


<210>  63
<211>  30
<212>  DNA
<213>  Artificial Sequence

<220>
<223>  primer

<400>  63
atataggatc cggcgtgaca tcgagtttgg                                       30


<210>  64
<211>  31
<212>  DNA
<213>  Artificial Sequence

<220>
<223>  primer

<400>  64
atatacatat gctacgtctc gccgcagagg g                                     31


<210>  65
<211>  20

<212>  DNA
<213>  Yarrowia lipolytica

<400>  65
agcgagaggt tatacggaag                                                    20


<210>  66
<211>  20
<212>  DNA
<213>  Yarrowia lipolytica

<400>  66
ctcctacagc ctctcaagac                                                    20


<210>  67
<211>  20
<212>  DNA
<213>  Yarrowia lipolytica

<400>  67
gtctacaaga cagcccagag                                                    20


<210>  68
<211>  20
<212>  DNA
<213>  Yarrowia lipolytica

<400>  68
ccgcttgaga agagcaatac                                                    20


<210>  69
<211>  31
<212>  DNA
<213>  Artificial Sequence

<220>
<223>  primer

<400>  69
atataaagct tcgccacctg tccacgtctc g                                       31


<210>  70
<211>  31
<212>  DNA
<213>  Artificial Sequence

<220>
<223>  primer

<400>  70
atataggatc cgcgaagcga cgtgtggtga g                                       31


<210>  71
<211>  31
<212>  DNA
<213>  Artificial Sequence

<220>
<223>  primer

<400>  71
atataggatc cgctgagcac gcgagtacac c                                    31


<210>  72
<211>  31
<212>  DNA
<213>  Artificial Sequence

<220>
<223>  primer

<400>  72
atatagaatt cgatctgtcg tacaactaag g                                    31


<210>  73
<211>  20
<212>  DNA
<213>  Yarrowia lipolytica

<400>  73
cagaagttac gacgccaagg                                                 20


<210>  74
<211>  31
<212>  DNA
<213>  Artificial Sequence

<220>
<223>  primer

<400>  74
atataaagct tccgtaataa atgcccgtct c                                    31


<210>  75
<211>  31
<212>  DNA
<213>  Artificial Sequence

<220>
<223>  primer

<400>  75
atataggatc caggtcgatt tcggcgctgt g                                    31


<210>  76
<211>  31
<212>  DNA
<213>  Artificial Sequence

<220>
<223>  primer

<400>  76
atataggatc cggttaggca aatagctaat g                                    31

<210>  77
<211>  31
<212>  DNA
<213>  Artificial Sequence

<220>
<223>  primer

<400>  77
atatagaatt cgcctggagc gagtttctga g                                    31


<210>  78
<211>  20
<212>  DNA
<213>  Yarrowia lipolytica

<400>  78
tcgcaagggc catagaggtg                                                 20


<210>  79
<211>  31
<212>  DNA
<213>  Artificial Sequence

<220>
<223>  primer

<400>  79
atataaagct tccttgcaca tctccgatga c                                    31


<210>  80
<211>  31
<212>  DNA
<213>  Artificial Sequence

<220>
<223>  primer

<400>  80
atataggatc cggtgttacg ccaccacgtt g                                    31


<210>  81
<211>  31
<212>  DNA
<213>  Artificial Sequence

<220>
<223>  primer

<400>  81
atataggatc cgggacctgc gatacgaatg c                                    31


<210>  82
<211>  31
<212>  DNA
<213>  Artificial Sequence

<220>
<223>  primer

<400>  82
atatagaatt cgcttgcgca gccggtgtat c                                    31


<210>  83
<211>  20
<212>  DNA
<213>  Yarrowia lipolytica

<400>  83
ggcgttgtgg agctatcacc                                                 20


<210>  84
<211>  31
<212>  DNA
<213>  Artificial Sequence

<220>
<223>  primer

<400>  84
atataaagct tgcgtgcggt gcgtgcgtat g                                    31


<210>  85
<211>  31
<212>  DNA
<213>  Artificial Sequence

<220>
<223>  primer

<400>  85
atataggatc ccagcaccac gaaattatac g                                    31


<210>  86
<211>  31
<212>  DNA
<213>  Artificial Sequence

<220>
<223>  primer

<400>  86
atataggatc cgtgcgctta catgtggacc g                                    31


<210>  87
<211>  31
<212>  DNA
<213>  Artificial Sequence

<220>
<223>  primer

<400>  87
atatagaatt ccaggcagct tcttgccaat g                                    31

<210>  88
<211>  20
<212>  DNA
<213>  Yarrowia lipolytica

<400>  88
gagataggag gttcccatac                                                    20


<210>  89
<211>  20
<212>  DNA
<213>  Yarrowia lipolytica

<400>  89
ctacgcttgt tcagactttg                                                    20


<210>  90
<211>  46
<212>  DNA
<213>  Artificial Sequence

<220>
<223>  primer

<400>  90
gtgtgcttgt cgtcagacat tttgaatgat tcttatactc agaagg                       46


<210>  91
<211>  46
<212>  DNA
<213>  Artificial Sequence

<220>
<223>  primer

<400>  91
ccttctgagt ataagaatca ttcaaaatgt ctgacgacaa gcacac                       46


<210>  92
<211>  26
<212>  DNA
<213>  Artificial Sequence

<220>
<223>  primer

<400>  92
gcatgcttag attcgaggtc ggagat                                             26


<210>  93
<211>  26
<212>  DNA
<213>  Artificial Sequence

<220>
<223>  primer

<400>  93
ggtaccacgc acggatagtt tatcca                                            26


<210>  94
<211>  31
<212>  DNA
<213>  Artificial Sequence

<220>
<223>  primer

<400>  94
ggtacccaaa gtcaactaat gtcaagtaaa g                                      31


<210>  95
<211>  17
<212>  DNA
<213>  Yarrowia lipolytica

<400>  95
cctccaacgt gactttc                                                      17


<210>  96
<211>  17
<212>  DNA
<213>  Yarrowia lipolytica

<400>  96
agagacctcc cacaaag                                                      17


<210>  97
<211>  46
<212>  DNA
<213>  Artificial Sequence

<220>
<223>  primer

<400>  97
agagagtcga gtagagccat tttgaatgat tcttatactc agaagg                      46


<210>  98
<211>  46
<212>  DNA
<213>  Artificial Sequence

<220>
<223>  primer

<400>  98
ccttctgagt ataagaatca ttcaaaatgg ctctactcga ctctct                      46


<210>  99
<211>  35
<212>  DNA
<213>  Yarrowia lipolytica

<400>  99
atatagcatg cctaccacac atcttcctgg tagac                                35

<210>  100
<211>  18
<212>  DNA
<213>  Yarrowia lipolytica

<400>  100
ctcaagatac ggcattgg                                                   18

<210>  101
<211>  18
<212>  DNA
<213>  Yarrowia lipolytica

<400>  101
tccttggcta gacgaatg                                                   18

1

## Beschreibung

Die Erfindung betrifft verschiedene Hefestämme der Art *Yarrowia lipolytica* sowie zugehörige Verfahren zur biokatalytischen Herstellung von ω-Hydroxyfettsäuren oder Dicarbonsäuren mithilfe dieser Stämme.

Bei Dicarbonsäuren handelt es sich um Carbonsäuren mit zwei Carboxy-Gruppen (allgemeine Struktur: HOOC-(CH₂)ₙ-COOH), die vielfältige Anwendungen in der chemischen Industrie, z.B. zur Herstellung von Duftstoffen, Klebstoffen, Nylon und anderen Polyamiden, Harzen, Korrosionsinhibitoren oder Schmierstoffen, finden. Insbesondere die biotechnologische Produktion langkettiger Dicarbonsäuren ist von besonderem Interesse, da bei der chemischen Synthese der selbigen eine Reihe an unerwünschten Nebenprodukten gebildet wird.

ω-Hydroxyfettsäuren enthalten am C_{ω}-Atom (C-Atom mit der weitest möglichen Entfernung zur Carboxygruppe) eine Hydroxylgruppe (allgemeine Struktur: HOOC-(CH₂)ₙ-CH₂OH). ω-Hydroxyfettsäuren sind ebenfalls von großer Bedeutung in der chemischen Industrie, da sie u.a. in Schmiermitteln, Klebstoffen, Kosmetika und Krebstherapeutika Anwendung finden. Weiterhin könnten ω-Hydroxyfettsäuren in Zukunft eine große Bedeutung erlangen, da sie als Monomere zur Synthese von Biokunststoffen genutzt werden können.

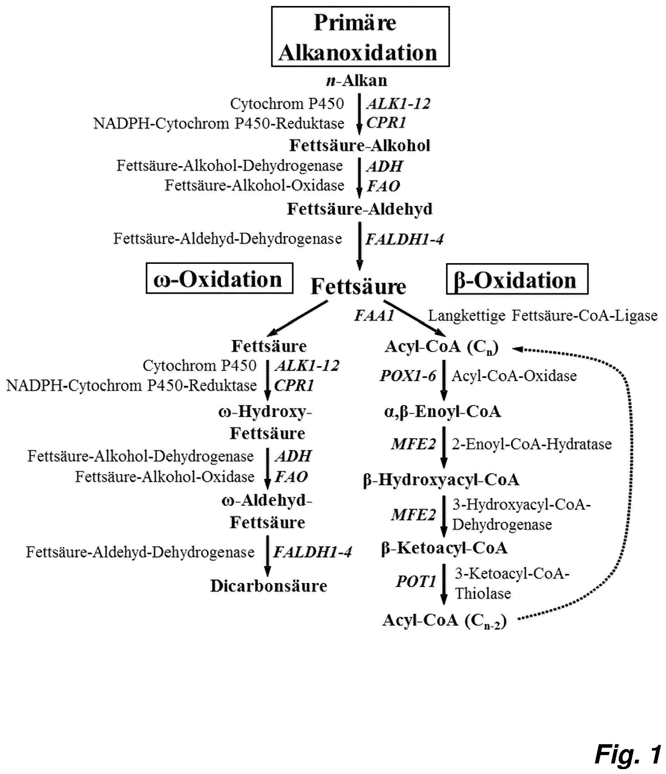

Zur Gewinnung von ω-Hydroxyfettsäuren und Dicarbonsäuren aus *n*-Alkanen und Fettsäuren soll im Rahmen dieser Erfindung die nicht-konventionelle Hefe *Yarrowia* (*Y.*) *lipolytica* genutzt werden. Die Hefe *Y. lipolytica* wurde als Wirtsorganismus für die Biokonversion ausgewählt, weil sie in der Lage ist eine Vielzahl an Substraten als Kohlenstoffquelle zu nutzen. Hierzu zählen neben Glucose, Glycerol, Proteinen, Alkoholen und Acetat auch eine Vielzahl an hydrophoben Substraten wie Pflanzenöle, Fette, Fettsäuren und *n*-Alkane (Barth G & Gaillardin C (1997) FEMS Microbiol Rev 19: 219-237.). Die hydrophoben Substrate werden durch die Hefen emulgiert und mithilfe spezialisierter Membrantransporter in das Zellinnere aufgenommen (Fickers P, Benetti PH, Wache Y, Marty A, Mauersberger S, Smit MS & Nicaud JM (2005) FEMS Yeast Res 5: 527-543.). In die Zelle aufgenommene *n*-Alkane werden im Rahmen der primären (monoterminalen) Alkanoxidation schrittweise zur Fettsäure mit gleicher Kettenlänge umgesetzt (Fig. 1). Die Fettsäuren werden anschließend im Rahmen der β-Oxidation zu Acetyl-CoA abgebaut, das in den Tricarbonsäure- und den Glyoxylatzyklus einfließt. Parallel zur β-Oxidation von Fettsäuren in den Peroxisomen findet die ω-Oxidation im Endoplasmatischen Retikulum statt (vgl. Fig. 1). Diese diterminale Oxidation von Fettsäuren findet natürlicher Weise jedoch in einem weitaus geringeren Umfang als die β-Oxidation statt.

Verschiedene Hefen, wie z.B. *Candida* (*C.*) *tropicalis* und *Y. lipolytica* sind in der Lage, langkettige Alkane oder Fettsäuren zu α,ω-Dicarbonsäuren umzusetzen. Damit die hydrophoben Substrate überhaupt zur jeweiligen Dicarbonsäure umgesetzt werden können, muss deren Verstoffwechselung im Rahmen der β-Oxidation unterbunden werden. Dies kann z.B. durch die Deletion der für die Acyl-CoA-Oxidase kodierenden POX-Gene geschehen. Ist die β-Oxidation ausgeschaltet, führt dies zu einer gesteigerten ω-Oxidation der Fettsäuren (Smit et al. (2005) Biotechnol. Lett. 27: 859-864.).

Die mikrobielle Produktion langkettiger Dicarbonsäuren wurde bereits mithilfe der Hefe *C. tropicalis* durchgeführt:
- Picataggio S, Rohrer T, Deanda K, Lanning D, Reynolds R, Mielenz J & Eirich LD (1992) Biotechnology (N Y) 10: 894-898.

Die Produktion der Dicarbonsäuren wurde hierbei vor allem durch die Deletion der Gene *POX4* und *POX5* erreicht. Es bestehen eine Reihe von Patenten, die entsprechende C. tropicalis-Produktionsstämme sowie zugehörige Produktionsverfahren schützen (z.B. US4339536A, EP296506A2 und WO200017380A1).

In einer Patentschrift von 2013 wird beschrieben, wie Hefen allgemein (in an sich bekannter Weise) zur Produktion erhöhter Mengen an Dicarbonsäuren veranlasst werden können. In den Ausführungsbeispielen wird hier jedoch im Speziellen nur auf *C. tropicalis* eingegangen (WO201306730A2, WO201306733A2).

Ebenso wurde die mikrobielle Produktion langkettiger ω-Hydroxyfettsäuren bereits mithilfe der Hefe *C. tropicalis* durchgeführt:
- Lu W, Ness JE, Xie W, Zhang X, Minshull J & Gross RA (2010) J Am Chem Soc 132: 15451-15455.

Hierfür wurden zusätzlich zu den Genen *POX4* und *POX5* sechs Cytochrom P450-, vier Alkohol-Oxidase- und sechs Alkohol-Dehydrogenase-Gene deletiert. Auch diese Stämme und Produktionsverfahren sind bereits patentrechtlich geschützt (WO2011008232A2).

Des Weiteren wurde bereits beschrieben, wie *Y. lipolytica* zur Bildung von Dicarbonsäuren veranlasst werden kann:
- Smit MS, Mokgoro MM, Setati E & Nicaud JM (2005) Biotechnol Lett 27: 859-864.

Hierbei wurden alle derzeit bekannten Acyl-CoA-Oxidase-Gene (*POX1, POX2, POX3, POX4, POX5 und POX6)* sowie das Acyl-CoA-Diacylglycerol-Acyltransferase-Gen (*DGA1*) und das Lecithin-Cholesterol-Acyltransferase-Gen (*LRO1*) deletiert. Weiterhin wurde das NADPH-Cytochrom P450-Reduktase-Gen (*CPR1*) überexprimiert (WO200664131A8).

Neben der biotechnologischen Produktion von ω-Hydroxyfettsäuren und Dicarbonsäuren mithilfe von gentechnisch veränderten Hefen unter Ausnutzung der intrazellulären ω-Oxidation von Fettsäuren gibt es auch weitere biotechnologische Verfahren, die auf anderen enzymatischen Umsetzungen beruhen. Ein Beispiel hierfür stellt die Verwendung der Baeyer-Villiger-Monooxygenase, die die Umwandlung eines Ketons zum Ester katalysiert, dar (WO2013151393A1).

Aufgabe der vorliegenden Erfindung ist die Bereitstellung neuartiger Hefestämme der Art *Y. lipolytica* die zur mikrobiellen Produktion von ω-Hydroxyfettsäuren oder Dicarbonsäuren, in größeren Mengen als bisher bekannt, geeignet sind sowie eines Verfahren zur Kultivierung ebendieser Stämme.

Die Aufgabe wird durch einen Hefestamm gemäß Anspruch 1 sowie durch Verfahren gemäß den Ansprüchen 8 und 11 gelöst. Vorteilhafte Ausgestaltungen sind in den abhängigen Ansprüchen angegeben.

Gemäß eines ersten Aspekts der Erfindung wird zur mikrobiellen Produktion von *ω-*Hydroxyfettsäuren aus hydrophoben Substraten ein Hefestamm der Art *Y. lipolytica* bereitgestellt, der eine Verringerung der Aktivitäten der Acyl-CoA-Oxidasen (namentlich *POX1*: YALI0E32835g, *POX2*: YALI0F10857g, *POX3*: YALl0D24750g, *POX4:* YALI0E27654g, *POX5*: YALI0C23859g, *POX6*: YALI0E06567g) aufweist, wobei in diesem Hefestamm zusätzlich die Aktivitäten relevanter (Fett-)Alkohol-Dehydrogenasen (=(Fettsäure-)Alkohol-Dehydrogenasen, namentlich ausgewählt aus *FADH*: YALI0F09603g, *ADH1:* YALI0D25630g, *ADH2:* YALI0E17787g, *ADH3:* YALI0A16379g, *ADH4:* YALl0E15818g, *ADH5:* YALl0D02167g, *ADH6:* YALI0A15147g und/oder *ADH7*: YALI0E07766g) reduziert sind und die Aktivitäten relevanter (Fett-)Alkohol-Oxidasen (=(Fettsäure-)Alkohol-Oxidasen, namentlich *FAO1*: YALI0B14014g) reduziert sind, was zu einer gesteigerten Bildung von ω-Hydroxyfettsäuren, vorzugsweise lineare ω-Hydroxyfettsäuren gemäß der allgemeinen Struktur: HOOC-(CH₂)ₙ-CH₂OH, HOOC-(CₙH₂ₙ-₂)-CH₂OH, COOC-(CₙH₂ₙ₋₄)-CH₂OH oder HOOC-(CₙH₂ₙ₋₆)-CH₂OH, mit n im Bereich von 6 bis 18, vorzugsweise im Bereich von 8 bis 16, aus einem hydrophoben Substrat, insbesondere einem entsprechenden *n*-Alkan oder einer Fettsäure (und anderen hydrophoben Substraten) führt.

Besonders bevorzugt ist die ω-Hydroxyfettsäure eine lineare ω-Hydroxyfettsäure gemäß der allgemeinen Struktur: HOOC-(CH₂)ₙ-CH₂OH mit n im Bereich von 8 bis 16.

Die Bildung von ω-Hydroxyfettsäuren bzw. Dicarbonsäuren ist im Sinne der vorliegenden Erfindung gesteigert, wenn diese gegenüber Hefezellen ohne die entsprechende gentechnische Veränderung um mindestens 10 Mol.-%, besonders bevorzugt mindestens 20 Mol.-% erhöht ist.

Der Erfindung liegt die jüngst gewonnene Erkenntnis zugrunde, dass das Gen, welches für die Fett-Alkohol-Oxidase *FAO1:* YALI0B14014g in dem Hefestamm der Art *Y. lipolytica* kodiert, identifiziert werden konnte.

Die Erfinder haben zudem nun überraschend gefunden, dass die Deletion des für das Enzym Fao1p kodierenden Gens die weitere ω-Oxidation der ω-Hydroxyfettsäuren zum Fettsäurealdehyd, bzw. zur Dicarbonsäure im Rahmen der ω-Oxidation von Fettsäuren vermindert.

Besonders vorteilhaft unterbindet oder verringert die Verringerung der Aktivität der Produkte von *FADH, ADH1, ADH2, ADH3, ADH4, ADH5, ADH6, ADH7* und/oder *FAO1* die weitere Oxidation der ω-Hydroxyfettsäuren zum Fettsäurealdehyd, bzw. zur Dicarbonsäure im Rahmen der ω-Oxidation von Fettsäuren.

Das Fett-Alkohol-Oxidase-Gen wurde in *Y. lipolytica* bisher noch nicht beschrieben. Dessen Nutzung im Rahmen von biotechnologischen Anwendungsfeldern stellt ebenfalls einen Teil dieser Erfindung dar.

Unter einer Verringerung der Aktivität eines Proteins ist vorzugsweise zu verstehen, dass die Aktivität des Proteins pro Zelle verringert wird. Besonders bevorzugt ist darunter das Fehlen der Aktivität des Proteins in der Hefezelle zu verstehen.
Die Verringerung der Aktivität von Proteinen kann durch verschiedene, dem Fachmann bekannte Verfahren erlangt werden, wie bspw.:
(i) durch die Inhibierung oder Verminderung der Expression der dafür kodierenden Gene
(ii) durch partielle oder komplette Deletion der dafür kodierenden Gene
(iii) durch Expression von nicht-funktionalen Genen
(iv) durch Inhibierung oder Verminderung der Aktivität der exprimierten Gene.

Die Inhibierung oder Verminderung der Expression eines für ein Protein kodierenden Gens kann beispielsweise durch Inhibierung oder Verminderung der Transkription des kodierenden Gens oder der Translation der gebildeten mRNA erfolgen. Die Deletion der kodierenden Gene kann beispielsweise durch einen Ausbau der Gene mittels Deletionskassetten durchgeführt werden. Die Expression eines dysfunktionalen oder aktivitätsreduzierten Genproduktes kann beispielsweise durch Insertion, Substitution oder Punktmutation in dem für das Protein kodierenden Gen bewerkstelligt werden.

Bei der Aktivitätsverringerung wird die Deletion eines kodierenden Gens bevorzugt.

Vorzugsweise weist der erfindungsgemäße Hefestamm zur Produktion von ω-Hydroxyfettsäuren somit eine Deletion der Gene für die Acyl-CoA-Oxidasen (*POX1, POX2, POX3, POX4, POX5* und *POX6*) sowie für die (Fett-)Alkohol-Dehydrogenasen *(FADH, ADH1, ADH2, ADH3, ADH4, ADH5, ADH6, ADH7*) und/oder für die (Fett-)Alkohol-Oxidasen (*FAO1*) auf. Weiterhin ist die Bildung der Fettkörperchen verringert, vorzugsweise durch die Verringerung der Aktivität der Genprodukte von *PAH1* und/oder *SCT1.*

Die Deletion der Gene *POX1, POX2, POX3, POX4, POX5* und *POX6* unterbindet die β-Oxidation und verhindert somit den Abbau von Fettsäuren (zu Acetyl-CoA, bzw. Propionyl-CoA).

Bevorzugt sind in dem erfindungsgemäßen Hefestamm zur mikrobiellen Herstellung von ω-Hydroxyfettsäuren zusätzlich die Aktivitäten einzelner oder aller Cytochrom P450-Genprodukte, insbesondere von *ALK1*: YALI0E25982g, *ALK2:* YALI0F01320g, *ALK3:* YALl0A20130g, *ALK4*: YALl0B13816g, *ALK5*: YALl0B13838g, *ALK6*: YALI0B01848g, *ALK7*: YALI0A15488g, *ALK8*: YALI0C12122g, *ALK9*: YALI0B06248g, *ALK10:* YALI0B20702g, *ALK11*: YALI0C10054g und/oder *ALK12*: YALI0A20130g erhöht. Vorteilhaft führt die Erhöhung einzelner oder aller Cytochrom P450-Genprodukte zu einer gesteigerten Bildung von ω-Hydroxyfettsäuren aus dem entsprechenden hydrophoben Substrat, insbesondere *n*-Alkanen und Fettsäuren.

Es kann vorteilhaft vorgesehen sein, dass in dem erfindungsgemäßen Hefestamm zur mikrobiellen Herstellung von ω-Hydroxyfettsäuren zusätzlich die Aktivität der NADPH-Cytochrom P450-Reduktase (*CPR1*: YALl0D04422g) erhöht ist, was zu einer gesteigerten Bildung von ω-Hydroxyfettsäuren aus dem entsprechenden hydrophoben Substrat, insbesondere *n*-Alkanen und Fettsäuren führt.

Bevorzugt ist in dem erfindungsgemäßen Hefestamm zur mikrobiellen Herstellung von ω-Hydroxyfettsäuren zusätzlich die Aktivität der Phosphatidsäure-Dephosphohydrolase (*PAH1*: YALl0D27016p) verringert. Vorteilhaft führt dies zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren.

Nach einer bevorzugten Ausgestaltung der vorliegenden Erfindung ist in dem erfindungsgemäßen Hefestamm zur mikrobiellen Herstellung von ω-Hydroxyfettsäuren zusätzlich die Aktivität der Glycerol-3-phosphat-Acyltransferase (*SCT1*: YALI0C00209g) verringert, was vorteilhaft zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren (und anderen hydrophoben Substraten) führt.

Es kann ferner auch vorgesehen sein, dass in dem erfindungsgemäßen Hefestamm zur mikrobiellen Herstellung von ω-Hydroxyfettsäuren zusätzlich die Aktivität der Phospholipid-Diacylglycerol-Acyltransferase (*LRO1*: YALI0E16797g) verringert ist, was zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren führt.

Nach einer bevorzugten Ausgestaltung der vorliegenden Erfindung ist in dem erfindungsgemäßen Hefestamm zur mikrobiellen Herstellung von ω-Hydroxyfettsäuren zusätzlich die Aktivität der Diacylglycerol-Acyltransferase (*DGA1*: YALI0E32769g) verringert, was zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren führt.

Nach einer bevorzugten Ausgestaltung der vorliegenden Erfindung ist in dem erfindungsgemäßen Hefestamm zur mikrobiellen Herstellung von ω-Hydroxyfettsäuren zusätzlich die Aktivität der Glycerol-3-phosphat-Dehydrogenase (*GUT2*: YALI0B13970g) erhöht ist, was zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren führt.

Besonders vorteilhaft wurde nun gefunden, dass die zusätzlich Erhöhung der Aktivitäten relevanter Triacylglycerol-Lipasen (*TGL3*/*TGL4*: YALI0F10010g, YALI0D16379g, YALI0D17534g) in dem erfindungsgemäßen Hefestamm zur mikrobiellen Herstellung von ω-Hydroxyfettsäuren, zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren führt.

Besonders vorteilhaft hat sich die die Verringerung der Aktivität des Citrat- und Oxalacetat-Transporters (*YHM2*: YALI0B10736g) und/oder die Verringerung der Aktivität des Citrat-Transport-Proteins (*CTP1*: YALI0F26323g) in dem erfindungsgemäßen Hefestamm zur mikrobiellen Herstellung von ω-Hydroxyfettsäuren herausgestellt, da dadurch eine geringere Mengen an organischen Säuren produziert und/oder ins Kulturmedium sekretiert wird.

Optional kann vorgesehen sein, dass in dem erfindungsgemäßen Hefestamm zur mikrobiellen Herstellung von ω-Hydroxyfettsäuren zusätzlich der Transport von ω-Hydroxyfettsäuren aus der Hefezelle bzw. aus dem Endoplasmatisches Retikulum ins Cytoplasma, z.B. durch die erhöhte Aktivität spezifischer Membrantransporter, gesteigert ist, was zu einer gesteigerten Bildung von ω-Hydroxyfettsäuren aus dem entsprechenden hydrophoben Substrat, insbesondere *n*-Alkanen und Fettsäuren führt.

Bevorzugt ist in dem erfindungsgemäßen Hefestamm zur mikrobiellen Herstellung von ω-Hydroxyfettsäuren zusätzlich der Transport der hydrophoben Substrate in die Hefezelle bzw. die jeweiligen Organellen (Peroxisomen, Endoplasmatisches Retikulum), z.B. durch die erhöhte Aktivität spezifischer Membrantransporter, gesteigert ist, was zu einer gesteigerten Bildung von ω-Hydroxyfettsäuren aus dem entsprechenden hydrophoben Substrat, insbesondere *n*-Alkanen und Fettsäuren führt.

Die Erfindung betrifft auch die Verwendung des erfindungsgemäßen Hefestamm zur mikrobiellen Herstellung von ω-Hydroxyfettsäuren der allgemeinen Struktur: HOOC-(CH2)n-OH, HOOC-(CₙH₂ₙ₋₂)-CH₂OH, COOC-(CₙH₂ₙ₋₄)-CH₂OH oder HOOC-(CₙH₂ₙ₋₆)-CH₂OH, mit n im Bereich von 6 bis 18, aus einem hydrophoben Substrat.

Die Erfindung umfasst auch einen Hefestamm der Art *Y. lipolytica* zur mikrobiellen Produktion von Dicarbonsäuren aus *n*-Alkanen und Fettsäuren (und anderen hydrophoben Substraten) der eine Verringerung der Aktivitäten der Acyl-CoA-Oxidasen (namentlich *POX1*: YALI0E32835g, *POX2*: YALI0F10857g, *POX3*: YALI0D24750g, *POX4:* YALI0E27654g, *POX5*: YALI0C23859g, *POX6*: YALI0E06567g) aufweist und dadurch gekennzeichnet ist, dass in diesem Hefestamm zusätzlich die Aktivitäten relevanter (Fett-)Alkohol-Dehydrogenasen (namentlich ausgewählt aus *FADH*: YALI0F09603g, *ADH1:* YALl0D25630g, *ADH2:* YALI0E17787g, *ADH3:* YALI0A16379g, *ADH4:* YALI0E15818g, *ADH5:* YALl0D02167g, *ADH6:* YALI0A15147g und/oder *ADH7:* YALI0E07766g) und die Aktivitäten relevanter (Fett-)Alkohol-Oxidasen (namentlich *FAO1:* YALI0B14014g) erhöht sind, was zu einer gesteigerten Bildung von Dicarbonsäuren, vorzugsweise gemäß der allgemeinen Struktur: HOOC-(CH₂)ₙ-COOH, HOOC-(CₙH₂ₙ₋₂)-COOH, COOC-(CₙH₂ₙ₋₄)-COOH oder HOOC-(CnH₂ₙ₋₆)-COOH, mit n im Bereich von 6 bis 18, vorzugsweise im Bereich von 8 bis 16, aus *n*-Alkanen und Fettsäuren (und anderen hydrophoben Substraten) führt.

Der erfindungsgemäße Hefestamm zur Produktion von Dicarbonsäuren weist vorzugsweise eine Deletion der Gene *POX1, POX2, POX3, POX4, POX5* und *POX6* auf. Außerdem weist er eine erhöhte Aktivität der Produkte von *FADH, ADH1, ADH2, ADH3, ADH4, ADH5, ADH6, ADH7* und/oder *FAO1* auf. Weiterhin ist die Bildung der Fettkörperchen verringert, vorzugsweise durch die Verringerung der Aktivität der Genprodukte von *PAH1* und/oder *SCT1.*

Besonders vorteilhaft verstärkt die Aktivitätssteigerung der Genprodukte von *FADH, ADH1, ADH2, ADH3, ADH4, ADH5, ADH6, ADH7* und/oder *FAO1* die weitere Oxidation der ω-Hydroxyfettsäuren zum Fettsäurealdehyd, bzw. zur Dicarbonsäure im Rahmen der ω-Oxidation von Fettsäuren, so dass die gewünschte Dicarbonsäuren verstärkt gebildet wird.

Unter einer erhöhten Aktivität eines Proteins ist vorzugsweise zu verstehen, dass die Aktivität pro Zelle erhöht ist, was durch die Steigerung der Proteinmenge oder durch die Steigerung der proteineigenen Aktivität erzielt werden kann. Die Steigerung der Proteinmenge ist dadurch gekennzeichnet, dass mehr Protein gebildet wird, als bei einem unveränderten Stamm unter gleichen Bedingungen. Die Steigerung der proteineigenen Aktivität ist dadurch gekennzeichnet, dass die gleiche Menge an Protein in einem veränderten Stamm eine höhere Aktivität aufweist als in einem unveränderten Stamm.

Eine erhöhte Aktivität der Proteine kann auf verschiedene Weise erzeugt werden:
(i) durch Einbringen des relevanten Gens in mehrfacher, vorzugsweise 8 bis 12facher, Kopie in das Genom des Organismus (Gen-Dosis-Effekt)
(ii) durch Austausch des natürlichen Promotors des relevanten Gens durch einen stärkeren als den natürlichen Promotor, vorzugsweise einen Promotor, der mindestens 50% der Aktivität des Promotors des Translationselongationsfaktor 1 alpha-Gens (*TEF1*: *YALI0C09141g*) besitzt, höchstvorzugsweise einen Promotor, der mindestens die Aktivität des *TEF1*-Promotors besitzt. Dabei können Promotoren gewählt werden, die entweder während der gesamten Kultivierung oder nur in einer gewünschten Wachstumsphase aktiv sind (Gen-Regulations-Effekt).

Bei der Aktivitätserhöhung wird das zusätzliche Einbringen des relevanten Gens unter Kontrolle eines starken Promotors besonders bevorzugt, vorzugsweise der *TEF1-*Promotor aus *Yarrowia lipolytica.*

Erfindungsgemäß sind die Hefestämme der Art *Y. lipolytica* zur Herstellung von ω-Hydroxyfettsäuren bzw. von Dicarbonsäuren nicht zum Abbau von Fettsäuren im Rahmen der β-Oxidation befähigt. Dies kann beispielsweise durch folgende Maßnahmen gewährleistet werden:
(i) Verringerung der Aktivität der Acyl-CoA-Oxidasen (*POX1*: YALI0E32835g, *POX2*: YALI0F10857g, *POX3*: YALI0D24750g, *POX4:* YALI0E27654g, *POX5*: YALI0C23859g, *POX6*: YALI0E06567g)
(ii) Verhinderung der Peroxisomenbiogenese, z.B. durch Verringerung der Aktivität der Peroxine (z.B. *PEX10*: YALI0C01023g)

Für die Produktion von ω-Hydroxyfettsäuren muss zusätzlich zur Verhinderung der β-Oxidation die Oxidation der ω-Hydroxyfettsäuren zum Fettsäure-Aldehyd, bzw. zur Dicarbonsäure im Rahmen der ω-Oxidation verhindert oder verringert werden. Dies kann folgendermaßen gewährleistet werden:
(i) Verringerung der Aktivität relevanter (Fett-)Alkohol-Dehydrogenasen (*FADH*: YALI0F09603g, *ADH1:* YALl0D25630g, *ADH2:* YALI0E17787g, *ADH3:* YALI0A16379g, *ADH4:* YALI0E15818g, *ADH5:* YALl0D02167g, *ADH6:* YALI0A15147g, *ADH7:* YALI0E07766g)
(ii) Verringerung der Aktivität relevanter (Fett-)Alkohol-Oxidasen (*FAO1*: YALI0B14014g)

Bevorzugt wird die Produktausbeute ferner durch die direkte Beeinflussung relevanter Stoffwechselwege erhöht, insbesondere sind diese:
(i) die Erhöhung der Aktivität der Cytochrom P450-Genprodukte (*ALK1*: YALI0E25982g, *ALK2*: YALI0F01320g, *ALK3*: YALl0A20130g, *ALK4*: YALI0B13816g, *ALK5*: YALI0B13838g, *ALK6*: YALI0B01848g, *ALK7:* YALI0A15488g, *ALK8*: YALI0C12122g, *ALK9*: YALI0B06248g, *ALK10:* YALI0B20702g, *ALK11*: YALI0C10054g, *ALK12*: YALI0A20130g)
(ii) die Erhöhung der Aktivität der NADPH-Cytochrom P450-Reduktase (*CPR1*: YALl0D04422g) Nur für die Produktion von Dicarbonsäuren:
(iii) die Erhöhung der Aktivität relevanter (Fett-)Alkohol-Dehydrogenasen (*FADH*: YALI0F09603g, *ADH1*: YALl0D25630g, *ADH2*: YALI0E17787g, *ADH3*: YALI0A16379g, *ADH4*: YALI0E15818g, *ADH5:* YALl0D02167g, *ADH6*: YALI0A15147g, *ADH7*: YALI0E07766g)
(iv) die Erhöhung der Aktivität relevanter (Fett-)Alkohol-Oxidasen (*FAO1*: YALI0B14014g)
(v) die Erhöhung der Aktivität relevanter (Fett-)Aldehyd-Dehydrogenasen (*FALDH1*: YALI0A17875g, *FALDH2*: YALI0E15400g, *FALDH3*: YALI0B01298g, *FALDH4*: YALI0F23793g)

Im Rahmen unserer Untersuchungen wurde festgestellt, dass die Zellen im Verlauf der Kultivierung sehr große Fettkörperchen (*Lipid bodies*) bilden. Es ist davon auszugehen, dass die Fettsäuren (oder intrazellulär vorliegende Derivate dieser) mit Glycerol verestert und intrazellulär in den Fettkörperchen gespeichert werden. Dies verringert die letztendlich erzielte Produktausbeute.

Vorzugsweise führen gentechnische Veränderungen zu einer Ausbildung kleinerer Fettkörperchen und somit zu einer gesteigerten Produktausbeute, insbesondere sind diese:
(i) die Verringerung der Aktivität der Phosphatidsäure-Dephosphohydrolase (*PAH1*: YALl0D27016p)
(ii) die Verringerung der Aktivität der Phospholipid-Diacylglycerol-Acyltransferase (*LRO1*: YALI0E16797g)
(iii) die Verringerung der Aktivität der Diacylglycerol-Acyltransferase (*DGA1*: YALI0E32769g)
(iv) die Verringerung der Aktivität der Glycerol-3-phosphat-Acyltransferase (*SCT1*: YALI0C00209g)
(v) die Erhöhung der Aktivität der Glycerol-3-phosphat-Dehydrogenase (*GUT2*: YALI0B13970g)
(vi) die Erhöhung der Aktivität der Triacylglycerol-Lipasen (*TGL3*/*TGL4*: YALI0F10010g, YALI0D16379g, YALI0D17534g)

Insbesondere die Verringerung der Aktivität der Phosphatidsäure-Dephosphohydrolase, der Diacylglycerol-Acyltransferase und/oder der Glycerol-3-phosphat-Acyltransferase führen zu einer gesteigerten Produktausbeute, d.h. Herstellung von ω-Hydroxyfettsäuren bzw. Dicarbonsäuren.

Weiterhin werden der Transport der Substrate in die Zelle bzw. die jeweiligen Organellen (Peroxisomen, Endoplasmatisches Retikulum) sowie der Transport der Produkte (ω-Hydroxyfettsäuren und Dicarbonsäuren) aus der Zelle bzw. aus den jeweiligen Organellen gesteigert. Dies kann beispielsweise durch die Erhöhung der Aktivität spezifischer Transporter gewährleistet werden.

Nach einer besonders bevorzugten Ausgestaltung der vorliegenden Erfindung führt die Kombination mehrerer vorgenannter gentechnischer Veränderungen zu einer gesteigerten Produktausbeute, einem gesteigerten Ertrag und/oder einer gesteigerten Raum-Zeit-Ausbeute.

Als Ausgangsstamm für die Konstruktion eines erfindungsgemäßen Stammes kann ein Wildtypisolat der Hefe *Y. lipolytica* verwendet werden, vorzugsweise der Stamm H222. Der Stamm H222 wurde am 29.04.2013 bei der DSMZ (Deutsche Sammlung für Mikroorganismen und Zellkulturen GmbH, D-38142 Braunschweig) unter der Nummer DSM 27185 gemäß Budapester Vertrag über die internationale Anerkennung der Hinterlegung von Mikroorganismen für die Zwecke von Patentverfahren hinterlegt.

Zur Verwendung eines Stammes für die weitere gentechnische Bearbeitung ist ein Selektionsmarker notwendig. Dieser Selektionsmarker kann z.B. in Form der Uracilauxotrophie in an sich bekannter Weise in den Stamm eingebracht werden. Alternativ kann auf bereits bekannte uracilauxotrophe Stämme zurückgegriffen, vorzugsweise auf den Stamm H222-S4 (Mauersberger S, Wang HJ, Gaillardin C, Barth G & Nicaud JM (2001) J Bacteriol 183: 5102-5109.).

Eingebracht werden zusätzliche Gene oder Konstrukte, die vorhandene Gene ersetzen, vorzugsweise mittels üblicher Verfahren, die im Stand der Technik bekannt sind.
Um eine effiziente Transkription der eingebrachten Gene zu gewährleisten, ist es bevorzugt, für den Organismus funktionierende Promotoren mit dem offenen Leserahmen der Gene zu verknüpfen. Hierbei können sowohl konstitutive Promotoren als auch von den Kultivierungsbedingungen abhängige Promotoren genutzt werden.

Bevorzugt sind die eingebrachten Gene, die für die genannten Enzyme kodieren, funktionell unter die Kontrolle eines konstitutiven Promotors gestellt, wobei die Promotoren identisch oder untereinander unterschiedlich sein können.

Vorzugsweise werden konstitutive Promotoren genutzt. Besonders bevorzugt wird der Promotor des Translationselongationsfaktors 1 alpha (pTEF1) verwendet.

Alternativ bevorzugt werden die eingebrachten Gene unter die Kontrolle eines, von den Kultivierungsbedingungen abhängigen Promotors gestellt. Vorzugsweise aktivieren Aktivatoren bzw. Induktoren den von den Kultivierungsbedingungen abhängigen Promotor (im Sinne der Anmeldung auch Operator genannt), indem sie direkt mit einem regulierbaren Promotor interagieren bzw. in dem sie an ein Repressorprotein binden, das sich daraufhin vom Promotor löst. Durch das Kontaktieren des erfindungsgemäßen Hefestamms mit einem Aktivator und/oder Induktor werden die o.g. Enzyme in dem erfindungsgemäßen Hefestamm synthetisiert und stehen somit für die biokatalytische Umsetzung eines hydrophoben Substrates, insbesondere eines entsprechenden *n*-Alkans oder einer Fettsäure, zur Verfügung.

In einer Ausgestaltung der Erfindung unterscheiden sich die von den Kultivierungsbedingungen abhängigen Promotoren voneinander, sodass die Promotoren Primärsignal-spezifisch aktivierbar sind. Vorteilhaft kann somit die Anwesenheit unterschiedlicher Aktivatoren und/oder Induktoren in dem Hefestamm zur Expression ausgewählter Gene führen, wodurch der Stress für eine rekombinante Hefezelle minimiert wird.

Die entsprechenden Nukleinsäuresequenzen für Promotoren (bspw. pTEF1, pPOX2, pPOT1, pXPR1, pICL1), die für ein erfindungsgemäßes Verfahren eingesetzt werden können, sind dem Fachmann bestens bekannt oder können bekannten Datenbanken entnommen werden. Vorteilhaft können für ein erfindungsgemäßes Verfahren neben künstlich in Organismen eingebrachte Promotoren ebenfalls natürliche Promotoren eingesetzt werden.

Ebenso ist es bevorzugt, den offenen Leserahmen der Gene mit einem Terminator zu versehen. Vorzugsweise wird der Terminator des Gens der Isocitratlyase (ICL1t) verwendet.

Gegenstand der Erfindung ist auch ein Verfahren zur biokatalytischen Herstellung von ω-Hydroxyfettsäuren, vorzugsweise gemäß der allgemeinen Struktur: HOOC-(CH₂)ₙ-CH₂OH, HOOC-(CₙH₂ₙ₋₂)-CH₂OH, COOC-(CₙH₂ₙ₋₄)-CH₂OH oder HOOC-(CₙH₂ₙ₋₆)-CH₂OH, mit n im Bereich von 6 bis 18, vorzugsweise im Bereich von 8 bis 16, aus einem hydrophoben Substrat, das die folgenden Schritte umfasst:
a) Bereitstellung von Hefezellen mindestens eines Hefestamms der Art *Y. lipolytica,* der
   (i) eine Verringerung der Aktivitäten der Acyl-CoA-Oxidasen,
   (ii) eine reduzierte Aktivität relevanter (Fett-)Alkohol-Dehydrogenasen und
   (iii) eine reduzierte Aktivität relevanter (Fett-)Alkohol-Oxidasen aufweist, in einem geeigneten Kultivierungsmedium,
b) Kontaktieren der Hefezellen mit dem hydrophoben Substrat, wobei das hydrophobe Substrat mit mindestens einem der Enzyme der Hefezellen, ausgewählt aus der Gruppe,
   (i) Cytochrom P450-Genprodukte,
   (ii) NADPH-Cytochrom P450-Reduktase und
   (iii) (Fett-)Aldehyd-Dehydrogenasen zu dem Reaktionsprodukt ω-Hydroxyfettsäure umgesetzt wird
c) Isolierung der ω-Hydroxyfettsäure.

Besonders bevorzugt ist die ω-Hydroxyfettsäure eine lineare ω-Hydroxyfettsäure gemäß der allgemeinen Struktur: HOOC-(CH₂)ₙ-CH₂OH mit n im Bereich von 8 bis 16.

Nach einer bevorzugten Ausgestaltung des erfindungsgemäßen Verfahrens zur biokatalytischen Herstellung von ω-Hydroxyfettsäuren kann vorgesehen sein, dass die Umsetzung zu dem Reaktionsprodukt ω-Hydroxyfettsäure zusätzlich mit in den verwendeten Hefezellen verbleibenden (Fett-)Alkohol-Oxidasen, (Fett-)Alkohol-Dehydrogenasen erfolgt.

Bevorzugt sind die Hefezellen mindestens eines Hefestamms in dem erfindungsgemäßen Verfahren zur biokatalytischen Herstellung von ω-Hydroxyfettsäuren der Art *Y. lipolytica* ausgewählt aus dem erfindungsgemäßen Hefestamm zur mikrobiellen Herstellung von ω-Hydroxyfettsäuren.

Überraschend hat sich gezeigt, dass nach der biokatalytischen Umsetzung des hydrophoben Substrates zu dem gewünschten Reaktionsprodukt ω-Hydroxyfettsäuren diese in den erfindungsgemäßen Hefezellen eines Hefestamms der Art *Y. lipolytica* nicht weiter verstoffwechselt (d.h. im Stoffwechsel des Ganzzellkatalysators abgebaut) werden und sich in der wässrigen Komponente anreichern. Vorteilhaft reichert sich die ω-Hydroxyfettsäure in Folge des Ausschleusens aus den Hefezellen in dem Kultivierungsmedium an.

Bevorzugt erfolgt das Kontaktieren des Hefestamms mit einem hydrophoben Substrat durch direkte Zugabe zur flüssigen Komponente in Form als Flüssigkeit und/oder Feststoff. Bei direkter Zugabe zum Hefestamm kann zudem eine organische Phase als Substratreservoir Anwendung finden, wodurch die Prozesslaufzeit optimiert werden kann.

Im Sinne der vorliegenden Erfindung wird unter einem hydrophoben Substrat ein an sich wasserunlöslicher Stoff verstanden, aufweisend ein gegebenenfalls verzweigtes und/oder ungesättigtes Kohlenwasserstoffgrundgerüst mit 8 bis 20 Kohlenstoffatomen. Bevorzugt ist das hydrophobe Substrat ausgewählt aus der Gruppe der *n*-Alkane der allgemeinen Struktur: H₃C-(CH₂)ₙ-CH₃ (bspw. Dodekan, Pentadekan, Hexadekan) oder Alkene der allgemeinen Struktur H₃C-(CₙH₂ₙ-₂)-CH₃, H₃C-(CₙH₂ₙ₋₄)-CH₃ oder H₃C-(CₙH₂ₙ₋₆)-CH₃, wobei n eine ganze Zahl im Bereich von 6 bis 18 ist, der gegebenenfalls verzweigten gesättigten Fettsäuren der allgemeinen Struktur: HOOC-(CH₂)ₙ-CH₃, wobei n eine ganze Zahl im Bereich von 6 bis 18 ist (bspw. Dodekansäure, Hexadekansäure, Pentadekansäure, Phytansäure), der ungesättigten Fettsäuren der allgemeinen Struktur: HOOC-(CₙH₂ₙ₋₂)-CH₃, COOC-(CₙH₂ₙ₋₄)-CH₃ oder HOOC-(CₙH₂ₙ₋₆)-CH₃, wobei n eine ganze Zahl im Bereich von 6 bis 18 ist (bspw. Ölsäure, Linolsäure, Arachidonsäure), und Fettsäurealkylester der allgemeinen Struktur: ROOC-(CH₂)ₙ-CH₃, mit n im Bereich von 6 bis 18 und wobei R ein gegebenenfalls verzweigter C₁₋₄-Alkylrest ist.

Insbesondere bevorzugt aber nicht ausschließlich dienen vorgenannte *n*-Alkane, Alkene oder gesättigte bzw. ungesättigte Fettsäuren mit einer Kettenlänge (Anzahl der Kohlenstoffatome der längsten Kette) von acht bis achtzehn Kohlenstoffatomen, d.h. n im Bereich von 6 bis 16 als hydrophobes Substrat für die Biokonversion.

Nach einer alternativ bevorzugten Ausgestaltung der vorliegenden Erfindung dienen für die Biokonversion vorzugsweise aber nicht ausschließlich *n*-Alkane und Fettsäuren mit einer Kettenlänge von acht bis achtzehn Kohlenstoffatomen, d.h. n im Bereich von 6 bis 16, als hydrophobes Substrat.

Nach einer bevorzugten Ausgestaltung des erfindungsgemäßen Verfahrens beträgt die Konzentration des hydrophoben Substrats zu Beginn der Herstellung 1 bis 50 Gramm pro Liter Kulturmedium (g l⁻¹), besonders bevorzugt sind 5 bis 40 g l⁻¹.

Die Erfindung betrifft ferner ein Verfahren zur Herstellung von Dicarbonsäuren, vorzugsweise gemäß der allgemeinen Struktur: HOOC-(CH₂)ₙ-COOH, HOOC-(CₙH₂ₙ₋₂)-COOH, COOC-(CₙH₂ₙ₋₄)-COOH oder HOOC-(CₙH₂ₙ₋₆)-COOH, mit n im Bereich von 6 bis 18 vorzugsweise im Bereich von 8 bis 16, aus einem hydrophoben Substrat, insbesondere ausgewählt aus *n*-Alkanen und Fettsäuren, das die folgenden Schritte umfasst:
a) Bereitstellung von Hefezellen mindestens eines Hefestamms der Art *Y. lipolytica,* der
   (i) eine Verringerung der Aktivitäten der Acyl-CoA-Oxidasen,
   (ii) eine erhöhte Aktivität relevanter (Fett-)Alkohol-Dehydrogenasen und
   (iii) eine erhöhte Aktivität relevanter (Fett-)Alkohol-Oxidasen aufweist, in einem geeigneten Kultivierungsmedium,
b) Kontaktieren der Hefezellen mit dem hydrophoben Substrat, wobei das hydrophobe Substrat mit mindestens einem der Enzyme der Hefezellen, ausgewählt aus der Gruppe,
   (i) der Cytochrom P450-Genprodukte,
   (ii) der NADPH-Cytochrom P450-Reduktase,
   (iii) der (Fett-)Alkohol-Dehydrogenasen nach a),
   (iv) der (Fett-)Alkohol-Oxidasen nach a) und
   (v) (Fett-)Aldehyd-Dehydrogenase,
   zu dem Reaktionsprodukt Dicarbonsäure umgesetzt wird,
c) Isolierung der Dicarbonsäure.

Besonders bevorzugt ist die Dicarbonsäure eine lineare Dicarbonsäure gemäß der allgemeinen Struktur: HOOC-(CH₂)ₙ-CH₂OH mit n im Bereich von 8 bis 16.

Bevorzugt sind in den Hefezellen für das erfindungsgemäße Verfahren zur Herstellung von ω-Hydroxyfettsäuren oder Dicarbonsäuren zusätzlich die Aktivitäten einzelner oder aller Cytochrom P450-Genprodukte, insbesondere von *ALK1*: YALI0E25982g, *ALK2*: YALI0F01320g, *ALK3*: YALl0A20130g, *ALK4:* YALI0B13816g, *ALK5*: YALI0B13838g, *ALK6:* YALI0B01848g, *ALK7:* YALI0A15488g, *ALK8*: YALI0C12122g, *ALK9*: YALI0B06248g, *ALK10*: YALI0B20702g, *ALK11*: YALI0C10054g und/oder *ALK12*: YALI0A20130g erhöht. Vorteilhaft führt die Erhöhung einzelner oder aller Cytochrom P450-Genprodukte zu einer gesteigerten Bildung von ω-Hydroxyfettsäuren oder Dicarbonsäuren aus dem entsprechenden hydrophoben Substrat, insbesondere *n*-Alkanen und Fettsäuren.

Es kann vorteilhaft vorgesehen sein, dass in den Hefezellen für das erfindungsgemäße Verfahren zur Herstellung von ω-Hydroxyfettsäuren oder Dicarbonsäuren zusätzlich die Aktivität der NADPH-Cytochrom P450-Reduktase (*CPR1*: YALl0D04422g) erhöht ist, was zu einer gesteigerten Bildung von ω-Hydroxyfettsäuren oder Dicarbonsäuren aus dem entsprechenden hydrophoben Substrat, insbesondere *n*-Alkanen und Fettsäuren, führt.

Nach einer bevorzugten Ausgestaltung des erfindungsgemäßen Verfahrens zur Herstellung von Dicarbonsäuren sind in den Hefezellen zusätzlich die Aktivitäten relevanter (Fetts-)Alkohol-Dehydrogenasen (namentlich *FADH*: YALI0F09603g, *ADH1*: YALl0D25630g, *ADH2:* YALI0E17787g, *ADH3:* YALI0A16379g, *ADH4:* YALI0E15818g, *ADH5:* YALl0D02167g, *ADH6:* YALI0A15147g, *ADH7*: YALI0E07766g) erhöht, was vorteilhaft zu einer gesteigerten Bildung von Dicarbonsäuren aus dem entsprechenden hydrophoben Substrat, insbesondere n-Alkanen und Fettsäuren führt.

Bevorzugt sind in den Hefezellen des erfindungsgemäßen Verfahrens zur Herstellung von Dicarbonsäuren zusätzlich die Aktivitäten relevanter (Fett-)Alkohol-Oxidasen (*FAO1*: YALI0B14014g) erhöht, was zu einer gesteigerten Bildung von Dicarbonsäuren aus dem entsprechenden hydrophoben Substrat, insbesondere n-Alkanen und Fettsäuren führt

Nach einer bevorzugten Ausgestaltung des erfindungsgemäßen Verfahrens zur Herstellung von Dicarbonsäuren, sind in den Hefezellen zusätzlich die Aktivitäten relevanter (Fett-)Aldehyd-Dehydrogenasen (*FALDH1*: YALI0A17875g, *FALDH2*: YALI0E15400g, *FALDH3*: YALI0B01298g, *FALDH4*: YALI0F23793g) erhöht, was zu einer gesteigerten Bildung von Dicarbonsäuren aus dem entsprechenden hydrophoben Substrat, insbesondere *n*-Alkanen und Fettsäuren führt.

Bevorzugt liegen die Ausbeuten von ω-Hydroxyfettsäuren bzw. Dicarbonsäuren in dem erfindungsgemäßen Verfahren für den Zeitraum von einem Tag (d.h. 24 h) im Bereich von 1 bis 20 g l⁻¹, besonders bevorzugt im Bereich von 4 bis 15 g l⁻¹.

Nach einer bevorzugten Ausgestaltung der Erfindung erfolgt die Herstellung von ω-Hydroxyfettsäuren und Dicarbonsäuren in einem Bioreaktor (Fermenter), in dem ein erfindungsgemäßer Hefestamm unter geeigneten Wachstumsbedingungen in einem geeigneten Kultivierungsmedium fermentiert wird.

Die Erfindung betrifft ferner Verfahren zur Herstellung von ω-Hydroxyfettsäuren und Dicarbonsäuren, die dadurch gekennzeichnet sind, dass ein erfindungsgemäßer Mikroorganismus in an sich bekannter Art und Weise in einem Bioreakor (Fermenter) kultiviert wird.

Bevorzugt erfolgt die Isolierung des Reaktionsproduktes ω-Hydroxyfettsäuren und Dicarbonsäuren durch Extraktion der wässrigen Komponente mit einem organischen Lösungsmittel ausgewählt aus der Gruppe der Phthalsäureester, besonders bevorzugt Ethylacetat, 1,2-Cyclo-hexandicarbonsäurediisononylester und Mesamoll®, und/oder der aliphatischen verzweigten und/oder linearen Kohlenwasserstoffe, vorzugsweise mit 5 bis 16 Kohlenstoffatomen, wie z.B. *n*-Pentan, Cyclopentan, *n*-Hexan, Cyclohexan, *n*-Heptan, *n*-Octan, Cyclooctan, *n*-Dekan, *n*-Dodekan oder *n*-Hexadekan. Bevorzugt dienen die genannten organischen Lösungsmittel in einem einphasigen, wässrigen System zur Extraktion nach dem Umsatz des hydrophoben Substrates. Alternativ dienen die genannten organischen Lösungsmittel in einem zweiphasigen System in Form einer zweiten Phase neben dem Kultivierungsmedium als Reservoir für das hydrophobe Substrat und/oder zur Abtrennung des Reaktionsprodukts.

Die Isolierung der Dicarbonsäuren und/oder ω-Hydroxyfettsäuren durch Extraktion der wässrigen Komponente mit einem organischen Lösungsmittel erfolgt vorzugsweise nach der Abtrennung der Hefezellen in Form von Biomasse von dem Kultivierungsmedium, wobei die Abtrennung der Hefezellen in Form von Biomasse von dem Kultivierungsmedium bevorzugt durch Zentrifugation oder Filtration erfolgt.

Bevorzugt erfolgt die Extraktion von den Reaktionsprodukten ω-Hydroxyfettsäuren und Dicarbonsäuren mit einem organischen Lösungsmittel bei einem pH-Wert zwischen 0 und 8, besonders bevorzugt zwischen 1 und 7, ganz besonders bevorzugt zwischen 1 und 6.

Gegebenenfalls erfolgt zur Reinigung der Dicarbonsäuren und/oder ω-Hydroxyfettsäuren im Anschluss an die Extraktion eine Destillation, wobei bevorzugt das organische Lösungsmittel abgetrennt wird. Bevorzugt erfolgt die Abtrennung des organischen Lösungsmittels durch Verdampfung bei einem Druck zwischen 0,1 und 1000 mbar, besonders bevorzugt zwischen 0,1 und 750 mbar, ganz besonders bevorzugt zwischen 1 und 400 mbar.

Bevorzugt ist in einem Hefestamm für das erfindungsgemäße Verfahren zur Herstellung von ω-Hydroxyfettsäuren bzw. Dicarbonsäuren zusätzlich die Aktivität der Phosphatidsäure-Dephosphohydrolase (*PAH1*: YALl0D27016p) verringert. Vorteilhaft führt dies zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren.

Nach einer bevorzugten Ausgestaltung des erfindungsgemäßen Verfahrens zur Herstellung von ω-Hydroxyfettsäuren bzw. Dicarbonsäuren ist in dem eingesetzten Hefestamm zusätzlich die Aktivität der Glycerol-3-phosphat-Acyltransferase (*SCT1*: YALI0C00209g) verringert, was vorteilhaft zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n-*Alkanen und Fettsäuren (und anderen hydrophoben Substraten) führt.

Es kann ferner auch vorgesehen sein, dass in dem erfindungsgemäßen Verfahren zur mikrobiellen Herstellung von Dicarbonsäuren bzw. ω-Hydroxyfettsäuren in den Hefezellen des eingesetzten Hefestamms zusätzlich die Aktivität der Phospholipid-Diacylglycerol-Acyltransferase (*LRO1*: YALI0E16797g) verringert ist, was zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Nach einer bevorzugten Ausgestaltung der vorliegenden Erfindung ist in dem erfindungsgemäßen Hefestamm zur mikrobiellen Herstellung von ω-Hydroxyfettsäuren bzw. Dicarbonsäuren zusätzlich die Aktivität der Diacylglycerol-Acyltransferase (*DGA1*: YALI0E32769g) verringert, was zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Nach einer bevorzugten Ausgestaltung des erfindungsgemäßen Verfahrens zur Herstellung von ω-Hydroxyfettsäuren bzw. Dicarbonsäuren ist in den Hefezellen des entsprechend eingesetzten Hefestamms zusätzlich die Aktivität der Glycerol-3-phosphat-Dehydrogenase (*GUT2*: YALI0B13970g) erhöht, was zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Besonders vorteilhaft wurde nun gefunden, dass die zusätzlich Erhöhung der Aktivitäten relevanter Triacylglycerol-Lipasen (*TGL3*/*TGL4*: YALI0F10010g, YALI0D16379g, YALI0D17534g) in den verwendeten Hefezellen zur mikrobiellen Herstellung von ω-Hydroxyfettsäuren bzw. Dicarbonsäuren, zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Bevorzugt ist in den Hefezellen des für das erfindungsgemäßes Verfahren zur Herstellung von ω-Hydroxyfettsäuren bzw. Dicarbonsäuren verwendeten Hefestamms zusätzlich der Transport der hydrophoben Substrate in die Hefezelle bzw. die jeweiligen Organellen (Peroxisomen, Endoplasmatisches Retikulum), z.B. durch die erhöhte Aktivität spezifischer Membrantransporter, gesteigert ist, was zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Optional kann vorgesehen sein, dass in den Hefezellen zur mikrobiellen Herstellung von ω-Hydroxyfettsäuren bzw. Dicarbonsäuren zusätzlich der Transport von ω-Hydroxyfettsäuren und Dicarbonsäuren aus der Hefezelle bzw. aus dem Endoplasmatisches Retikulum ins Cytoplasma, z.B. durch die erhöhte Aktivität spezifischer Membrantransporter, gesteigert ist, was zu einer gesteigerten Bildung von ω-Hydroxyfettsäuren oder Dicarbonsäuren aus dem entsprechenden hydrophoben Substrat, insbesondere *n*-Alkanen und Fettsäuren und Anreicherung in dem Kulturmedium führt.

Besonders vorteilhaft hat sich die die Verringerung der Aktivität des Citrat- und Oxalacetat-Transporters (*YHM2*: YALI0B10736g) und/oder die Verringerung der Aktivität des Citrat-Transport-Proteins (*CTP1*: YALI0F26323g) in den Hefezellen für das erfindungsgemäße Verfahren zur mikrobiellen Herstellung von ω-Hydroxyfettsäuren bzw. Dicarbonsäuren herausgestellt, da dadurch eine geringere Mengen an organischen Säuren produziert und/oder ins Kulturmedium sekretiert wird.

Bevorzugt ist das Kultivierungsmedium ein Minimalmedium. Unter einem Minimalmedium wird im Sinne der vorliegenden Erfindung ein wässriges Kulturmedium verstanden, welches alle zum Wachstum der Hefezellen benötigten Substrate (bspw. C-Quelle, Mineralsalze, Spurenelemente, Vitamine) in gelöster Form enthält, welches aber zugleich keine komplexen Inhaltsstoffe bzw. komplexen Biomolekülen (bspw. Hefeextrakt, Pepton, Trypton usw.) aufweist. Dem Fachmann ist aus dem Stand der Technik eine Vielzahl von Minimalmedien zur Kultivierung von Hefezellen bekannt.

Neben dem angebotenen hydrophoben Substrat für die Biokonversion ist während der Kultivierung eine weitere Kohlenstoffquelle (C-Quelle; Substrat für die Energiegewinnung), auf deren Grundlage die Zelle Energie gewinnen kann, bereitzustellen.

Vorzugsweise sind dem Kultivierungsmedium daher als C-Quelle Mono- bzw. Disaccharide, bspw. Glukose, D-Fructose, Xylose, Maltose, Saccharose und/oder mehrwertige Alkohole, wie bspw. Glycerol, Rohglycerol und Mannit zugesetzt, besonders bevorzugt ist die zugesetzte Kohlenstoffquelle Glucose.

Insbesondere dient als Stickstoffquelle (N-Quelle) für die Kultivierung von Hefestämmen in dem erfindungsgemäßen Verfahren Ammonium/Ammoniak oder Aminosäuren wie Glutamin/Glutamat.

Bei der Kultivierung eines erfindungsgemäßen Stammes sind Kultivierungsbedingungen, unter denen die Fettkörperchen vergleichsweise gering und die Umsatzrate der angebotenen Substrate vergleichsweise groß ausgeprägt sind, zu bevorzugen.

Eine Limitierung des Wachstums der Zellen verhindert, dass zu große Mengen des eingesetzten Substrates in den Zellen gespeichert werden, was die Produktausbeute verringert, und dass eine Sauerstofflimitierung eintritt, die die Produktivität vermindert.

Eine mögliche Form der Limitierung ist die Stickstofflimitierung. Diese Limitierung führt in der Hefe *Y. lipolytica* zu einem Wachstumsstopp nach Aufbrauchen der Stickstoffquelle und zur Bildung von Citronensäure und Iso-Citronensäure. Vorzugsweise sollte der Stickstoffgehalt im Medium während der Produktionsphase, also nach dem Kontaktieren der Hefezellen mit dem hydrophoben Substrat, kleiner als 2 g l⁻¹, vorzugsweise kleiner als 1 g l⁻¹, hoch vorzugswiese kleiner 0,5 g l⁻¹, höchst vorzugsweise 0 g l⁻¹ sein. Die Produktionsphase ist dadurch gekennzeichnet, dass sie sich einer Phase starken Wachstums (Wachstumsphase) anschließt und nur noch (im Vergleich zur Wachstumsrate) geringes Wachstum, jedoch erhöhte Produktbildung aufweist.

Vorzugsweise erfolgt die Kultivierung im Fermenter unter kontrollierten Prozessparametern, wobei besonders bevorzugt der pH-Wert im Kultivierungsmedium (während einer bestimmten Wachstumsphase) konstant gehalten wird. Insbesondere bevorzugt erfolgt die Fermentation unter Limitationsbedingungen.

Der pH-Wert im Kultivierungsmedium sollte, nach dem Kontaktieren, also während der Wachstumsphase dem physiologisch bevorzugten von 5,5 entsprechen und während der Produktionsphase, also nach dem Kontaktieren der Hefezellen mit dem hydrophoben Substrat, vorzugsweise 5,5 - 9,0, besonders bevorzugt 7,0 - 9,0 und insbesondere bevorzugt 8,0 betragen.

Bevorzugt erfolgt die Kultivierung der Hefezellen unter physiologischen Bedingungen bei einer Temperatur zwischen 0 und 60 °C, bevorzugt zwischen 10 und 50 °C, besonders bevorzugt zwischen 20 und 40 °C, wobei der pH-Wert des Kultivierungsmediums zwischen 2,0 und 9,0, bevorzugt zwischen 5,5 und 9,0, besonders bevorzugt zwischen 7,0 und 9,0, insbesondere bevorzugt um die 8,0 liegt.

Vorzugsweise werden im erfindungsgemäßen Verfahren die genannten Limitationsbedingungen miteinander kombiniert.

Gegenstand der Erfindung ist auch ein Kit zur biokatalytischen Herstellung von Dicarbonsäuren oder ω-Hydroxyfettsäuren enthaltend:
a) mindestens einen Typ an Hefezellen, bevorzugt ein Typ erfindungsgemäß modifizierter Hefezellen des Hefestamms der Art *Y. lipolytica* in einer wässrigen Komponente und/oder
b) mindestens einen Typ kryokonservierter Hefezellen, bevorzugt eines Typs erfindungsgemäß modifizierter Hefezellen des Hefestamms der Art *Y. lipolytica.*

Die Biomasse in Form von Hefezellen für das erfindungsgemäße Verfahren zur biokatalytischen Herstellung von Dicarbonsäuren oder ω-Hydroxyfettsäuren kann durch eine dem Fachmann bekannte Weise der Vorkultivierung der entsprechend einzusetzenden Hefezellen (Anzucht auf Vollmedium und/oder Minimalmedium) erhalten werden, beispielsweise durch Kultivierung in Vollmedium, wie YPD-Medium (DSM-Medium No. 393), vorzugsweise jedoch durch Kultivierung in einem Medium, das die Erzeugung hoher Zelldichten, beispielsweise durch Kultivierung in Minimalmedium (bspw. 5 % (w/v) Glucose, 20 g l⁻¹ CaCO₃, 1 g l⁻¹ KH₂PO₄, 0,16 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid) insbesondere größer als 1 x 10⁹ Zellen pro ml, zulässt. Die Anzucht dieser Vorkultur der Hefezellen des erfindungsgemäßen Kits erfolgt vorzugsweise in laborüblichen Schüttelkolben, zur Erzeugung größerer Mengen an Biomasse ist aber auch die Anzucht unter kontrollierten Bedingungen im Fermenter möglich.

Ein weiterer Aspekt der Erfindung betrifft auch die Verwendung erfindungsgemäß modifizierter Hefezellen des Hefestamms der Art *Y. lipolytica,* ein erfindungsgemäßes Verfahren oder eines erfindungsgemäßes Kits.

Bevorzugte Weiterbildungen der Erfindung ergeben sich aus den Kombinationen der Ansprüche oder einzelner Merkmale davon.

Nachfolgend soll die Erfindung anhand einiger Ausführungsbeispiele und zugehöriger Figuren eingehender erläutert werden. Die folgenden Beispiele dienen der weiteren Erläuterung der Erfindung, ohne die Erfindung auf diese zu beschränken.

Dabei zeigt:
- Fig. 1:: die Stoffwechselwege zum Abbau hydrophober Substrate durch *Y. lipolytica.*
- Fig. 2:: die Akkumulation von Dicarbonsäuren und ω-Hydroxyfettsäuren in *Y. lipolytica* H222ΔP, H222ΔPΔA und H222ΔPΔF unter Verwendung verschiedener hydrophober Substrate.
- Fig. 3:: die Biokonversion vom Pentadekansäure zu ω-Hydroxypentadekansäure durch *Y. lipolytica* H222ΔPΔF.
- Fig. 4:: die Ausbildung von Fettkörperchen bei *Y. lipolytica* H222ΔPΔF, H222ΔPΔFΔD, H222ΔPΔFΔH und H222ΔPΔFΔS.
- Fig. 5:: die Akkumulation von Dodekandisäure und ω-Hydroxydodekansäure bei der Kultivierung verschiedener Stämme der Hefe *Y. lipolytica* unter Verwendung von Dodekan als Substrat.
- Fig. 6:: die (Fett-)Alkohol-Dehydrogenase und -Oxidase-Enzymaktivität bei der Kultivierung verschiedener Stämme der Hefe *Y. lipolytica* unter Verwendung von Dodekan als Substrat.
- Fig. 7:: die Biokonversion vom Dodekan zu ω-Hydroxydodekansäure und Dodekandisäure durch *Y. lipolytica* H222ΔPΔF.

### Erläuterungen zu den genetischen Veränderungen:

- ΔP: Deletion der Acyl-CoA-Oxidase-Gene (*POX1-6*)
- ΔF: Deletion des (Fett-)Alkohol-Oxidase-Gens (*FAO1*)
- ΔA: Deletion der (Fett-)Alkohol-Dehydrogenase-Gene *(FADH*, A*DH1-7*)
- ΔD: Deletion des Diacylglycerol-Acyltransferase-Gens (*DGA1*)
- ΔH: Deletion des Phosphatidsäure-Dephosphohydrolase-Gens (*PAH1*)
- ΔS: Deletion der Glycerol-3-phosphat-Acyltransferase-Gens (*SCT1*)
- oC: Überexpression des NADPH-Cytochrom P450-Reduktase-Gens *CPR1*
- oF: Überexpression der (Fett-)Alkohol-OxidaseGens *FAO1*

### Erläuterungen zum Sequenzprotokoll

SEQ ID NO. 1: DNA-Sequenz des Plasmids pJET1.2/blunt (Fermentas).
SEQ ID NO. 2: DNA-Sequenz des Plasmids pUCBM21 (Boehringer Ingelheim).
SEQ ID NO. 3: DNA-Sequenz des artifiziellen Plasmids pUC-Lys2-DK2.
SEQ ID NO. 5: DNA-Sequenz des artifiziellen Plasmids pINTB_HMG1.

Die Fig. 1 zeigt, dass *n*-Alkane in die Zelle aufgenommen und dann im Rahmen der primären Alkanoxidation zur korrespondierenden Fettsäure mit gleicher Kettenlänge umgewandelt werden. Die Fettsäuren werden dann im Rahmen der β-Oxidation zu Acetyl-CoA (bzw. Propionyl-CoA) abgebaut. Die diterminale Oxidation von Fettsäuren (ω-Oxidation) kann parallel stattfinden. Die Namen der gebildeten chemischen Substanzen sind ebenso angegeben wie die Bezeichnungen der beteiligten Enzyme und der korrespondierenden Gene. *ALK1*: *YALI0E25982g*, *ALK2*: *YALI0F01320g*, *ALK3*: *YALI0A20130g*, *ALK4*: *YALI0B13816g*, *ALK5*: *YALI0B13838g*, *ALK6*: *YALI0B01848g*, *ALK7*: *YALI0A15488g*, *ALK8*: *YALI0C12122g*, *ALK9*: *YALI0B06248g*, *ALK10*: *YALI0B20702g*, *ALK11*: *YALI0C10054g*, *ALK12*: *YALI0A20130g*, *CPR1*: *YALI0D04422g*, *FADH*: *YALI0F09603g*, *ADH1*: *YALI0D25630g*, *ADH2*: *YALI0E17787g, ADH3*: *YALI0A16379g*, *ADH4*: *YALI0E15818g*, *ADH5*: *YALI0D02167g*, *ADH6*: *YALI0A15147g*, *ADH7*: *YALI0E07766g*, *ADH8*: *YALI0C12595g*, *FAO1*: *YALI0B14014g*, *FALDH1*: *YALI0A17875g*, *FALDH2*: *YALI0E15400g*, *FALDH3*: *YALI0B01298g*, *FALDH4*: *YALI0F23793g*, *FAA1*: *YALI0D17864g*, *POX1*: *YALI0E32835g*, *POX2*: *YALI0F10857g*, *POX3*: *YALI0D24750g*, *POX4*: *YALI0E27654g*, *POX5*: *YALI0C23859g*, *POX6*: *YALI0E06567g*, *MFE2*: *YALI0E15378g*, *POT1*: *YALI0E18568g*.

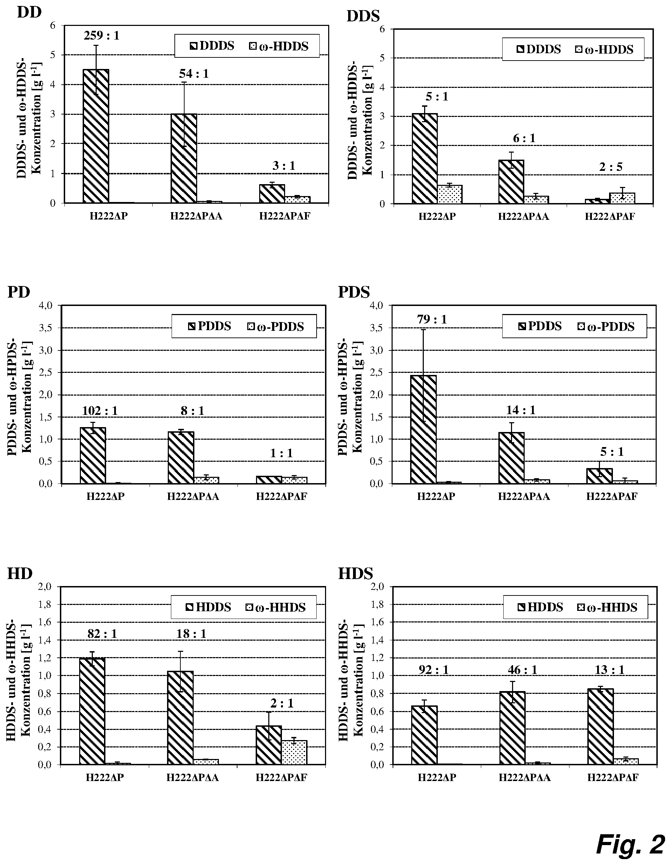

In Fig. 2 wurden *Y. lipolytica* H222ΔP, H222ΔPΔA und H222ΔPΔF in Minimalmedium mit Glycerol und verschiedenen *n*-Alkanen und Fettsäuren (DD: Dodekan; DDS: Dodekansäure; PD: Pentadekan; PDS: Pentadekansäure; HD: Hexadekan; HDS: Hexadekansäure) im Schüttelkolben kultiviert (3 % (v/v) Glycerol + 0,5 % (v/v) Glycerol nach 48 h, 1 % (v/v) *n*-Alkan oder 1 % (v/v) Fettsäure, 20 g l⁻¹ CaCO₃, 17,3 g l⁻¹ KH₂PO₄, 1,35 g l⁻¹ K₂HPO₄ x 3 H₂O, 3 g l⁻¹ (NH4)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Mengen der gebildeten Dicarbonsäuren und ω-Hydroxyfettsäuren (DDDS: Dodekandisäure; ω-HDDS: ω-Hydroxydodekansäure; PDDS: Pentadekandisäure; ω-HPDS: ω-Hydroxypentadekansäure; HDDS: Hexadekandisäure; ω-HHDS: ω-Hydroxyhexadekansäure) wurden nach 96 h Kultivierung mittels Gaschromatographie bestimmt.

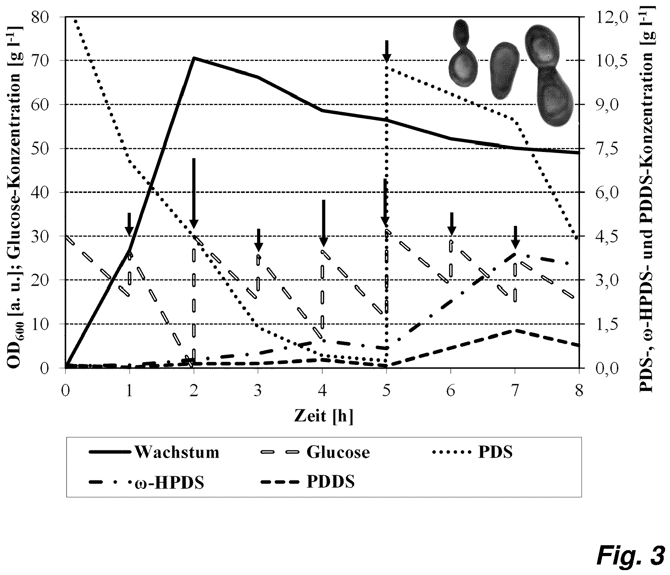

Bei Fig. 3 wurde *Y. lipolytica* H222ΔPΔF in Minimalmedium mit Glucose und Pentadekansäure (PDS) als Kohlenstoffquellen im Schüttelkolben kultiviert (3 % (v/v) Glucose, 1 % (w/v) PDS, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Glucosekonzentration wurde durch regelmäßiges Nachfüttern (Pfeile) zwischen 2 und 3 % (w/v) gehalten. Nach 5 Tagen wurde 1 % (w/v) Pentadekansäure nachgefüttert. Die gebildeten Pentadekansäurederivate, ω-Hydroxypentadekansäure (ω HPDS) und Pentadekandisäure (PDDS), wurden mittels Gaschromatographie quantifiziert. Die Zellen wurden gegen Ende der Kultivierung mikroskopiert, wobei die Fettkörperchen mit Nilrot angefärbt wurden.

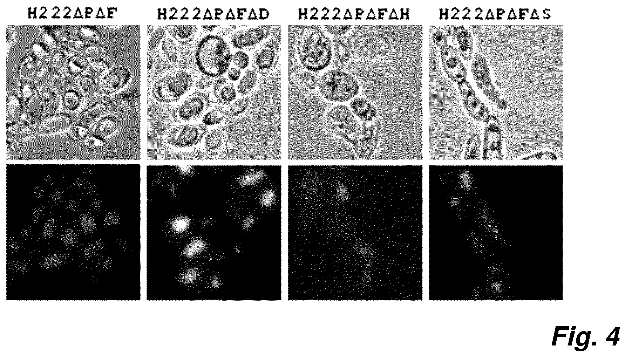

Fig. 4 zeigt die Kultivierung von *Y. lipolytica* H222ΔPΔF, H222ΔPΔFΔD, H222ΔPΔFΔH und H222ΔPΔFΔS in Minimalmedium mit Glucose und Pentadekansäure (PDS) als Kohlenstoffquellen (3% (v/v) Glucose, 1 % (w/v) PDS, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Glucosekonzentration wurde durch regelmäßiges Nachfüttern zwischen 2 und 3% (w/v) gehalten. Nach 3 Tagen wurde 1% (w/v) Pentadekansäure nachgefüttert. Die Zellen wurden nach 5 Tagen mikroskopiert, wobei die Fettkörperchen mit Nilrot angefärbt wurden (oben: lichtmikroskopische Aufnahme; unten: fluoreszenzmikroskopische Aufnahme).

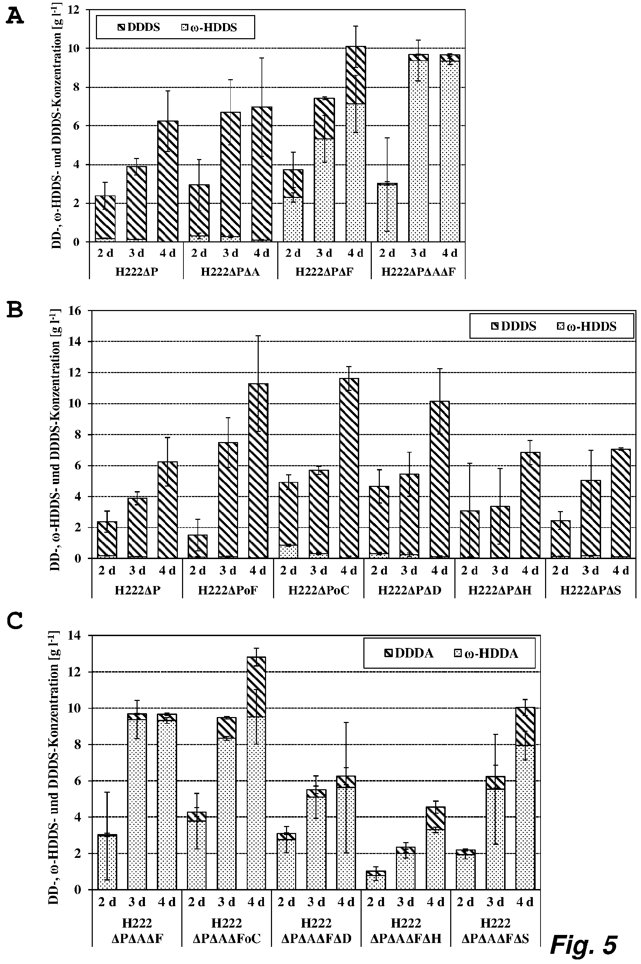

In Fig. 5 wurden *Y. lipolytica* H222ΔP, H222ΔPΔA, H222ΔPΔF, H222ΔPΔAΔF, H222ΔPoF, H222ΔPoC, H222ΔPΔD, H222ΔPΔH, H222ΔPΔS, H222ΔPΔAΔFoC, H222ΔPΔAΔFΔD, H222ΔPΔAΔFΔH und H222ΔPΔAΔFΔS im Fermenter kultiviert. Als Kultivierungmedium diente hierbei Minimalmedium mit Glucose (5 % (w/v) Glucose, 1 g l⁻¹ KH₂PO₄, 0,16 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Kultivierung erfolgte im Fermenter bei 28 °C. Die Sauerstoffsättigung wurde auf 55 % eingestellt. Die Zellen wurden mit einer OD₆₀₀ von 1 inokuliert und für 24 h bei pH 5,5 (automatisierte Titration von HCl und NaOH) inkubiert. Dann wurden 15 g l⁻¹ DD zugegeben und der pH wurde auf 8,0 eingestellt. Alle 24 h wurde Glucose zugegeben, um eine Endkonzentration von 5 % (w/v) einzustellen. Die Mengen der gebildeten Dodekandisäure (DDDS) und ω-Hydroxydodekansäure (ω-HDDS) wurden nach 2 d, 3 d und 4 d Kultivierung mittels Gaschromatographie bestimmt.

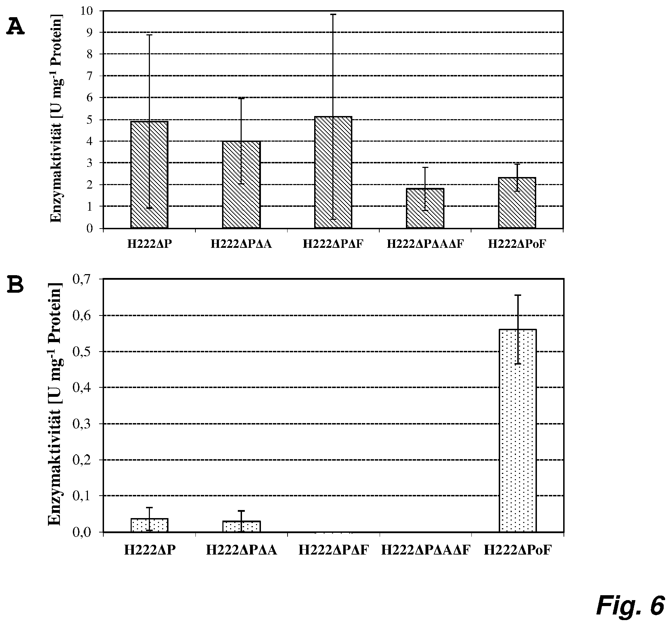

Fig. 6 zeigt die Enzymaktivitäten der (Fett-)Alkohol-Dehydrogenasen und -Oxidase in Zelllysaten der Stämme *Yarrowia lipolytica* H222ΔP, H222ΔPΔA, H222ΔPΔF, H222ΔPΔAΔF und H222ΔPoF. Die Zellen wurden wie in Beispiel 8 beschrieben kultiviert und nach drei Tagen geerntet und aufgeschlossen.

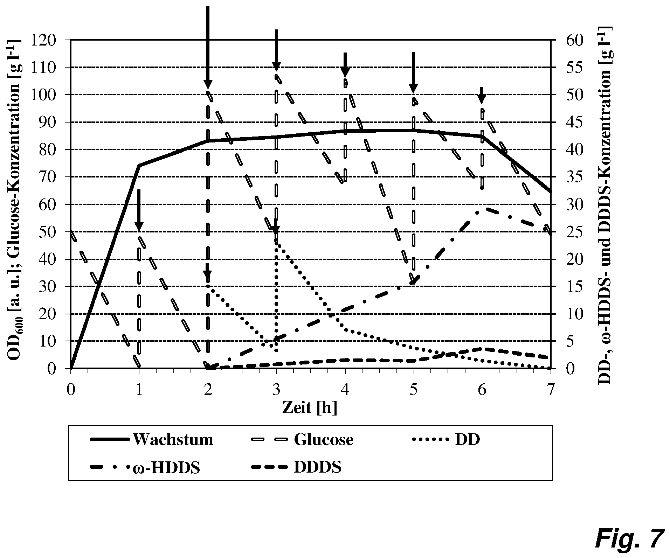

Bei Fig. 7 wurde *Y. lipolytica* H222ΔPΔF in Minimalmedium mit Glucose und Dodekan (DD) als Kohlenstoffquellen im Fermenter kultiviert (5 % (w/v) Glucose, 2 g l⁻¹ KH₂PO₄, 0,32 g l⁻¹ K₂HPO₄ x 3 H₂O, 6 g l⁻¹ (NH₄)₂SO₄, 1,4 g l⁻¹ MgSO₄ x 7 H₂O, 1 g l⁻¹ NaCl, 0,8 g l⁻¹ Ca(NO₃)₂ x 4 H₂O, 1 mg l⁻¹ H₃BO₃, 0,08 mg l⁻¹ CuSO₄ x 5 H₂O, 0,2 mg l⁻¹ Kl, 0,8 mg l⁻¹ MnSO₄ x 4 H₂O, 0,4 mg l⁻¹ Na₂MoO₄ x 2 H₂O, 0,8 mg l⁻¹ ZnSO₄ x 7 H₂O, 12 mg l⁻¹ FeCl₃ x 6 H₂O, 0,6 mg l⁻¹ Thiaminhydrochlorid). Die Glucosekonzentration wurde alle 24 h durch regelmäßiges Nachfüttern (Pfeile) auf 5-10 % (w/v) eingestellt. Nach 2 d wurde der pH auf 8,0 eingestellt und es wurden nach 2 d und 3 d 15 g l⁻¹ Dodekan zugegeben. Alle 24 h wurde Glucose zugegeben, um eine Endkonzentration von 5-10 % (w/v) einzustellen.

### Beispiel 1: Verhinderung des Abbaus von Fettsäuren durch Blockierung der β-Oxidation

Für die Konstruktion der Deletionskassetten für die *POX*-Gene wurde der jeweilige Promotor- und Terminator-Bereich durch PCR amplifiziert, wobei die Primer pXXX_fw/pXXX_rv und XXXt_fw/XXXt_rv genutzt wurden (XXX steht für das zu deletierende Gen). Als Template diente hierbei genomische DNA von *Y. lipolytica* H222.
Durch Verwendung von Überhang-Primern wurde am Ende des Promotor- sowie am Anfang des Terminator-Bereichs eine I-*Sce*I-Restriktionsschnittstelle eingefügt. Promotor- und Terminator-Fragment wurden durch Overlap-PCR mit dem Primern pXXX_fw und XXXt_rv miteinander fusioniert. Das Overlap-Fragment wurde anschließend in den Vektor pJET1.2/blunt (Fermentas; SEQ ID NO 1) ligiert. Das resultierende Plasmid wurde mit I-Scel geschnitten und die loxP-URA3-loxP-Kassette, die aus dem pJMP113-Plasmid (Fickers P, Le Dall MT, Gaillardin C, Thonart P & Nicaud JM (2003) New disruption cassettes for rapid gene disruption and marker rescue in the yeast Yarrowia lipolytica. J Microbiol Methods 55: 727-737.) unter Verwendung des Restiktionsenzyms I-Scel gewonnen wurde, wurde eingefügt.

Die jeweilige Deletionskassette wurde durch PCR oder Restriktion gewonnen und in *Y. lipolytica* H222-S4, der aus *Y. lipolytica* H222 hergestellt werden kann (Mauersberger, S., H. J. Wang, et al. (2001), J Bacteriol 183(17): 5102-5109), nach Barth und Gaillardin (Barth G & Gaillardin C (1996) Yarrowia lipolytica. Springer-Verlag, Berlin, Heidelberg, New York.) transformiert.

Vor einer erneuten Transformation wurde der Marker mithilfe des Cre-Iox-Rekombinationssystems zurückgewonnen (Fickers P, Le Dall MT, Gaillardin C, Thonart P & Nicaud JM (2003) New disruption cassettes for rapid gene disruption and marker rescue in the yeast Yarrowia lipolytica. J Microbiol Methods 55: 727-737.).
Die erfolgreiche Deletion eines Gens wurde durch PCR bestätigt, wobei die Primer pXXX_fw und XXXt_rv sowie pXXX_fw und XXXt_rv2 genutzt wurden. XXXt_rv2 bindet im Bereich des zu deletierenden Gens, jedoch außerhalb der Deletionskassette. Der Stamm, der die Deletionen der Gene *POX1, POX2, POX3, POX4, POX5* und *POX6* trug, wurde H222ΔP genannt und bildet die Grundlage für die Konstruktion von Stämmen, die zur biotechnologischen Produktion ω-Hydroxyfettsäuren und Dicarbonsäuren genutzt werden können.

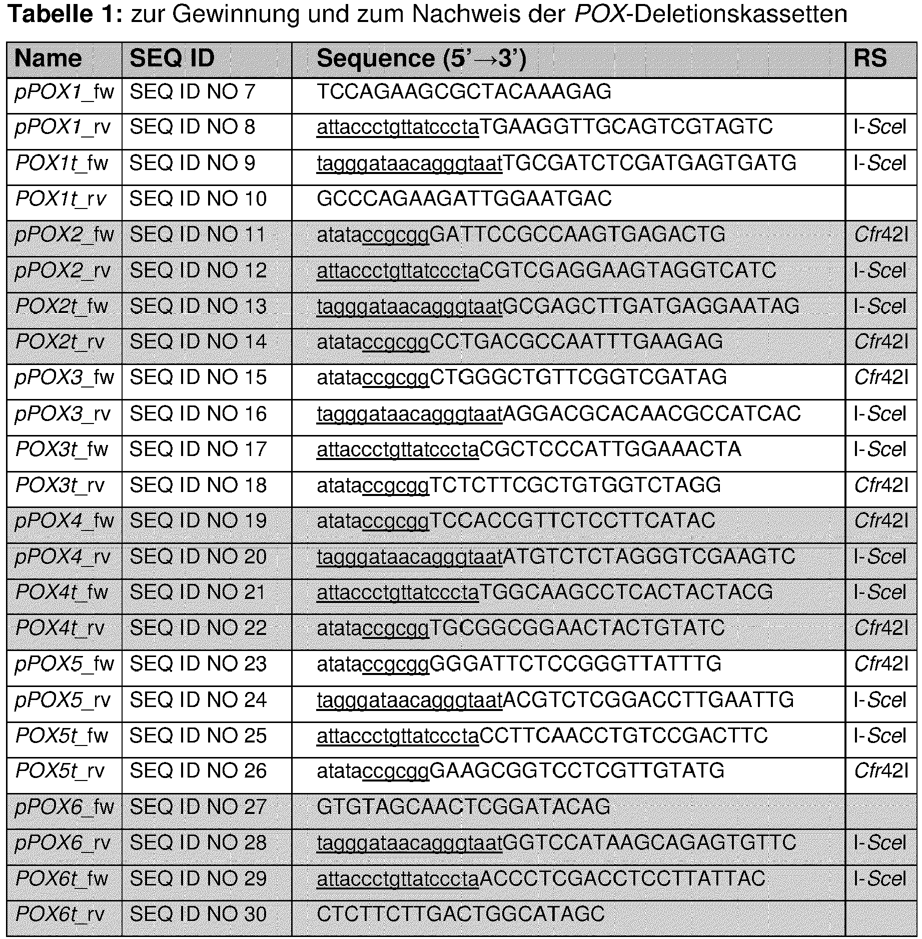

### Beispiel 2: Verhinderung der Oxidation der ω-Hydroxyfettsäuren zum Fettsäure-Aldehyd, bzw. zur Dicarbonsäure im Rahmen der ω-Oxidation

Für die Konstruktion der Deletionskassetten für die (Fett-)Alkohol-Dehydrogenase- und -Oxidase-Gene wurde der jeweilige Promotor- und Terminator-Bereich durch PCR amplifiziert, wobei die Primer pXXX_fw/pXXX_rv und XXXt_fw/XXXt_rv genutzt wurden (XXX steht für das zu deletierende Gen). Als Template diente hierbei genomische DNA von *Y. lipolytica* H222.

Für die Konstruktion der Deletionskassetten für die Gene *ADH1-ADH6* und *FAO1* wurde durch Verwendung von Überhang-Primern am Ende des Promotor- sowie am Anfang des Terminator-Bereichs eine *Bam*HI-Restriktionsschnittstelle eingefügt. Zusätzlich wurde am Anfang des Promotor-Bereichs eine *Hind*III*-*Restriktionsschnittstelle und am Ende des Terminator-Bereichs eine *Nde*I-Restriktionsschnittstelle (*ADH1*: *Not*I*, FAO1*: *Eco*RI) angefügt. Die Fragmente wurden in den Vektor pJET1.2/blunt (Fermentas; SEQ ID NO 1) oder pUCBM21 (Boehringer Ingelheim; SEQ ID NO 2) ligiert. Die so erhaltenen Plasmide wurden mit *Bam*HI linearisiert und der URA-Blaster (TcR-URA3-TcR-Kassette), der aus dem Plasmid pUC-Lys2-DK2(SEQ ID NO 3) durch Restriktion mit *Bam*HI und *Bg*/II gewonnen wurde, wurde eingefügt.

Für die Konstruktion des *ADH7*-Deletionsvektors wurde das vollständige Gen (inklusive des Promotor- und Terminator-Bereichs) mittels PCR amplifiziert, wobei die Primer pADH7_fw und ADH7t_rv verwendet wurden. Das erhaltene Fragment wurde in den Vektor pJET1.2/blunt (Fermentas; SEQ ID NO 1) ligiert, der offene Leserahmen des *ADH7*-Gens wurde durch Restriktion mit *San*DI und *Nsi*I entfernt und der URA-Blaster, der aus dem Plasmid pUC-Lys2-DK2 durch Restriktion mit *San*DI und *Nsi*I gewonnen wurde, wurde eingefügt.

Die jeweilige Deletionskassette wurde durch PCR oder Restriktion gewonnen und in *Y. lipolytica* H222-S4, der aus *Y. lipolytica* H222 hergestellt werden kann (Mauersberger, S., H. J. Wang, et al. (2001), J Bacteriol 183(17): 5102-5109), nach Barth und Gaillardin (Barth G & Gaillardin C (1996) Yarrowia lipolytica. Springer-Verlag, Berlin, Heidelberg, New York.) transformiert.

Vor einer erneuten Transformation wurde der Marker per FOA-Selektion (Boeke JD, LaCroute F & Fink GR (1984) Mol Gen Genet 197: 345-346.) zurückgewonnen.
Die erfolgreiche Deletion eines Gens wurde durch PCR bestätigt, wobei die Primer pXXX_fw und XXXt_rv sowie pXXX_fw und XXXt_rv2 genutzt wurden. XXXt_rv2 bindet im Bereich des zu deletierenden Gens, jedoch außerhalb der Deletionskassette. Der Stamm, der sowohl die Deletionen der Gene *POX1, POX2, POX3, POX4, POX5* und *POX6 als auch FADH*, *AHD1*, *AHD2*, *AHD3*, *AHD4*, *AHD5*, *AHD6* und *AHD7* trug, wurde H222ΔPΔA genannt.

Der Stamm, der sowohl die Deletionen der Gene *POX1, POX2, POX3, POX4, POX5* und *POX6* als auch *FAO1* trug, wurde H222ΔPΔF genannt.

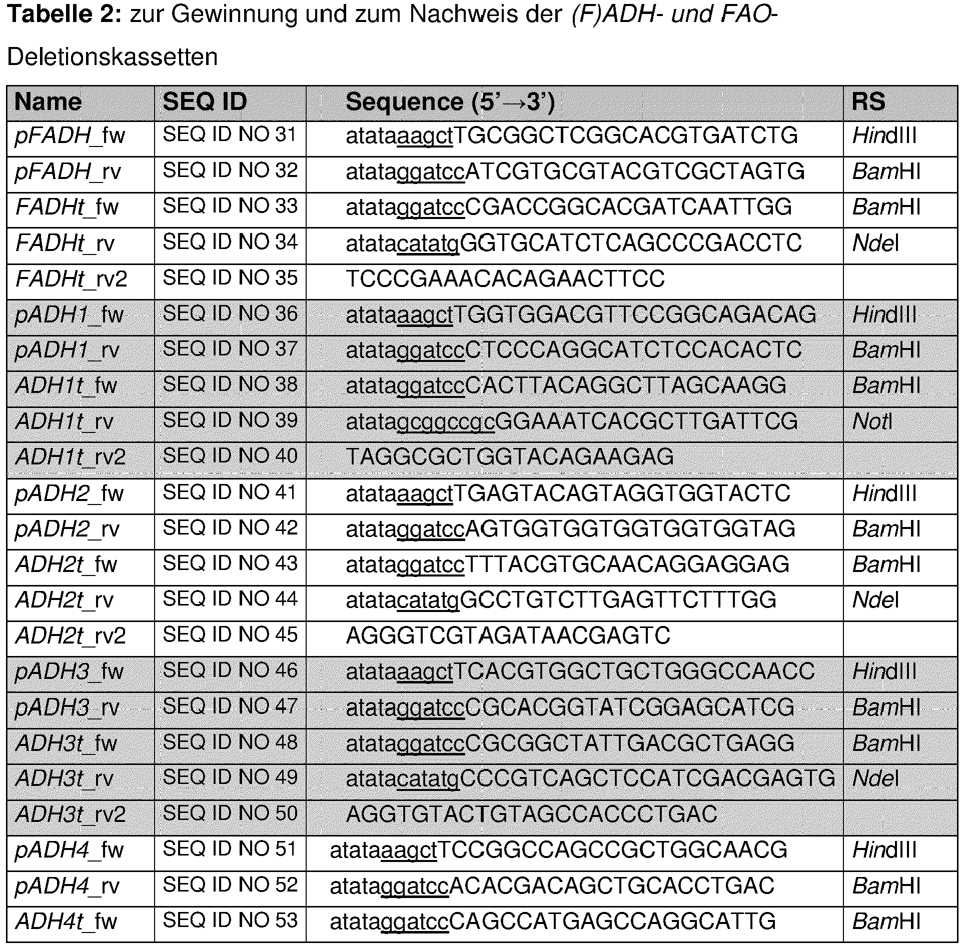

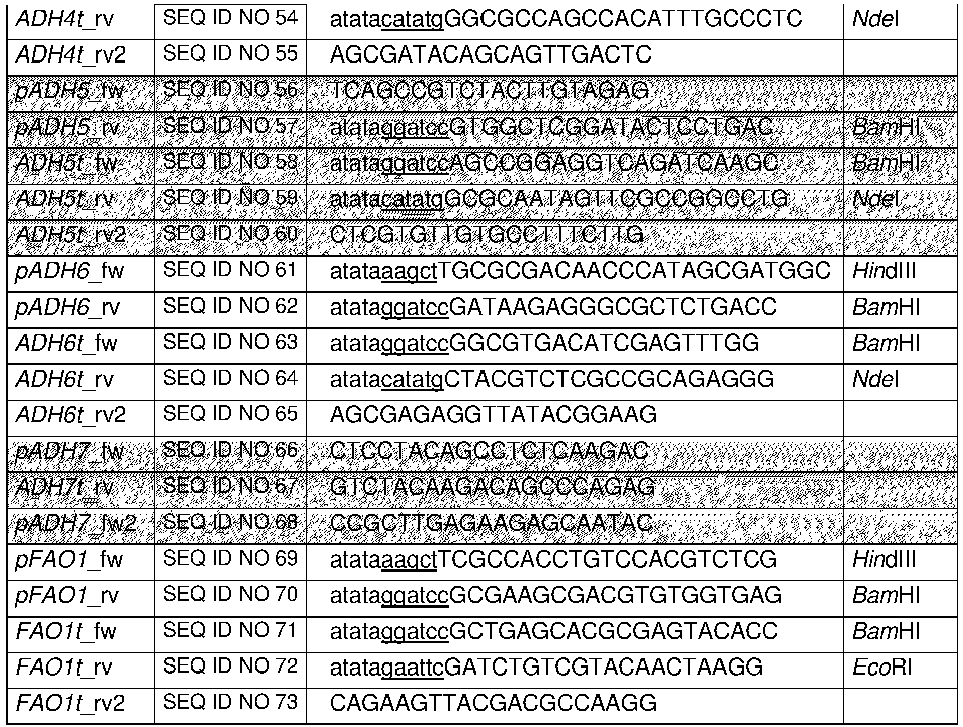

### Beispiel 3: Vergleichende Kultivierung verschiedener Produktionsstämme im Schüttelkolben

Um zu überprüfen, ob die erzeugten Ausgangsstämme *Yarrowia lipolytica* H222ΔP, H222ΔPΔA und H222ΔPΔF zur Produktion größerer Mengen an ω-Hydroxyfettsäuren und Dicarbonsäuren befähigt sind, wurden diese in Minimalmedium mit Glycerol und verschiedenen *n*-Alkanen (Dodekan, Pentadekan, Hexadekan) und Fettsäuren (Dodekansäure, Pentadekansäure, Hexadekansäure) kultiviert (3 % (v/v) Glycerol + 0,5 % (v/v) Glycerol nach 48 h, 1 % (v/v) *n*-Alkan oder 1 % (v/v) Fettsäure, 20 g l⁻¹ CaCO₃, 17,3 g l⁻¹ KH₂PO₄, 1,35 g l⁻¹ K₂HPO₄ x 3 H₂O, 3g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Mengen der gebildeten ω-Hydroxyfettsäuren (ω-Hydroxydodekansäure, ω-Hydroxypentadekansäure, ω-Hydroxyhexadekansäure) und Dicarbonsäuren (Dodekandisäure, Pentadekandisäure, Hexadekandisäure) wurden nach 96 h Kultivierung mittels Gaschromatographie bestimmt (Fig. 2).

*Y. lipolytica* H222ΔP bildete hierbei relativ große Mengen an Dicarbonsäuren und kann somit als Ausgangsstamm zur Konstruktion eines entsprechenden Produktionsstamms für Dicarbonsäuren genutzt werden.

Sowohl *Y. lipolytica* H222ΔPΔA als auch H222ΔPΔF bildeten gesteigerte Mengen an ω-Hydroxyfettsäuren und können somit als Ausgangsstämme zur Konstruktion entsprechender Produktionsstämme für ω-Hydroxyfettsäuren genutzt werden. *Y. lipolytica* H222ΔPΔF ist hierbei gegenüber H222ΔPΔA zu bevorzugen.

Sowohl *n*-Alkane als auch Fettsäuren können von allen Stämmen zur ω-Hydroxyfettsäure, bzw. Dicarbonsäure mit gleicher Kettenlänge umgesetzt werden.

### Beispiel 4: Nutzung verschiedener Kultivierungsbedingungen und -medien

Verschiedene Stämme (z.B. *Yarrowia lipolytica* H222ΔP und H222ΔPΔF) wurden in unterschiedlichen Medien unter verschiedenen Bedingungen kultiviert, wobei sich herausstellte, dass die verwendeten Medien und Bedingungen unterschiedlich gut geeignet sind.

Die Hefen wurden vergleichend in Minimalmedium mit Glucose (3 % (w/v) Glucose, 1 % (w/v) Pentadekansäure, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid) und Glycerol (3 % (v/v) Glycerol, 1 % (w/v) Pentadekansäure, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid) als Kohlenstoffquelle kultiviert, wobei nach 48 h 1 % (w/v) Glucose, bzw. 1 % (v/v) Glycerol nachgefüttert wurden. Nach 96 h bildeten alle untersuchten Stämme (z.B. *Y. lipolytica* H222ΔP, H222ΔPΔA und H222ΔPΔF) bei Wachstum in Minimalmedium mit Glucose größere Mengen an ω-Hydroxypentadekansäure, bzw. Pentadekandisäure als bei Wachstum in Minimalmedium mit Glycerol.

Die Hefen wurden weiterhin in Vollmedium mit Glucose und Pentadekansäure kultiviert (1 % (w/v) Hefeextrakt, 2 % (w/v) Pepton, 2 % (w/v) Glucose und 1 % (w/v) Pentadekansäure in 1 % Tween 80), wobei 1 % (w/v) Glucose nachgefüttert wurden, wenn diese aufgebraucht war. Nach 4 Tagen wurden 1 % (w/v) Pentadekansäure in 1 % Tween 80 nachgefüttert. Unter diesen Bedingungen wurde nahezu keine ω-Hydroxypentadekansäure, bzw. Pentadekandisäure gebildet (je < 0,1 g l⁻¹).

Die Hefen wurden in Minimalmedium mit Glucose und Pentadekansäure kultiviert (3 % (v/v) Glucose, 1 % (w/v) Pentadekansäure in 1 % Tween 80, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid), wobei 1 % (w/v) Glucose nachgefüttert wurden, wenn diese aufgebraucht war. Nach 4 Tagen wurden 1 % (w/v) Pentadekansäure in 1 % Tween 80 nachgefüttert. Unter diesen Kultivierungsbedingungen bildete der Stamm *Y. lipolytica* H222ΔPΔF nach 10 Tagen rund 0,7 g l⁻¹ ω-Hydroxypentadekansäure und 2,4 g l⁻¹ Pentadekandisäure.

Die Hefen wurden in Minimalmedium mit Glucose und Pentadekansäure kultiviert (3 % (v/v) Glucose, 1 % (w/v) Pentadekansäure in 1 % Tween 80, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ Kl, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid), wobei die Glucose so nachgefüttert wurde, dass deren Konzentration im Medium zwischen 1 und 3 % (w/v) Iag. Nach 5 Tagen wurden 1 % (w/v) Pentadekansäure in 1 % Tween 80 nachgefüttert. Unter diesen Kultivierungsbedingungen bildete der Stamm *Y. lipolytica* H222ΔPΔF nach 7 Tagen rund 3,9 g l⁻¹ ω-Hydroxypentadekansäure und 1,3 g l⁻¹ Pentadekandisäure.

Zusammenfassend ist festzustellen, dass Minimalmedium für die Kultivierung besser geeignet ist als Vollmedium. Weiterhin ist Glucose als Kohlenstoffquelle für die Energiebereitstellung besser geeignet als Glycerol. Die Glucose sollte im Verlauf der Kultivierung derart nachgefüttert werden, dass die Glucose nicht aufgebraucht wird (d. h. dass deren Konzentration nicht auf 0 g l⁻¹ absinkt).

### Beispiel 5: Reduktion der Bildung vom Fettkörperchen

Für die Konstruktion der Deletionskassetten für die Gene *DGA1, PAH1* und *SCT1* wurde der jeweilige Promotor- und Terminator-Bereich durch PCR amplifiziert, wobei die Primer pXXX_fw/pXXX_rv und XXXt_fw/XXXt_rv genutzt wurden (XXX steht für das zu deletierende Gen). Als Template diente hierbei genomische DNA von *Y*. *lipolytica* H222.

Für die Konstruktion der Deletionskassetten für die Gene *DGA1, PAH1* und *SCT1* wurde durch Verwendung von Überhang-Primern am Ende des Promotor- sowie am Anfang des Terminator-Bereichs eine *Bam*HI-Restriktionsschnittstelle eingefügt. Zusätzlich wurde am Anfang des Promotor-Bereichs eine *Hin*dIII-Restriktionsschnittstelle und am Ende des Terminator-Bereichs eine *Eco*RI-Restriktionsschnittstelle angefügt. Die Fragmente wurden in den Vektor pUCBM21 (Boehringer Ingelheim; SEQ ID NO 2) ligiert. Die so erhaltenen Plasmide wurden mit *Bam*HI linearisiert und der URA-Blaster (TcR-URA3-TcR-Kassette), der aus dem Plasmid pUC-Lys2-DK2(SEQ ID NO 3) durch Restriktion mit *Bam*HI und *Bgl*II gewonnen wurde, wurde eingefügt. Die jeweilige Deletionskassette wurde durch PCR oder Restriktion gewonnen und in *Y. lipolytica* H222-S4, der aus *Y. lipolytica* H222 hergestellt werden kann (Mauersberger, S., H. J. Wang, et al. (2001), J Bacteriol 183(17): 5102-5109), nach Barth und Gaillardin (Barth G & Gaillardin C (1996) Yarrowia lipolytica. Springer-Verlag, Berlin, Heidelberg, New York.) transformiert.

Vor einer erneuten Transformation wurde der Marker per FOA-Selektion (Boeke JD, LaCroute F & Fink GR (1984) Mol Gen Genet 197: 345-346.) zurückgewonnen.

Die erfolgreiche Deletion eines Gens wurde durch PCR bestätigt, wobei die Primer pXXX_fw und XXXt_rv sowie pXXX_fw und XXXt_rv2 genutzt wurden. XXXt_rv2 bindet im Bereich des zu deletierenden Gens, jedoch außerhalb der Deletionskassette.

Der Stamm, der sowohl die Deletionen der Gene *POX1, POX2, POX3, POX4, POX5* und *POX6* als auch *DGA1* trug, wurde H222ΔPΔD genannt.

Der Stamm, der sowohl die Deletionen der Gene *POX1, POX2, POX3, POX4, POX5* und *POX6* als auch *PAH1* trug, wurde H222ΔPΔH genannt.

Der Stamm, der sowohl die Deletionen der Gene *POX1, POX2, POX3, POX4, POX5* und *POX6* als auch *SCT1* trug, wurde H222ΔPΔS genannt.

Der Stamm, der sowohl die Deletionen der Gene *POX1, POX2, POX3, POX4, POX5* und *POX6* als auch *FAO1* und *DGA1* trug, wurde H222ΔPΔFΔD genannt.

Der Stamm, der sowohl die Deletionen der Gene *POX1, POX2, POX3, POX4, POX5* und *POX6* als auch *FAO1* und *PAH1* trug, wurde H222ΔPΔFΔH genannt.

Der Stamm, der sowohl die Deletionen der Gene *POX1, POX2, POX3, POX4, POX5* und *POX6* als auch *FAO1* und *SCT1* trug, wurde H222ΔPΔFΔS genannt.

Der Stamm, der sowohl die Deletionen der Gene *POX1, POX2, POX3, POX4, POX5* und *POX6* als auch *FADH, ADH1, ADH2, ADH3, ADH4, ADH5, ADH6, ADH7, FAO1* und *DGA1* trug, wurde H222ΔPΔAΔFΔD genannt.

Der Stamm, der sowohl die Deletionen der Gene *POX1, POX2, POX3, POX4, POX5* und *POX6* als auch *FADH, ADH1, ADH2, ADH3, ADH4, ADH5, ADH6, ADH7, FAO1* und *PAH1* trug, wurde H222ΔPΔAΔFΔH genannt.

Der Stamm, der sowohl die Deletionen der Gene *POX1, POX2, POX3, POX4, POX5* und *POX6* als auch *FADH, ADH1, ADH2, ADH3, ADH4, ADH5, ADH6, ADH7, FAO1* und *SCT1* trug, wurde H222ΔPΔAΔFΔS genannt.

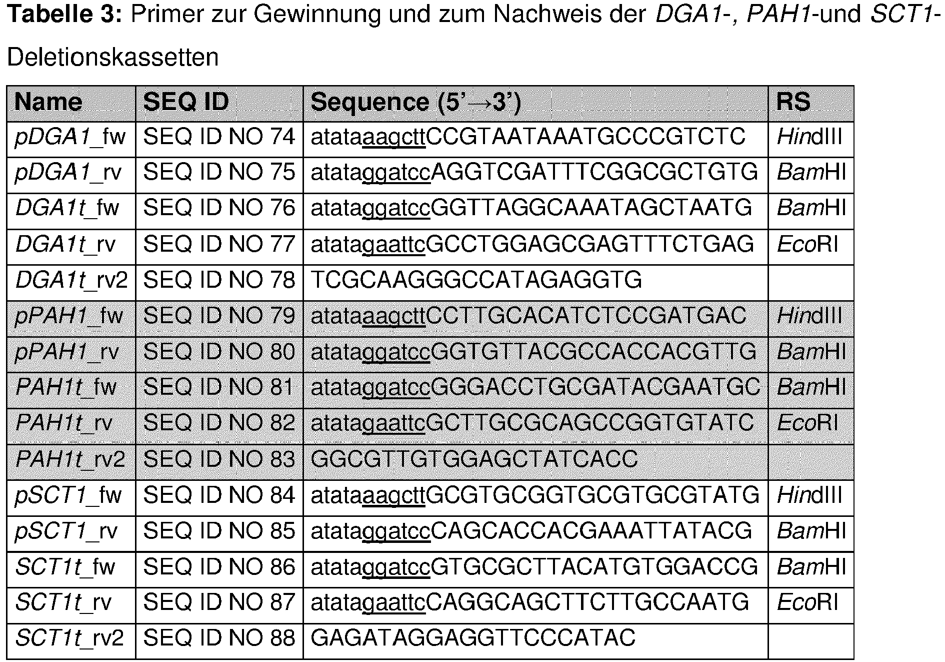

Die Stämme *Y. lipolytica* H222ΔPΔFΔD,H222ΔPΔFΔH und H222ΔPΔFΔS sowie deren Ausgangsstamm *Y. lipolytica* H222ΔPΔF wurden in Minimalmedium mit Glucose und Pentadekansäure kultiviert (3 % (v/v) Glucose, 1 % (w/v) Pentadekansäure, 17,3 g I⁻¹ KH₂PO₄, 1,35 g I⁻¹ K₂HPO₄ x 3 H₂O, 3 g I⁻¹ (NH₄)₂SO₄, 0,7 g I⁻¹ MgSO₄ x 7 H₂O, 0,5 g I⁻¹ NaCl, 0,4 g I⁻¹ Ca(NO₃)₂ x 4 H₂O, 0,5 mg I⁻¹ H₃BO₃, 0,04 mg I⁻¹ CuSO₄ x 5 H₂O, 0,1 mg I⁻¹ KI, 0,4 mg I⁻¹ MnSO₄ x 4 H₂O, 0,2 mg I⁻¹ Na₂MoO₄ x 2 H₂O, 0,4 mg I⁻¹ ZnSO₄ x 7 H₂O, 6 mg I⁻¹ FeCl₃ x 6 H₂O, 0,3 mg I⁻¹ Thiamin-Hydrochlorid). Alle 24 h wurde die Glucose-Konzentration auf 3 % (w/v) eingestellt und nach 3 Tagen wurde 1 % (w/v) Pentadekansäure nachgefüttert. Die Mengen an ω-Hydroxypentadekansäure und Pentadekandisäure wurden nach 8 Tagen Kultivierung mittels Gaschromatographie bestimmt. Die Zellen wurden mikroskopisch untersucht, wobei die Fettkörperchen mit Nilrot angefärbt wurden und somit fluoreszenzmikroskopisch detektierbar sind (Fig. 4).

Hierbei ist zu erkennen, dass die Größe der Fettkörperchen durch die Deletionen von *PAH1* und *SCT1* unter den genannten Kultivierungsbedingungen deutlich reduziert war. Dies konnte, wie bereits gezeigt, nicht für die bereits beschriebene Deletion von *DGA1* (Thevenieau F (2006) Institut National Agronomique Paris-Grignon, PhD thesis.; Athenstaedt K (2011) Biochim Biophys Acta 1811: 587-596.)festgestellt werden.

Die Verfügbarkeit von Stämmen mit verkleinerten Fettkörperchen bildet die Grundlage für die Entwicklung von Stämmen für die effiziente Produktion von ω-Hydroxyfettsäuren und Dicarbonsäuren, da die Veresterung der angebotenen Substrate mit Glycerol und deren Einlagerung in den Fettkörperchen die Umsatzrate verringert.

### Beispiel 6: Erhöhte Expression von FAO1 in Yarrowia lipolytica H222ΔP

Für die Konstruktion eines Vektors zu Überexpression von *FAO1* wurde ein Teil des konstitutiven Promotors des Translationselongationsfaktor 1 alpha-Gens (*TEF1: YALI0C09141g*) mittels PCR gewonnen, wobei die Primer pTef_Spel_fw3 und pTef_FAOo_ol_rv genutzt wurden. Als Template diente hierbei das Plasmid pINTB_HMG1 (SEQ ID NO 5).

Weiterhin wurde das *FAO1*-Gen(YALI0B14014g) mittels PCR unter Verwendung der Primer pTef_FAOo_ol_fw und FAO1o_Sphl_rv amplifiziert (Template: genomische DNA von *Y. lipolytica* H222).

Durch die Primer pTef_FAOo_ol_rv und pTef_FAOo_ol_fw wurde ein Überhangbereich angefügt, mit dessen Hilfe beide PCR-Fragmente mittels Overlap-PCR unter Verwendung der Primer pTef_Spel_fw3 und FAO1o_Sphl_rv verbunden wurden. Das Overlap-Fragment wurde anschließend in den Vektor pJET1.2/blunt (Fermentas; SEQ ID NO 1) ligiert, aus diesem unter Verwendung der Restriktionsenzyme *Spe*I und *Sph*I herausgeschnitten und letztendlich in das mit *Spe*I und *Sph*I geschnittene Rückgrat des Plasmids pINTB_HMG1 (SEQ ID NO 5) ligiert, wobei des Plasmid pINTB-FAO1 entstand.

Weiterhin wurde eine Integrationplattform mittels PCR unter Verwendung der Primer INT_AscI_fw_KpnI und INT_AscI_rv_KpnI gewonnen, mit Kpnl geschnitten und in das durch Restriktionsverdau mit Kpnl gewonnene Rückgrat des Vektors pINTB-FAO1 ligiert, wobei das Plasmid pINTC-FAO1 entstand.

Das Plasmid pINTC-FAO1 wurde letztendlich mit Ascl linearisiert und in den gewünschten Y. lipolytica-Empfängerstamm transformiert.

Die erfolgreiche Integration des FAO1-Gens wurde durch PCR bestätigt, wobei die Primer INT_Ascl_fw_out und INT_AscI_rv_out genutzt wurden, die in der genomischen DNA außerhalb des integrierten Konstruktes binden.

Der Stamm, der sowohl die Deletionen der Gene POX1, POX2, POX3, POX4, POX5 und POX6 als auch die zusätzliche Kopie von FAO1 unter Kontrolle des TEF1-Promotors trug, wurde H222ΔPoF genannt und diente als Ausgangsstamm zu Konstruktion weiterer Stämme für die Produktion von Dicarbonsäuren.

**Tabelle 4: Primer für die Überexpression von FAO1**

| **Name** | **SEQ ID** | **Seqouence (5'→3')** | **RS** |
|---|---|---|---|
| pTef_Spel_fw3 | SEQ ID NO 89 | CTACGCTTGTTCAGACTTTG | |
| pTef_FAOo_ol_rv | SEQ ID NO 90 | gtgtgcttgtcgtcagacatTTTGAA TGATTCTTATACTCAGAAGG | |
| pTef_FAOo_ol_fw | SEQ ID NO 91 | ccttctgagtataagaatcattcaaa ATGTCTGACGACAAGCACAC | |
| FAO1o-Sphl-rv | SEQ ID NO 92 | gcatgcTTAGATTCGAGGTCGGAGAT | *Sph*I |
| INT_Ascl_fw_Kpnl | SEQ ID NO 93 | qqtacCACGCACGGATAGTTTATCCA | *Kpn*I |
| INT_Ascl_rv_Kpnl | SEQ ID NO 94 | ggtacCCAAAGTCAACTAATGTCAAG TAAAG | *Kpn*I |
| INT_Ascl_fw_out | SEQ ID NO 95 | CCTCCAACGTGACTTTC | |
| INT_Ascl_rv_out | SEQ ID NO 96 | AGAGACCTCCCACAAAG | |

### Beispiel 7: Erhöhte Expression von CPR1 in Yarrowia lipolytica H222ΔP und H222ΔPΔAΔF

Für die Konstruktion eines Vektors zu Überexpression von *FAO1* wurde ein Teil des konstitutiven Promotors des Translationselongationsfaktor 1 alpha-Gens (*TEF1: YALI0C09141g*) mittels PCR gewonnen, wobei die Primer pTef_Spel_fw3 und pTEF_CPR1_ol_rv genutzt wurden. Als Template diente hierbei das Plasmid pINTB_HMG1 (SEQ ID NO 5).

Weiterhin wurde das *CPR1*-Gen(YALI0D04422g) mittels PCR unter Verwendung der Primer pTEF_CPR1_ol_fw und CPR1_SphI_rv amplifiziert (Template: genomische DNA von *Y. lipolytica* H222).

Durch die Primer pTEF_CPR1_ol_rv und pTEF_CPR1_ol_fw wurde ein Überhangbereich angefügt, mit dessen Hilfe beide PCR-Fragmente mittels Overlap-PCR unter Verwendung der Primer pTef_Spel_fw3 und CPR1_Sphl_rv verbunden wurden. Das Overlap-Fragment wurde anschließend in den Vektor pJET1.2/blunt (Fermentas; SEQ ID NO 1) ligiert, aus diesem unter Verwendung der Restriktionsenzyme *Spe*I und *Sph*I herausgeschnitten und letztendlich in das mit *Spe*I und *Sph*I geschnittene Rückgrat des Plasmids pINTB_HMG1 (SEQ ID NO 5) ligiert, wobei das Plasmid pINTB-CPR1 entstand.

Das resultierende Plasmid wurde letztendlich mit *Not*I linearisiert und in die gewünschten *Y*. *lipolytica*-Empfängerstämme transformiert.

Die erfolgreiche Integration des *CPR1-Gens* wurde durch PCR bestätigt, wobei die Primer INTB_out_fw und INTB_out_rv genutzt wurden, die in der genomischen DNA außerhalb des integrierten Konstruktes binden.

Der Stamm, der sowohl die Deletionen der Gene *POX1, POX2, POX3, POX4, POX5* und *POX6* als auch die zusätzliche Kopie von *CPR1* unter Kontrolle des *TEF*1-Promotors trug, wurde H222ΔPoC genannt und diente als Ausgangsstamm zu Konstruktion weiterer Stämme für die Produktion von Dicarbonsäuren.

Der Stamm, der sowohl die Deletionen der Gene *POX1, POX2, POX3, POX4, POX5, POX6, FADH, ADH1, ADH2, ADH3, ADH4, ADH5, ADH6, ADH7, FAO1* als auch die zusätzliche Kopie von *CPR1* unter Kontrolle des *TEF*1-Promotors trug, wurde H222ΔPΔAΔFoC genannt.

**Tabelle 5: Primer für die Überexpression von CPR1**

| **Name** | **SEQ ID** | **Sequence (5'→3')** | **RS** |
|---|---|---|---|
| pTef_Spel_fw3 | SEQ ID NO 89 | CTACGCTTGTTCAGACTTTG | |
| pTEF_CPR1_ol_rv | SEQ ID NO 97 | AGAGAGTCGAGTAGAGCCATTTT GAATGATTCTTATACTCAGAAGG | |
| pTEF_CPR1_ol_fw | SEQ ID NO 98 | CCTTCTGAGTATAAGAATCATTC AAAATGGCTCTACTCGACTCTCT | |
| CPR1_Sphl_rv | SEQ ID NO 99 | atatagcatgcCTACCACACATC TTCCTGGTAGAC | *Sph*I |
| INTB_out_fw | SEQ ID NO | CTCAAGATACGGCATTGG | |
| INTB_out_rv | SEQ ID NO | TCCTTGGCTAGACGAATG | |

### Beispiel 8: Vergleichende Kultivierung verschiedener Produktionsstämme im Fermenter

Um zu überprüfen, inwieweit sich verschiedene gentechnische Modifikationen auf die Produktion von ω-Hydroxyfettsäuren und/oder Dicarbonsäuren auswirken, wurden die Stämme *Yarrowia lipolytica* H222ΔP, H222ΔPΔA, H222ΔPΔF, H222ΔPΔAΔF, H222ΔPoF, H222ΔPoC, H222ΔPΔD, H222ΔPΔH, H222ΔPΔS, H222ΔPΔAΔFoC, H222ΔPΔAΔFΔD, H222ΔPΔAΔFΔH und H222ΔPΔAΔFΔS, deren Konstruktion in den Beispielen 1,2,5,6 und 7 beschrieben wurde, im Fermenter kultiviert. Als Kultivierungmedium diente hierbei Minimalmedium mit Glucose (5 % (w/v) Glucose, 1 g I⁻¹ KH₂PO₄, 0,16 g I⁻¹ K₂HPO₄ x 3 H₂O, 3 g I⁻¹ (NH₄)₂SO₄, 0,7 g I⁻¹ MgSO₄ x 7 H₂O, 0,5 g I⁻¹ NaCl, 0,4 g I⁻¹ Ca(NO₃)₂ x 4 H₂O, 0,5 mg I⁻¹ H₃BO₃, 0,04 mg I⁻¹ CuSO₄ x 5 H₂O, 0,1 mg I⁻¹ KI, 0,4 mg I⁻¹ MnSO₄ x 4 H₂O, 0,2 mg I⁻¹ Na₂MoO₄ x 2 H₂O, 0,4 mg I⁻¹ ZnSO₄ x 7 H₂O, 6 mg I⁻¹ FeCl₃ x 6 H₂O, 0,3 mg I⁻¹ Thiaminhydrochlorid). Die Kultivierung erfolgte im Fermenter bei 28 °C. Die Sauerstoffsättigung wurde auf 55 % eingestellt. Die Zellen wurden mit einer OD₆₀₀ von 1 inokuliert und für 24 h bei pH 5,5 (automatisierte Titration von HCl und NaOH) inkubiert. Dann wurden 15 g I⁻¹ DD zugegeben und der pH wurde auf 8,0 eingestellt. Alle 24 h wurde Glucose zugegeben, um eine Endkonzentration von 5 % (w/v) einzustellen.

*Y. lipolytica* H222ΔP bildete hierbei relativ große Mengen an Dicarbonsäuren und kann somit als Ausgangsstamm zur Konstruktion eines entsprechenden Produktionsstamms für Dicarbonsäuren genutzt werden (Fig. 5A).

*Y. lipolytica* H222ΔPΔA als auch H222ΔPΔF bildeten gesteigerte Mengen an ω-Hydroxyfettsäuren und können somit als Ausgangsstämme zur Konstruktion entsprechender Produktionsstämme für ω-Hydroxyfettsäuren genutzt werden. *Y*. *lipolytica* H222ΔPΔF ist hierbei gegenüber H222ΔPΔA zu bevorzugen(Fig. 5A).

*Y. lipolytica* **H222ΔPΔAΔF** bildete nur geringe Mengen Dicarbonsäuren und ist somit der bevorzugte Ausgangsstamm zur Konstruktion von Produktionsstämmen für ω-Hydroxyfettsäuren (Fig. 5A).

Die Überexpressionen von *FAO1* und *CPR1* in dem Stamm *Y. lipolytica* H222ΔP (resultierende Stämme: *Y. lipolytica* H222ΔPoF und H222ΔPoC) führte jeweils zu einer gesteigerten Produktion von Dicarbonsäuren (Fig. 5B).

Die Deletion von *DGA1* in dem Stamm *Y. lipolytica* H222ΔP (resultierender Stamm: *Y. lipolytica* H222ΔPΔD) führte zu einer gesteigerten Produktion von Dicarbonsäuren, wohingegen die Deletionen von *PAH1* und *SCT1* (resultierende Stämme: *Y. lipolytica* H222ΔPΔH und H222ΔPΔS) die Produktion von Dicarbonsäuren nicht signifikant steigerte (Fig. 5B).

Die Überexpression von *CPR1* in dem Stamm *Y. lipolytica* **H222ΔPΔAΔF** (resultierender Stamm: *Y. lipolytica* H222ΔPΔAΔFoC) führte zu einer gesteigerten Produktion von Dicarbonsäuren, nicht jedoch von ω-Hydroxyfettsäuren (Fig. 5C).

Die Deletionen von *DGA1, PAH1* und *SCT1* in dem Stamm *Y. lipolytica* **H222ΔPΔAΔF** (resultierende Stämme: *Y. lipolytica* H222ΔPΔAΔFΔD, H222ΔPΔAΔFΔH und H222ΔPΔAΔFΔS) führte nicht zu einer gesteigerten Produktion von ω-Hydroxyfettsäuren (Fig. 5B).

### Beispiel 9: Enzymtest zur Bestimmung der (Fett-)Alkohol-Dehydrogenase und - Oxidase-Aktivität

Die Enzymaktivitäten der (Fett-)Alkohol-Dehydrogenasen und -Oxidase wurden in Zelllysaten der Stämme *Yarrowia lipolytica* H222ΔP, H222ΔPΔA, H222ΔPΔF, **H222ΔPΔAΔF** und H222ΔPoF bestimmt. Die Zellen wurden wie in Beispiel 8 beschrieben kultiviert und nach drei Tagen geerntet und aufgeschlossen.

Die (Fett-)Alkohol-Dehydrogenase- und -Oxidase-Enzymaktivitäten wurden wie bei Matatiele (2005) beschrieben durchgeführt (Matatiele PR (2005) PhD thesis, University of the Free State, Republic of South Africa).

Das (Fett-)Alkohol-Dehydrogenase-Assay (50 mM Tris-HCI pH 8,5, 1,3 mM Dodekan-1-ol in DMSO, 2 mM NAD⁺, 2 mM NAP⁺, 1,5 % (v/v) Zellextrakt) wurde mit einem wurde mit einem aufzeichnenden Spektrophotometer bei 30 °C und λ = 340 nm eingemessen und die Enzymaktivität wurde mithilfe des bestimmten Extinktions-Anstiegs berechnet (ε_{NAD(P)H} = 6,3 mM⁻¹ cm⁻¹).

Das (Fett-)Alkohol-Oxidase-Assay (50 mM Glycin-NaOH pH 9,0, 0,35 mM Dodekan-1-ol in DMSO, 0,013 % (w/v) Peroxidase (150 U/mg), 0,044 % (w/v) ABTS, 6 mM Natriumazid, 1-5 % (v/v) Zellextrakt) wurde mit einem wurde mit einem aufzeichnenden Spektrophotometer bei λ = 405 nm eingemessen und die Enzymaktivität wurde mithilfe des bestimmten Extinktions-Anstiegs berechnet (ε_{ABTSox} = 18,4 mM⁻¹ cm⁻¹).
Überraschenderweise wurde zwischen den Stämmen keinen deutlicher Unterschied der (Fett-)Alkohol-Dehydrogenase festgestellt (Fig. 6A). Dies könnte mit verbleibender Alkohol-Dehydrogenase-Aktivität begründet werden. Weiterhin ist es möglich, dass die gemessene Umsetzung Dodekan-1-ol auch durch Cytochrom P450 katalysiert und letztendlich die Aktivität von Aldehyd-Dehydrogenasen bestimmt wird.

Wie erwartet war die (Fett-)Alkohol-Oxidase-Aktivität nur in den Stämmen *Y. lipolytica* H222ΔP und H222ΔPΔA nachweisbar und nicht in den *FAO1*-Deletionsstämmen *Y*. *lipolytica* H222ΔPΔF und H222ΔPΔAΔF (Fig. 6B). Die gemessene (Fett-)Alkohol-Oxidase-Aktivität war durch die Überexpression von *FAO1* (Stamm: *Y. lipolytica* H222ΔPoF) mehr als zehnfach erhöht.

### Beispiel 9: Kultivierung Y. lipolytica H222ΔPΔF im Fermenter

Um zu überprüfen, ob die Produktionsstämme in der Lage sind, auch größere Mengen an Dicarbonsäuren und/oder ω-Hydroxyfettsäuren zu produzieren und im Kulturmadium anzuhäufen, wurde der Stamm *Y. lipolytica* H222ΔPΔF für 7 d im Fermenter kultiviert. Als Kultivierungmedium diente hierbei Minimalmedium mit Glucose und einer erhöhten Menge an Mineralsalzen, Spurenelementen und Vitaminen (5 % (w/v) Glucose, 2 g I⁻¹ KH₂PO₄, 0,32 g I⁻¹ K₂HPO₄ x 3 H₂O, 6 g I⁻¹ (NH₄)₂SO₄, 1,4 g I⁻¹ MgSO₄ x 7 H₂O, 1 g I⁻¹ NaCl, 0,8 g I⁻¹ Ca(NO₃)₂ x 4 H₂O, 1 mg I⁻¹ H₃BO₃, 0,08 mg I⁻¹ CuSO₄ x 5 H₂O, 0,2 mg I⁻¹ KI, 0,8 mg I⁻¹ MnSO₄ x 4 H₂O, 0,4 mg I⁻¹ Na₂MoO₄ x 2 H₂O, 0,8 mg I⁻¹ ZnSO₄ x 7 H₂O, 12 mg I⁻¹ FeCl₃ x 6 H₂O, 0,6 mg I⁻¹ Thiaminhydrochlorid). Die Kultivierung erfolgte im Fermenter bei 28 °C. Die Sauerstoffsättigung wurde auf 55 % eingestellt. Die Zellen wurden mit einer OD₆₀₀ von 1 inokuliert und für 2 d bei pH 5,5 (automatisierte Titration von HCl und NaOH) inkubiert. Nach 2 d wurde der pH auf 8,0 eingestellt und es wurden nach 2 d und 3 d 15 g I⁻¹ Dodekan zugegeben. Alle 24 h wurde Glucose zugegeben, um eine Endkonzentration von 5-10 % (w/v) einzustellen.

Nach 6 d Kultiviernung (d.h. nach 4 d in der Produktionsphase) bildete *Y. lipolytica* H222ΔPΔF 29,5 g I⁻¹ ω-Hydroxydodekansäure und 3,5 g I⁻¹ Dodekandisäure (Fig. 7).

Die Erfinding ist in the folgenden Aspekten beschrieben:
1. Ein Hefestamm der Art *Y. lipolytica,* aufweisend eine Verringerung der Aktivitäten der Acyl-CoA-Oxidasen, der dadurch gekennzeichnet ist, dass in diesem zusätzlich
   a) die Aktivitäten relevanter (Fett-)Alkohol-Dehydrogenasen und/oder
   b) die Aktivitäten relevanter (Fett-)Alkohol-Oxidasen reduziert sind,
   wodurch eine gesteigerten Bildung von ω-Hydroxyfettsäuren gemäß der allgemeinen Struktur: HOOC-(CH₂)ₙ-OH, HOOC-(CₙH₂ₙ₋₂)-CH₂OH, COOC-(CₙH₂ₙ₋₄)-CH₂OH oder HOOC-(CₙH₂ₙ₋₆)-CH₂OH, mit n im Bereich von 6 bis 16, aus einem hydrophoben Substrat erfolgt.
2. Hefestamm nach Aspekt 1, dadurch gekennzeichnet, dass zusätzlich die Aktivitäten einzelner oder aller Cytochrom P450-Genprodukte erhöht sind.
3. Hefestamm nach Aspekt 1 oder 2, dadurch gekennzeichnet, dass zusätzlich die Aktivität der NADPH-Cytochrom P450-Reduktase erhöht ist.
4. Hefestamm nach einem der Aspekte 1 bis 3, dadurch gekennzeichnet, dass zusätzlich die Aktivität der Phosphatidsäure-Dephosphohydrolase verringert ist.
5. Hefestamm nach einem der Aspekte 1 bis 4, dadurch gekennzeichnet, dass zusätzlich die Aktivität der Glycerol-3-phosphat-Acyltransferase verringert ist.
6. Hefestamm nach einem der Aspekte 1 bis 5, dadurch gekennzeichnet, dass der Hefestamm eine Deletion der Gene für die Acyl-CoA-Oxidasen, die (Fett-)Alkohol-Dehydrogenasen und/oder die (Fett-)Alkohol-Oxidasen aufweist.
7. Verwendung eines Hefestamms nach einem der Aspekte 1 bis 6 zur mikrobiellen Herstellung von ω-Hydroxyfettsäuren gemäß der allgemeinen Struktur: HOOC-(CH2)n-OH, HOOC-(CₙH₂ₙ₋₂)-CH₂OH, COOC-(CₙH₂ₙ₋₄)-CH₂OH oder HOOC-(CₙH₂ₙ₋₆)-CH₂OH, mit n im Bereich von 6 bis 18, aus einem hydrophoben Substrat.
8. Verfahren zur biokatalytischen Herstellung von ω-Hydroxyfettsäuren aus einem hydrophoben Substrat, das die folgenden Schritte umfasst:
   d) Bereitstellung von Hefezellen mindestens eines Hefestamms der Art *Y. lipolytica,* der
      (iv) eine Verringerung der Aktivitäten der Acyl-CoA-Oxidasen,
      (v) eine reduzierte Aktivität relevanter (Fett-)Alkohol-Dehydrogenasen und
      (vi) eine reduzierte Aktivität relevanter (Fett-)Alkohol-Oxidasen aufweist, in einem geeigneten Kultivierungsmedium,
   e) Kontaktieren der Hefezellen mit dem hydrophoben Substrat, wobei das hydrophobe Substrat mit mindestens einem der Enzyme der Hefezellen, ausgewählt aus der Gruppe,
      (iv) Cytochrom P450-Genprodukte,
      (v) NADPH-Cytochrom P450-Reduktase,
      (vi) (Fett-)Aldehyd-Dehydrogenasen zu dem Reaktionsprodukt ω-Hydroxyfettsäure umgesetzt wird,
   f) Isolierung der ω-Hydroxyfettsäure.
9. Verfahren nach Aspekt 8, dadurch gekennzeichnet, dass die Umsetzung zu dem Reaktionsprodukt ω-Hydroxyfettsäure zusätzlich mit verbleibenden (Fett-)Alkohol-Oxidasen, (Fett-)Alkohol-Dehydrogenasen erfolgt.
10. Verfahren nach Aspekt 8 oder 9, dadurch gekennzeichnet, dass die Hefezellen mindestens eines Hefestamms der Art *Y. lipolytica* ausgewählt sind aus einem Hefestamm nach den Aspekte 1 bis 6.
11. Verfahren zur biokatalytischen Herstellung von Dicarbonsäuren aus einem hydrophoben Substrat, das die folgenden Schritte umfasst:
   d) Bereitstellung von Hefezellen mindestens eines Hefestamms der Art *Y. lipolytica,* der
      (iv) eine Verringerung der Aktivitäten der Acyl-CoA-Oxidasen,
      (v) eine erhöhte Aktivität relevanter (Fett-)Alkohol-Dehydrogenasen und
      (vi) eine erhöhte Aktivität relevanter (Fett-)Alkohol-Oxidasen aufweist, in einem geeigneten Kultivierungsmedium,
   e) Kontaktieren der Hefezellen mit dem hydrophoben Substrat, wobei das hydrophobe Substrat mit mindestens einem der Enzyme der Hefezellen, ausgewählt aus der Gruppe,
      (vi) der Cytochrom P450-Genprodukte,
      (vii) der NADPH-Cytochrom P450-Reduktase,
      (viii) der (Fett-)Alkohol-Dehydrogenasen nach a),
      (ix) der (Fett-)Alkohol-Oxidasen nach a) und
      (x) (Fett-)Aldehyd-Dehydrogenasen zu dem Reaktionsprodukt Dicarbonsäure umgesetzt wird,
   f) Isolierung der Dicarbonsäure.
12. Verfahren nach Aspekt 11, dadurch gekennzeichnet, dass in den Hefezellen zusätzlich die Aktivitäten einzelner oder aller Cytochrom P450-Genprodukte erhöht sind.
13. Verfahren nach Aspekt 11 oder 12, dadurch gekennzeichnet, dass in den Hefezellen zusätzlich die Aktivität der NADPH-Cytochrom P450-Reduktase erhöht ist.
14. Verfahren nach einem der Aspekte 11 bis 13, dadurch gekennzeichnet, dass in den Hefezellen zusätzlich die Aktivitäten relevanter (Fett-)Alkohol-Dehydrogenasen erhöht sind.
15. Verfahren nach einem der Aspekte 11 bis 14, dadurch gekennzeichnet, dass in den Hefezellen zusätzlich die Aktivitäten relevanter (Fett-)Alkohol-Oxidasen erhöht sind.
16. Verfahren nach einem der Aspekte 11 bis 15, dadurch gekennzeichnet, dass in den Hefezellen zusätzlich die Aktivitäten relevanter (Fett-)Aldehyd-Dehydrogenasen erhöht sind.
17. Verfahren nach einem der Aspekte 8 bis 10 oder 11 bis 16, dadurch gekennzeichnet, dass das hydrophobe Substrat ausgewählt ist aus der Gruppe der *n*-Alkane der allgemeinen Struktur: H₃C-(CH₂)ₙ-CH₃, der Alkene der allgemeinen Struktur H₃C-(CₙH₂ₙ₋₂)-CH₃, H₃C-(CₙH₂ₙ₋₄)-CH₃ oder H₃C-(CₙH₂ₙ₋₆)-CH₃, Fettsäuren der allgemeinen Struktur: HOOC-(CH₂)ₙ-CH₃, HOOC-(CₙH₂ₙ₋₂)-CH₃, COOC-(CₙH₂ₙ₋₄)-CH₃ oder HOOC-(CₙH₂ₙ₋₆)-CH₃, und Fettsäurealkylester der allgemeinen Struktur: ROOC-(CH₂)ₙ-CH₃, mit n im Bereich von 6 bis 18.
18. Verfahren nach einem der vorgenannten Aspekte 8 bis 17, dadurch gekennzeichnet, dass zusätzlich zu dem hydrophoben Substrat Glucose als Kohlenstoffquelle verwendet wird.
19. Verfahren nach einem der vorgenannten Aspekte 8 bis 18, dadurch gekennzeichnet, dass die Glucose-Konzentration im Verlauf der Kultivierung nicht auf 0 g I⁻¹ absinkt.
20. Verfahren nach einem der vorgenannten Aspekte 8 bis 19, dadurch gekennzeichnet, dass der Stickstoffgehalt im Kulturmedium nach dem Kontaktieren kleiner als 2 g I⁻¹ ist.
21. Verfahren nach einem der vorgenannten Aspekte 8 bis 20, dadurch gekennzeichnet, dass der pH-Wert nach dem Kontaktieren größer als 5,5 ist.
22. Verfahren nach einem der vorgenannten Aspekte 8 bis 21, dadurch gekennzeichnet, dass das hydrophobe Substrat ein *n*-Alkane, ein Alken oder eine Fettsäure mit n im Bereich 8 bis 18 ist.

## Patentansprüche

1. Ein Hefestamm der Art *Yarrowia lipolytica,* aufweisend eine Verringerung der Aktivität von Fao1p, kodiert von *Gen FA01: YALIOB14014g.*

2. Ein Hefestamm nach Anspruch 1, **dadurch gekennzeichnet, dass** die Aktivität von Fao1p durch Inhibierung oder Verminderung der Expression des kodierenden Gens für Fao1p verringert wird.

3. Ein Hefestamm nach Anspruch 1, **dadurch gekennzeichnet, dass** die Aktivität von Fao1p durch eine partielle oder komplette Deletion des kodierenden Gens für Fao1p verringert wird.

4. Ein Hefestamm nach Anspruch 1, **dadurch gekennzeichnet, dass** die Aktivität von Fao1p durch Expression von einem nicht-funktionalen Gen für Fao1p verringert wird.

5. Ein Hefestamm nach Anspruch 1, **dadurch gekennzeichnet, dass** die Aktivität von Fao1p durch Insertion, Substitution oder Punktmutation in dem für das Protein kodierenden Gen verringert wird.

6. Ein Hefestamm nach einem der Ansprüche 1 bis 5 **dadurch gekennzeichnet, dass** zusätzlich die Aktivität einer Fett-Alkohol-Dehydrogenasen ausgewählt aus FADH, ADH1, ADH2, ADH3, ADH4, ADH5, ADH6 und ADH7 verringert ist.

7. Ein Hefestamm nach Anspruch 6, **dadurch gekennzeichnet, dass** die Aktivität einer Fett-Alkohol-Dehydrogenase durch
(i) Inhibierung oder Verminderung der Expression des kodierenden Gens für die Fett-Alkohol-Dehydrogenase;
(ii) partielle oder komplette Deletion des kodierenden Gens für die Fett-Alkohol-Dehydrogenase;
(iii) Expression von einem nicht-funktionalen Gen für die Fett-Alkohol-Dehydrogenase und/oder
(iv) Inhibierung oder Verminderung der Aktivität der Fett-Alkohol-Dehydrogenase durch Insertion, Substitution oder Punktmutation in dem für das Protein kodierenden Gen
verringert wird.
